(19) Europäisches Patentamt
European Patent Office
Office européen des brevets

(11) EP 1 560 823 B1

(12) EUROPÄISCHE PATENTSCHRIFT

(45) Veröffentlichungstag und Bekanntmachung des Hinweises auf die Patenterteilung:
**09.07.2008 Patentblatt 2008/28**

(21) Anmeldenummer: **03779845.1**

(22) Anmeldetag: **05.11.2003**

(51) Int Cl.:
***C07D 403/04*** $^{(2006.01)}$ ***C07D 401/04*** $^{(2006.01)}$
***A61K 31/454*** $^{(2006.01)}$ ***A61K 31/404*** $^{(2006.01)}$
***A61P 25/00*** $^{(2006.01)}$ ***A61P 29/00*** $^{(2006.01)}$

(86) Internationale Anmeldenummer:
**PCT/EP2003/012306**

(87) Internationale Veröffentlichungsnummer:
**WO 2004/043949 (27.05.2004 Gazette 2004/22)**

(54) **4-SUBSTITUIERTE 1-AMINOCYCLOHEXAN-DERIVATE ZUR VERWENDUNG ALS ORL1-REZEPTOR- UND MU-OPIAT-REZEPTOR-LIGANDEN**

4-SUBSTITUTED 1-AMINOCYCLOHEXANE DERIVATIVES FOR UTILIZATION AS ORL1-RECEPTOR AND MU-OPIATE RECEPTOR LIGANDS

DERIVES DE 1-AMINOCYCLOHEXANE 4-SUBSTITUE UTILISES COMME LIGANDS DE RECEPTEUR ORL1 ET DE RECEPTEUR D'OPIACE MU

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **11.11.2002 DE 10252666**

(43) Veröffentlichungstag der Anmeldung:
**10.08.2005 Patentblatt 2005/32**

(73) Patentinhaber: **Grünenthal GmbH**
**52078 Aachen (DE)**

(72) Erfinder:
- **HINZE, Claudia**
  **52066 Aachen (DE)**
- **SCHICK, Hans**
  **10405 Berlin (DE)**
- **SONNENSCHEIN, Helmut**
  **10245 Berlin (DE)**

(74) Vertreter: **Kutzenberger, Helga**
**Kutzenberger & Wolff**
**Theodor-Heuss-Ring 23**
**50668 Köln (DE)**

(56) Entgegenhaltungen:
**EP-A- 1 323 710**
**WO-A-01/87838**
**US-A- 4 113 866**
**US-A1- 2002 128 288**
**US-B1- 6 172 067**

EP 1 560 823 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft 4-substituierte 1-Aminocyclohexan-Derivate, Verfahren zu deren Herstellung, Arzneimittel enthaltend diese Verbindungen und die Verwendung von 4-substituierte 1-Aminocyclohexan-Derivaten zur Herstellung von Arzneimitteln.

Das Heptadekapeptid Nociceptin ist ein endogener Ligand des ORL1(Opioid-Receptor-Like)-Rezeptors (Meunier et al., Nature 377, 1995, S. 532-535), der zu der Familie der Opioid Rezeptoren gehört und in vielen Regionen des Gehirns und des Rückenmarks zu finden ist und eine hohe Affinität für den ORL1-Rezeptor aufweist. Der ORL1-Rezeptor ist homolog zu den $\mu$, $\kappa$ und $\delta$ Opioid-Rezeptoren und die Aminosäuresequenz des Nociceptin-Peptids weist eine starke Ähnlichkeit mit denen der bekannten Opioidpeptide auf. Die durch das Nociceptin induzierte Aktivierung des Rezeptors führt über die Kopplung mit $G_{i/o}$-Proteinen zu einer Inhibierung der Adenylatcyclase (Meunier et al., Nature 377, 1995, S. 532-535).

Das Nociceptin-Peptid zeigt nach intercerebroventicularer Applikation eine pronociceptive und hyperalgetische Aktivität in verschiedenen Tiermodellen (Reinscheid et al., Science 270, 1995, S. 792-794). Diese Befunde können als Hemmung der stressinduzierten Analgesie erklärt werden (Mogil et al., Neuroscience 75, 1996, S. 333-337). In diesem Zusammenhang konnte auch eine anxiolytische Aktivität des Nociceptin nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin gezeigt werden. Nociceptin wirkt antinociceptiv in verschiedenen Schmerzmodellen, beispielsweise im Tail Flick-Test in der Maus (King et al., Neurosci. Lett., 223, 1997, 113-116. In Modellen für neuropathische Schmerzen konnte ebenfalls eine antinociceptive Wirkung von Nociceptin nachgewiesen, die insofern besonders interessant ist, als dass die Wirksamkeit von Nociceptin nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18, 1998, S. 9685-9694).

Der ORL1-Rezeptor ist außerdem noch an der Regulation weiterer physiologischer und pathophysiologischer Prozesse beteiligt. Hierzu gehören unter anderem Lernen und Gedächtnisbildung (Manabe et al., Nature, 394, 1997, S. 577-581), Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261-1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf $\mu$-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, arterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika diskutiert.

Entsprechend vielfältig sind die Anwendungsmöglichkeiten von Verbindungen, die an den ORL1-Rezeptor binden und diesen aktivieren oder inhibieren. Neben diesem spielen gerade im Bereich der Schmerztherapie, aber auch bei anderen der genannten Indikationen, Opioidrezeptoren wie der $\mu$-Rezeptor und andere Subtypen eine große Rolle. Entsprechend ist es günstig, wenn die Verbindung auch Wirkung an diesen Opioidrezeptoren zeigen.

Dokument WO01/87838 und US4113866 beschreiben Verbindungen, die zur Behandlung von Schmerz geeignet sind.

Aufgabe der vorliegenden Erfindung war es, Arzneimittel zur Verfügung zu stellen, die auf das Nociceptin/ORL1-Rezeptor-System und/oder den $\mu$-Opiat-Rezeptor wirken und damit für Arzneimittel insbesondere zur Behandlung der verschiedenen mit diesem System nach dem Stand der Technik in Verbindung stehenden Krankeiten bzw. zum Einsatz in den dort genannten Indikationen geeignet sind.

Gegenstand der Erfindung sind daher 4-substituierte 1-Aminocyclohexan-Derivate der allgemeinen Formel I,

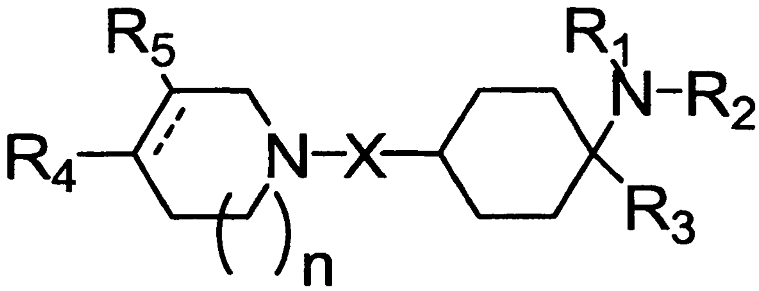

, worin

n = 0 oder 1,

X = eine Bindung, oder C(O), C(O)NH, $C(O)CH_2$, C(O)CH= oder $C(O)NHCH_2$,

$R^1$ und $R^2$ unabhängig voneinander für H; Methyl oder Ethyl stehen oder die Reste $R^1$ und $R^2$ zusammen einen Ring bilden und $(CH_2)_5$ bedeuten;

$R^3$ für $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert, steht;

$R^4$ und $R^5$ unabhängig voneinander für H; oder $(CH_2)_mR^7$, stehen
wobei m = 0-6 und $R^7$H; $C_{3-8}$-Cycloalkyl, Aryl oder Heteroaryl, jeweils unsubstituiert oder einfach oder mehrfach substituiert, bedeutet, wobei nur einer der Reste $R^4$ und $R^5$ H sein kann,
mit der Maßgabe, dass wenn n = 1, einer der Reste $R^1$ oder $R^2$ $CH_3$ und der andere Rest $R^1$ oder $R^2$ H bedeutet, $R^3$ Phenyl und $R^5$ H bedeutet, der Rest $R^4$ nicht 4-Fluorphenyl bedeutet,

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

Alle diese erfindungsgemäßen Verbindungen zeigen eine gute Bindung an den ORL1-Rezeptor, aber auch an andere Opiatrezeptoren, insbesondere an den μ-Opiat-Rezeptor.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht $C_{1-2}$-Alkyl für C1- oder C2-Alkyl, $C_{1-3}$-Alkyl für C1-, C2- oder C3-Alkyl, $C_{1-4}$-Alkyl für C1-, C2-, C3- oder C4-Alkyl, $C_{1-5}$-Alkyl für C1-, C2-, C3-, C4-oder C5-Alkyl, $C_{1-6}$-Alkyl für C1-, C2-, C3-, C4-, C5- oder C6-Alkyl, $C_{1-7}$-Alkyl für C1-, C2-, C3-, C4-, C5-, C6- oder C7-Alkyl, $C_{1-8}$-Alkyl für C1-, C2-, C3-, C4-, C5-, C6-, C7- oder C8-Alkyl, $C_{1-10}$-Alkyl für C1-, C2-, C3-, C4-, C5-, C6-, C7-, C8,- C9- oder C10-Alkyl und $C_{1-18}$-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht $C_{3-4}$-Cycloalkyl für C3- oder C4-Cycloalkyl, $C_{3-5}$-Cycloalkyl für C3-, C4- oder C5-Cycloalkyl, $C_{3-6}$-Cycloalkyl für C3-, C4-, C5- oder C6-Cycloalkyl, $C_{3-7}$-Cycloalkyl für C3-, C4-, C5-, C6- oder C7-Cycloalkyl, $C_{3-8}$-Cycloalkyl für C3-, C4-, C5-, C6-, C7- oder C8-Cycloalkyl, $C_{4-5}$-Cycloalkyl für C4- oder C5-Cycloalkyl, $C_{4-6}$-Cycloalkyl für C4-, C5- oder C6-Cycloalkyl, $C_{4-7}$-Cycloalkyl für C4-, C5-, C6- oder C7-Cycloalkyl, $C_{5-6}$-Cycloalkyl für C5- oder C6-Cycloalkyl und $C_{5-7}$-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch

Adamantyl, $CHF_2$, $CF_3$ oder $CH_2OH$ sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines (gegebenenfalls auch mehrerer) Wasserstoffreste(s) durch F, Cl, Br, I, $NH_2$, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von $CF_3$ oder an verschiedenen Stellen wie im Falle von $-CH(OH)-CH=CH-CHCl_2$. Besonders bevorzugte Substituenten sind hier F, Cl und OH. In Bezug auf Cycloalkyl kann der Wasserstoffrest auch durch $OC_{1-3}$-Alkyl oder $C_{1-3}$-Alkyl (jeweils ein- oder mehrfach substituiert oder unsubstituiert), insbesondere Methyl; Ethyl, n-Propyl, i-Propyl, $CF_3$, Methoxy oder Ethoxy, ersetzt sein.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ zu verstehen, unter $(CH_2)_{1-4}$ ist $-CH_2-$,$-CH_2-CH_2-$, $-CH_2-CH_2-CH_2-$ und $-CH_2-CH_2-CH_2-CH_2-$ zu verstehen, unter $(CH_2)_{4-5}$ ist$-CH_2-CH_2-CH_2-CH_2-$ und $-CH_2-CH_2-CH_2-CH_2-CH_2-$ zu verstehen, etc.

Unter einem Aryl-Rest werden Ringsysteme mit mindestens einem aromatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5] thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert die Substitution des Aryls oder Heteroaryls mit $R^{22}$, $OR^{22}$ einem Halogen, vorzugsweise F und/oder Cl, einem $CF_3$, einem CN, einem $NO_2$, einem $NR^{23}R^{24}$, einem $C_{1-6}$-Alkyl (gesättigt), einem $C_{1-6}$-Alkoxy, einem $C_{3-8}$-Cycloalkoxy, einem $C_{3-8}$-Cycloalkyl oder einem $C_{2-6}$-Alkylen.

Dabei steht der Rest $R^{22}$ für H, einen $C_{1-10}$-Alkyl-, vorzugsweise einen $C_{1-6}$-Alkyl-, einen Aryl- oder Heteroaryl- oder für einen über $C_{1-3}$-Alkyl, gesättigt oder ungesättigt, gebundenen Aryl- oder Heteroaryl-Rest, wobei diese Aryl und Heteroarylreste nicht selbst mit Aryl- oder Heteroaryl-Resten substituiert sein dürfen,
die Reste $R^{23}$ und $R^{24}$, gleich oder verschieden, für H, einen $C_{1-10}$-Alkyl-, vorzugsweise einen $C_{1-6}$-Alkyl-, einen Aryl-, einen Heteroaryl- oder einen über $C_{1-3}$-Alkyl, gesättigt oder ungesättigt, gebundenen Aryl- oder Heteroaryl-Rest bedeuten, wobei diese Aryl und Heteroarylreste nicht selbst mit Aryl- oder Heteroaryl-Resten substituiert sein dürfen,
oder die Reste $R^{23}$ und $R^{24}$ bedeuten zusammen $CH_2CH_2OCH_2CH_2$, $CH_2CH_2NR^{25}CH_2CH_2$ oder $(CH_2)_{3-6}$, und der Rest $R^{25}$ für H, einen $C_{1-10}$-Alkyl-, vorzugsweise einen $C_{1-6}$-Alkyl-, einen Aryl-, oder Heteroaryl- Rest oder für einen über $C_{1-3}$-Alkyl, gesättigt oder ungesättigt, gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/ oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz und das Citrat-Salz.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch -

insbesondere bei Anwendung im Menschen und/oder Säugetier - verträglich sind. Besonders bevorzugt ist das Hydrochlorid oder Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1$\lambda^6$-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind.

Besonders bevorzugt sind die Salze der Alkali- und Erdalkalimetalle aber auch mit $NH_4^+$, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch $NH_4^+$, insbesondere aber (Mono-) oder (Di-) Natrium-, (Mono-) oder (Di-) Kalium-, Magnesium- oder Calzium-Salze.

Für eine bevorzugte Ausführungsform der erfindungsgemäßen 4-substituierte 1-Aminocyclohexan-Derivate gilt, dass

Besonders bevorzugt sind 4-substituierte 1-Aminocyclohexan-Derivate, worin $R^1$ und $R^2$ unabhängig voneinander für $CH_3$ oder H stehen, wobei $R^1$ und $R^2$ nicht gleichzeitig H bedeuten.

Bevorzugt im Sinne dieser Erfindung sind auch 4-substituierte 1-Aminocyclohexan-Derivate, worin
$R^3$ für $C_{3-8}$-Cycloalkyl, Aryl oder Heteroaryl, jeweils unsubstituiert oder einfach oder mehrfach substituiert; über eine gesättigte oder ungesättigte, unverzweigte, substituierte oder unsubstituierte $C_{1-2}$-Alkyl-Gruppe gebundenen Aryl, $C_{3-8}$-Cycloalkyl oder Heteroaryl, jeweils unsubstituiert oder einfach oder mehrfach substituiert, steht;
vorzugsweise
$R^3$ Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; über eine gesättigte, unverzweigte $C_{1-2}$-Alkyl-Gruppe gebundenen $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, bedeutet;
insbesondere
$R^3$ Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl oder Benzothiophenyl, jeweils unsubstituiert oder einfach oder mehrfach substituiert; über eine gesättigte, unverzweigte $C_{1-2}$-Alkyl-Gruppe gebundenen Phenyl, Furyl oder Thiophenyl, jeweils unsubstituiert oder einfach oder mehrfach substituiert, bedeutet.

Besonders bevorzugt sind 4-substituierte 1-Aminocyclohexan -Derivate, worin $R^3$ für Phenyl, Thiophenyl, Pyridyl oder Benzyl, jeweils substituiert oder unsubstituiert, steht, besonders bevorzugt für Phenyl.

Weiterhin bevorzugt sind 4-substituierte 1-Aminocyclohexan-Derivate, worin
$R^7$ H; Cyclopentyl, Cyclohexyl, Cycloheptyl, Cyclooctyl, Indolyl, Naphthyl, Benzofuranyl, Benzothiophenyl, Indanyl, Acenaphthyl, Phenyl, Thiophenyl, Furyl, Pyridyl, Pyrrolyl, Pyrazinyl oder Pyrimidyl, jeweils unsubstituiert oder einfach oder mehrfach substituiert, bedeutet.

Ganz besonders bevorzugt sind 4-substituierte 1-Aminocyclohexan-Derivate, worin $R^7$ für Indolyl, unsubstituiert oder einfach oder mehrfach substituiert, steht.

Darüber hinaus sind ganz besonders bevorzugt 4-substituierte 1-Aminocyclohexan-Derivate aus der Gruppe
{4-[3-(1*H*-indol-3-yl)-pyrrolidin-1-yl]-1-phenyl-cyclohexyl}-dimethylamin; dihydrochlorid (unpolareres Diastereoisomer)
{4-[3-(1*H*-Indol-3-yl)-pyrrolidin-1-yl]-1-phenyl-cyclohexyl}-dimethylamin; dihydrochlorid (Diastereoisomerengemisch)
{4-[4-(5-Chlor-1*H*-indol-3-yl)-piperidin-1-yl]-1-phenyl-cyclohexyl}-dimethylamin; dihydrochlorid (unpolareres Diastereoisomer)
{4-[4-(5-Chlor-1H-indol-3-yl)-piperidin-1-yl]-1-phenyl-cyclohexyl}-dimethylamin; dihydrochlorid (polareres Diastereoisomer)
{4-[4-(1H-Indol-3-yl)-piperidin-1-yl]-1-phenyl-cyclohexyl}-dimethylamin; dihydrochlorid (polareres Diastereoisomer)
{4-[4-(1*H*-Indol-3-yl)piperidin-1-yl]-1-phenylcyclohexyl}-dimethylamin; dihydrochlorid (unpolareres Diastereoisomer)
{4-[4-(5-Methoxy-1H-indol-3-yl)-piperidin-1-yl]-1-phenyl-cyclohexyl}-dimethylamin; dihydrochlorid (unpolareres Diastereoisomer)
{4-[4-(5-Methoxy-1*H*-indo)-3-yl)piperidin-1-yl]-1-phenylcyclohexyl}-dimethylamin; dihydrochlorid (polareres Diastereoisomer)

{4-[3-(1H-Indol-3-yl)-piperidin-1-yl]-1-phenyl-cyclohexyl}-dimethylamin; dihydrochlorid (unpolareres Diastereoisomer)
{4-[3-(1*H*-Indol-3-yl)piperidin-1-yl]-1-phenylcyclohexyl}-dimethylamin; dihydrochlorid (polareres Diastereoisomer)
2-(4-Dimethylamino-4-phenylcyclohexyliden)-1-[3-(1*H*-indol-3-yl)pyrrolidin-1-yl]ethanon; hydrochlorid (Diastereoisomerengemisch)
{1-(4-Fluorphenyl)-4-[3-(1*H*-indol-3-yl)pyrrolidin-1-yl]cyclohexyl}-dimethylamin; dihydrochlorid (polareres und unpolareres Diastereoisomer)
4-(5- Methoxy- 1*H*-indol- 3- yl)- 3,6- dihydro- 2*H*-pyridin- 1- carbonsäure-(4- dimethylamino- 4- phenylcyclohexylmethyl)-amid; citrat (polareres und unpolareres Diastereoisomer)
4-(1*H*-Indol-3-yl)piperidin-1-carbonsäure-(4-dimethylamino-4-phenylcyclohexylmethyl)-amid; hemicitrat bzw. -citrat (polareres und unpolareres Diastereoisomer)
4-(5-Chlor-1*H*-indol-3-yl)piperidin-1-carbonsäure-(4-dimethylamino-4-phenylcyclohexylmethyl)-amid; citrat (polareres Diastereoisomer) bzw. -hemicitrat (unpolareres Diastereoisomer)
4-(1*H*-Indol-3-yl)-3,6-dihydro-2*H*-pyridin-1-carbonsäure-(4-dimethylamino-4-phenylcyclohexylmethyl)-amid; citrat (polareres und unpolareres Diastereoisomer)
4-(5-Fluor-1*H*-indol-3-yl)piperidin-1-carbonsäure-(4-dimethylamino-4-phenylcyclohexylmethyl)-amid; hemicitrat bzw. -citrat (polareres und unpolareres Diastereoisomer)
2-(4-Dimethylamino-4-phenylcyclohexyliden)-1-[4-(1*H*-indol-3-yl)-3,6-dihydro-2*H*-pyridin-1-yl]ethanon; Hydrochlorid
2-(4-Dimpethylamino-4-phenylcyclohexyliden)-1-[4-(1*H*-indol-3-yl)-piperidin-1-yl]ethanon; Hydrochlorid
1-[4-(5-Chlor-1*H*-indol-3-yl)piperidin-1-yl]-2-(4-dimethylamino-4-phenylcyclohexyliden)ethanon; Hydrochlorid
2-(4-Dimethylamino-4-phenylcyclohexyliden)-1-[4-(5-methoxy-1*H*-indol-3-yl)-3,6-dihydro-2*H*-pyridin-1-yl]ethanon; Hydrochlorid
4-(1*H*-Indol-3-yl)piperidin-1-carbonsäure-(4-dimethylamino-4-phenylcyclohexyl)-amid; Hydrochlorid (polareres und unpolareres Diastereoisomer)
4-(5-Chlor-1*H*-indol-3-yl)piperidin-1-carbonsäure-(4-dimethylamino-4-phenylcyclohexyl)-amid; Hydrochlorid (unpolareres Diastereoisomer)
4-(5-Methoxy-1*H*-indol-3-yl)piperidin-1-carbonsäure-(4-dimethylamino-4-phenylcyclohexyl)-amid; Hydrochlorid (unpolareres Diastereoisomer)
4-(5-Fluor-1*H*-indol-3-yl)piperidin-1-carbonsäure-(4-dimethylamino-4-phenylcyclohexyl)-amid; Hydrochlorid (polareres und unpolareres Diastereoisomer)
3-(5-Chlor-1*H*-indol-3-yl)piperidin-1-carbonsäure-(4-dimethylamino-4-phenylcyclohexyl)-amid; Hydrochlorid (polareres und unpolareres Diastereoisomer)
3-(5-Methoxy-1*H*-indol-3-yl)piperidin-1-carbonsäure-(4-dimethylamino-4-phenylcyclohexyl)-amid; Hydrochlorid (polareres und unpolareres Diastereoisomer)
2-(4-Dimethylamino-4-phenylcyclohexyl)-1-[3-(1*H*-indol-3-yl)pyrrolidin-1-yl]-ethanon; Hydrochlorid
2-[4-Dimethylamino-4-(4-fluorphenyl)cyclohexyl]-1-[3-(1*H*-indol-3-yl)pyrrolidin-1-yl]-ethanon; Hydrochlorid (polareres und unpolareres Diastereoisomer)
3-(5-Fluor-1*H*-Indol-3-yl)piperidin-1-carbonsäure-(4-dimethylamino-4-phenylcyclohexyl)-amid; Hydrochlorid (polareres Diastereoisomer)
4-(5-Fluor-1*H*-indol-3-yl)-3,6-dihydro-2*H*-pyridin-1-carbonsäure-(4-dimethylamino-4-phenylcyclohexylmethyl)-amid; citrat (polareres und unpolareres Diastereoisomer)
4-(5-Methoxy-1*H*-indol-3-yl)piperidin-1-carbonsäure-(4-dimethylamino-4-phenylcyclohexylmethyl)-amid; citrat (polareres und unpolareres Diastereoisomer)
3-(1*H*-Indol-3-yl)piperidin-1-carbonsäure-(4-dimethylamino-4-phenylcyclohexylmethyl)-amid; citrat (polareres und unpolareres Diastereoisomer)
3-(5-Fluor-1*H*-indol-3-yl)piperidin-1-carbonsäure-(4-dimethylamino-4-phenylcyclohexylmethyl)-amid; citrat (polareres und unpolareres Diastereoisomer)
3-(1*H*-Indol-3-yl)pyrrolidin-1-carbonsäure-(4-dimethylamino-4-phenylcyclohexylmethyl)-amid; citrat (polareres und unpolareres Diastereoisomer)
2-(4-Dimethylamino-4-phenylcyclohexyl)-1-[3-(1*H*-indol-3-yl)piperidin-1-yl]-ethanon; Hydrochlorid (Diastereoisomerengemisch)
2-(4-Dimethylamino-4-phenylcyclohexyliden)-1-[3-(1*H*-indol-3-yl)-piperidin-1-yl]-ethanon; Hydrochlorid (Diastereoisomerengemisch)
4-[4-(5-Chlor-1*H*-indol-3-yl)-3,6-dihydro-2*H*-pyridin-1-yl]-1-phenylcyclohexyldimethylamin; dihydrochlorid (Diastereoisomerengemisch)
{4-[4-(1*H*-Indol-3-yl)-3,6-dihydro-2*H*-pyridin-1-yl]-1-phenylcyclohexyl}-dimethylamin; dihydrochlorid (Diastereoisomerengemisch)
{4-[4-(5-Methoxy-1*H*-indol-3-yl)-3,6-dihydro-2*H*-pyridin-1-yl]-1-phenylcyclohexyl}-dimethylamin; dihydrochlorid (Diastereoisomerengemisch)

4-(5-Chlor-1*H*-indol-3-yl)-3,6-dihydro-2*H*-pyridin-1-carbonsäure-(4-dimethylamino-4-phenyl-cyclohexylmethyl)-amid; Hydrochlorid (unpolareres und polareres Diastereoisomer)
2-(4-Dimethylamino-4-(4-fluorphenyl)cyclohexyliden)-1-[3-(1*H*-indol-3-yl)pyrrolidin-1-yl]-ethanon; Hydrochlorid (Diastereoisomerengemisch)
Dimethyl-(1-phenyl-4-piperidin-1-ylcyclohexyl)amin; dihydrochlorid (polareres Diastereoisomer)

Die erfindungsgemäßen Substanzen wirken beispielsweise auf den im Zusammenhang mit verschiedenen Erkrankungen relevanten ORL1-Rezeptor, sodass sie sich als pharmazeutischer Wirkstoff in einem Arzneimittel eignen. Ein weiterer Gegenstand der Erfindung sind daher Arzneimittel enthaltend wenigstens ein 4-substituierte 1-Aminocyclohexan-Derivat.

Die erfindungsgemäßen Arzneimittel enthalten neben mindestens einem erfindungsgemäßen 4-substituierte 1-Aminocyclohexan-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße 4-substituierte 1-Aminocyclohexan-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen 4-substituierte 1-Aminocyclohexan-Derivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines erfindungsgemäßen 4-substituierte 1-Aminocyclohexan-Derivats appliziert.

Für alle vorstehenden Formen der erfindungsgemäßen Arzneimittel ist es besonders bevorzugt, wenn das Arzneimittel neben wenigstens einem 4-substituierte 1-Aminocyclohexan-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes 4-substituierte 1-Aminocyclohexan-Derivat als reines Diastereomer und/oder Enantiomer, als Razemat oder als nicht-äquimolare oder äquimolare Mischung der Diastereomere und/oder Enantiomere vor.

Wie in der Einleitung am Stand der Technik abzulesen, wurde der ORL1-Rezeptor insbesondere im Schmerzgeschehen identifiziert. Entsprechend können erfindungsgemäße 4-substituierte 1-Aminocyclohexan-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen 4-substituierte 1-Aminocyclohexan-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist daher die Verwendung eines erfindungsgemäßen 4-substituierte 1-Aminocyclohexan-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes 4-substituierte 1-Aminocyclohexan-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung sind Verfahren zur Herstellung der erfindungsgemäßen 4-substituierten 1-Aminocyclohexan-Derivate wie in der folgenden Beschreibung und Beispielen ausgeführt. Insbesondere geeignet

sind dabei folgende Verfahren zur Herstellung erfindungsgemäßer 4-substituierte 1-Aminocyclohexan-Derivate mit folgenden Schritten,
wobei $R^1$, $R^2$, $R^3$, $R^4$, $R^5$ die für erfindungsgemäße Verbindungen gemäß Formel I angegebene Bedeutung haben, und
$R^{01}$ und $R^{02}$ unabhängig voneinander für eine Schutzgruppe stehen oder die für erfindungsgemäße Verbindungen gemäß Formel I für $R^1$ und $R^2$ angegebene Bedeutung haben und "Bn" für die Benzyl-Schutzgruppe steht:

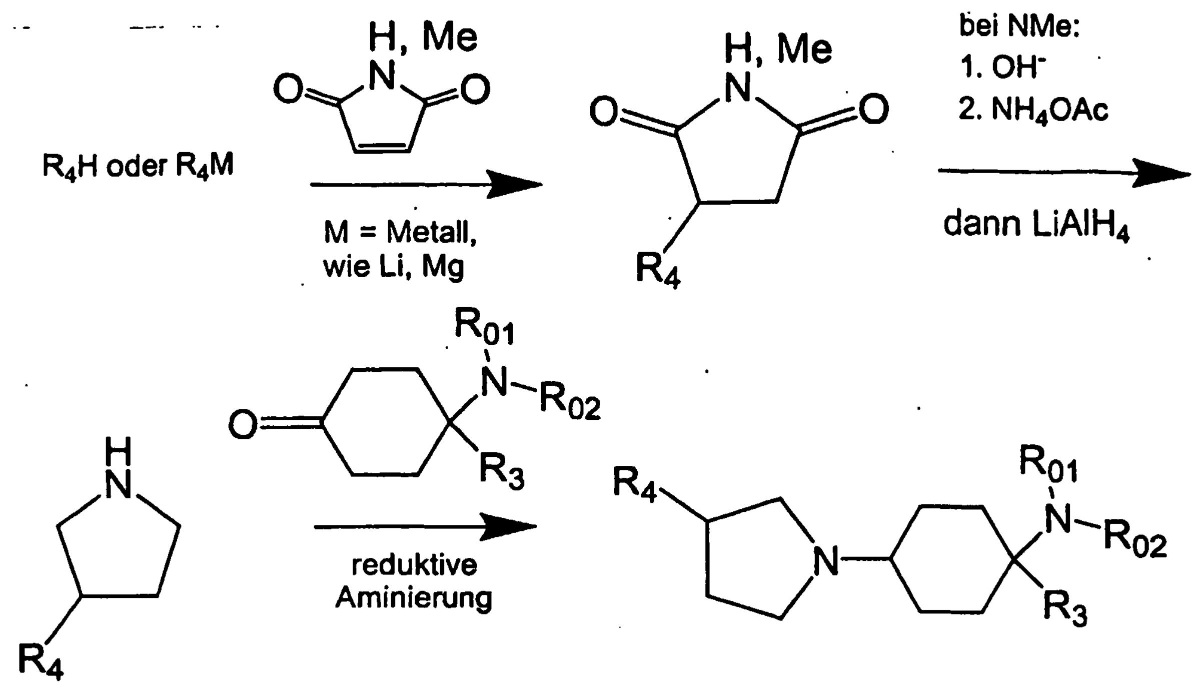


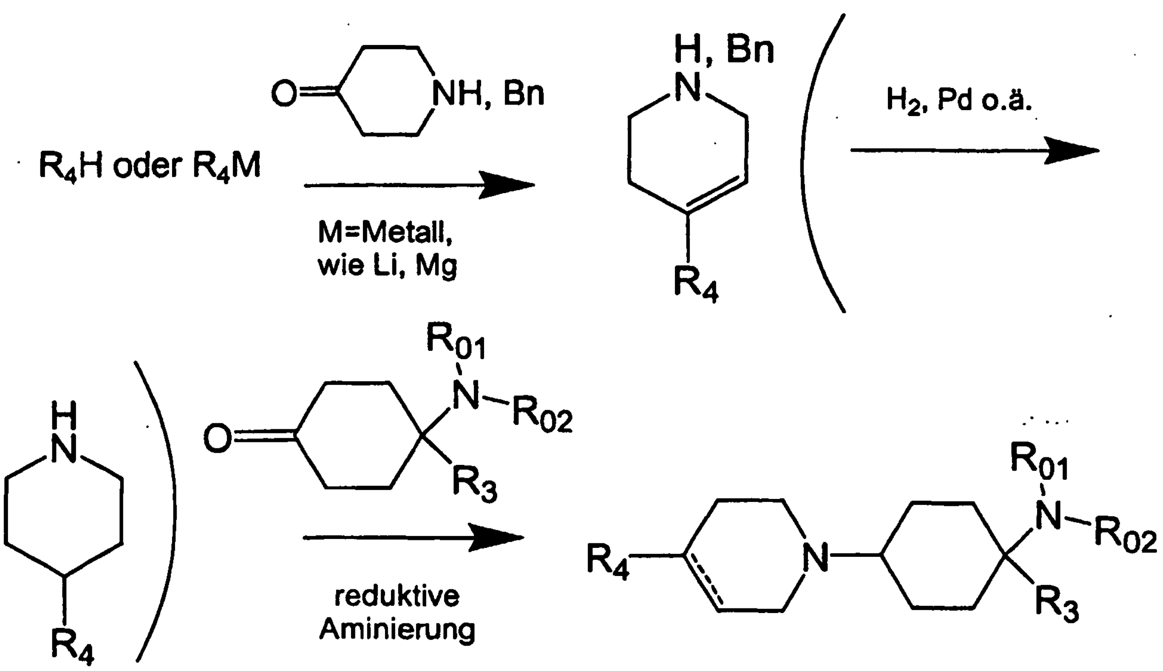


3-Substituierte Piperidine werden analog aus 3-Piperidon hergestellt.

In Verfahren I zur Herstellung von 4-substituierten 1-Aminocyclohexan-Derivaten der allgemeine Formel I, bei

denen n 0 bedeutet, wird Maleinimid oder sein N-Methyl-Derivat mit $R^4H$ unter Zugabe von Säure, beispielsweise Essigsäure, oder $R^4M$, wobei M für Li oder MgX (X = Cl, Br) steht, umgesetzt. Das erhaltene Produkt wird gegebenenfalls durch Zugabe von Base, beispielsweise NaOH und anschließender Zugabe einer $NH_4^+$-Quelle, beispielsweise $NH_4OAc$, demethyliert und danach mit Lithiumaluminiumhydrid zum entsprechenden Pyrrolidin-Derivat reduziert.

Zur Herstellung von 4-substituierten 1-Aminocyclohexan-Derivaten der allgemeine Formel I, bei denen n 1 bedeutet, wird 4-Piperidon oder 3-Piperidon bzw. die entsprechenden N-Benzyl-Derivate mit $R^4H$ unter Zugabe von Säure, beispielsweise Essigsäure, oder $R^4M$, wobei M für Li oder Mg steht, und Zugabe von Säure, umgesetzt. Das erhaltene Tetrahydropyridin wird gegebenenfalls zu dem entsprechenden Piperidin reduziert, beispielsweise mit $H_2$/Pd.

Das erhaltene Pyrrolidin- oder Piperidin-Derivat wird mit einem geeigneten 4-Aminocyclohexanon-Derivat unter dem Fachmann bekannten Bedingungen zur reduktiven Aminierung, beispielsweise mit Hydriden wie Natrium- oder Lithiumborhydrid, Natriumcyanoborhydrid, Natriumtriacetoxyborhydrid, Diisobutylaluminiumhydrid, Lithium-tri-(sec.-butyl)borhydrid (L-Selectride®) oder Lithiumaluminiumhydrid, zu den erfindungsgemäßen 4-substituierten 1-Aminocyclohexan-Derivaten umgesetzt.

Die Herstellung geeigneter 4-Aminocyclohexanone ist aus der Literatur bekannt (Lednicer et al., J. Med. Chem., 23, 1980, 424-430; WO 0290317).

Die Isolierung der erfindungsgemäßen Verbindungen durch Säulenchromatographie mit Kieselgel als stationärer Phase und Ethylacetat, Methanol, Gemischen aus Ethylacetat und Methanol oder Gemischen aus Ethylacetat und Diethylether als Laufmittel führt zu einer Auftrennung der unterschiedlich polaren Diastereoisomeren. Diese wurden aufgrund ihrer Laufzeit bei der Trennung als "unpolarstes Diastereoisomer" (kürzeste Laufzeit) bis "polarstes Diastereoisomer" (längste Laufzeit) charakterisiert.

**Verfahren II**

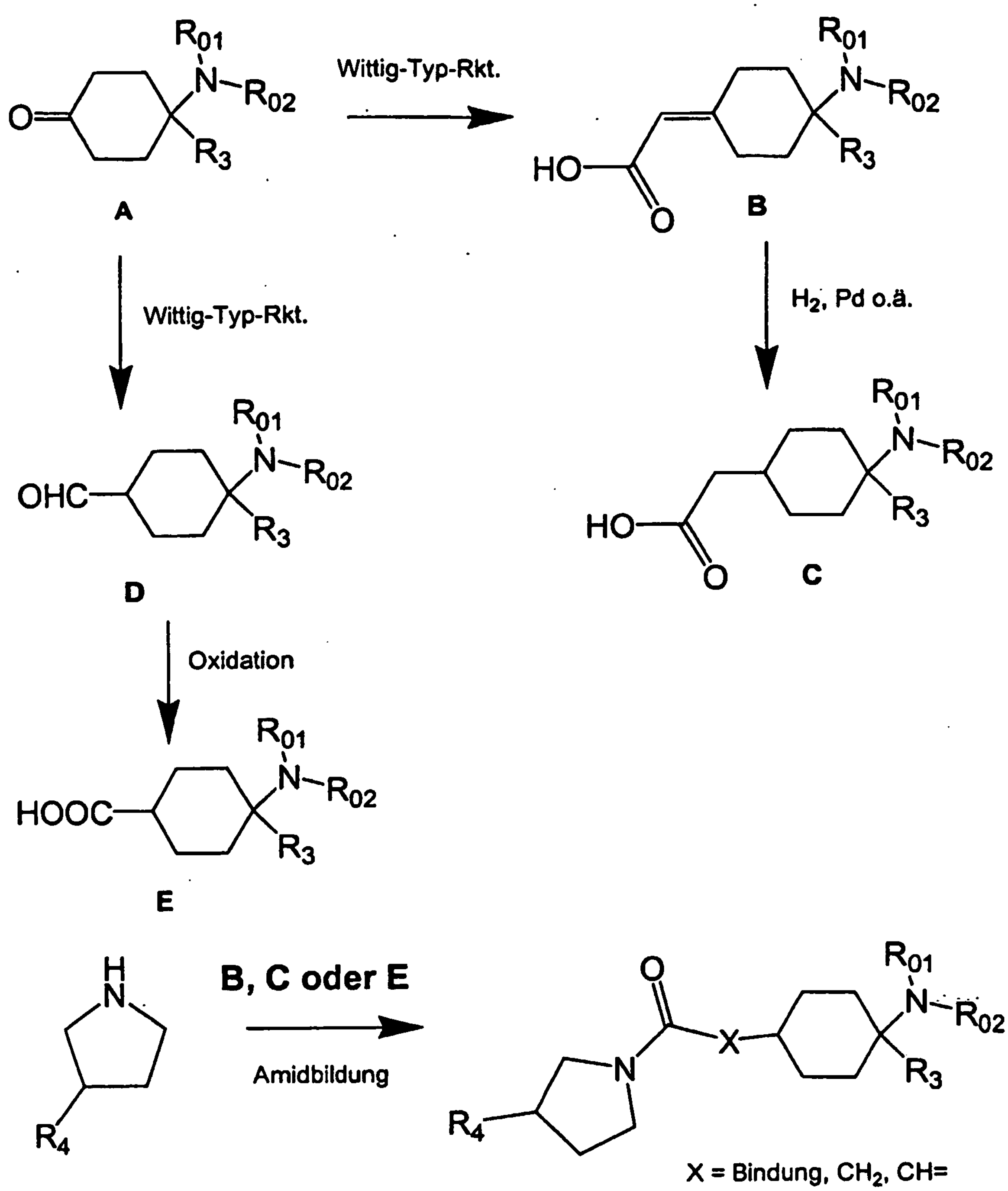

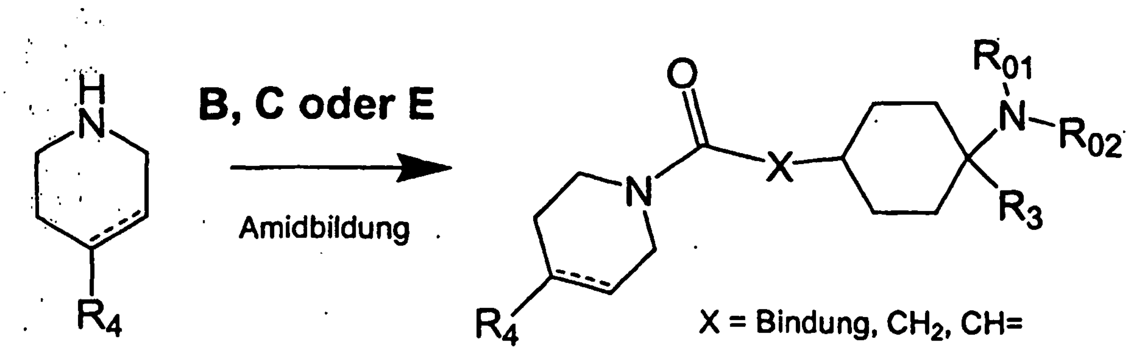


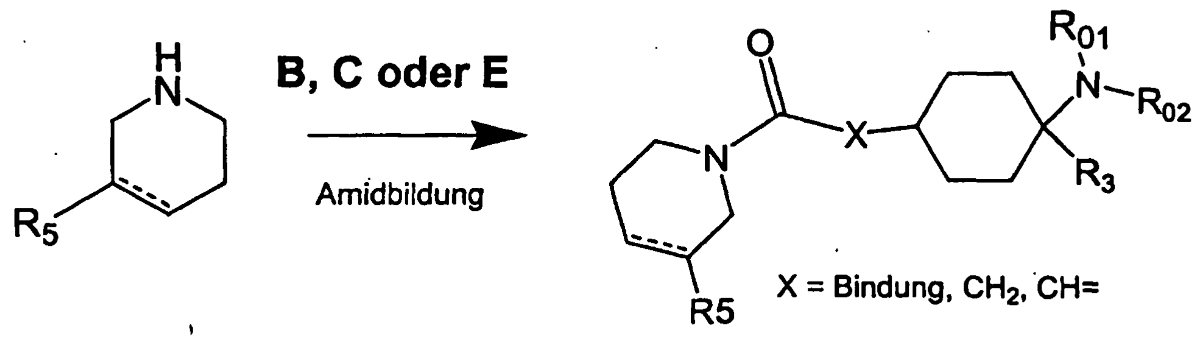


In Verfahren II zur Herstellung von 4-substituierten 1-Aminocyclohexan-Derivaten der allgemeinen Formel I werden 4-Aminocyclohexanon-Derivate a) mit Methoxytriphosphoniumchlorid und einer Base, beispielsweise Natriumhydrid, und anschließend mit wäßriger Säure, beispielsweise HCl, zu den entsprechenden Aldehyden umgesetzt. Durch Zugabe eines Oxidationsmittels, beispielsweise Kaliumpermanganat, werden die entsprechenden Carbonsäuren erhalten. b) 4-Aminocyclohexanon-Derivate können auch mit Phosphonoessigsäuretrimethylsilylester und einer Base, beispielsweise Natriumhydrid, zu Cyclohexylidenessigsäurederivaten umgesetzt werden. Gegebenenfalls wird die Doppelbindung auch hydriert, beispielsweise mit $H_2$/Pd.

Die so erhaltenen Carbonsäurederivate werden mit den zuvor beschriebenen Piperidin-, Pyrrolidin-und Tetrahydropyridin-Derivaten unter Einsatz von Kupplungsreagenzien, die dem Fachmann aus der Peptidchemie bekannt sind, oder nach Überführung der Carbonsäuren in ein Säurechlorid oder einen Aktivester, beispielsweise einen 4-Nitrophenylester oder einen N-Hydroxysuccinimidester, umgesetzt. '

**Verfahren III**

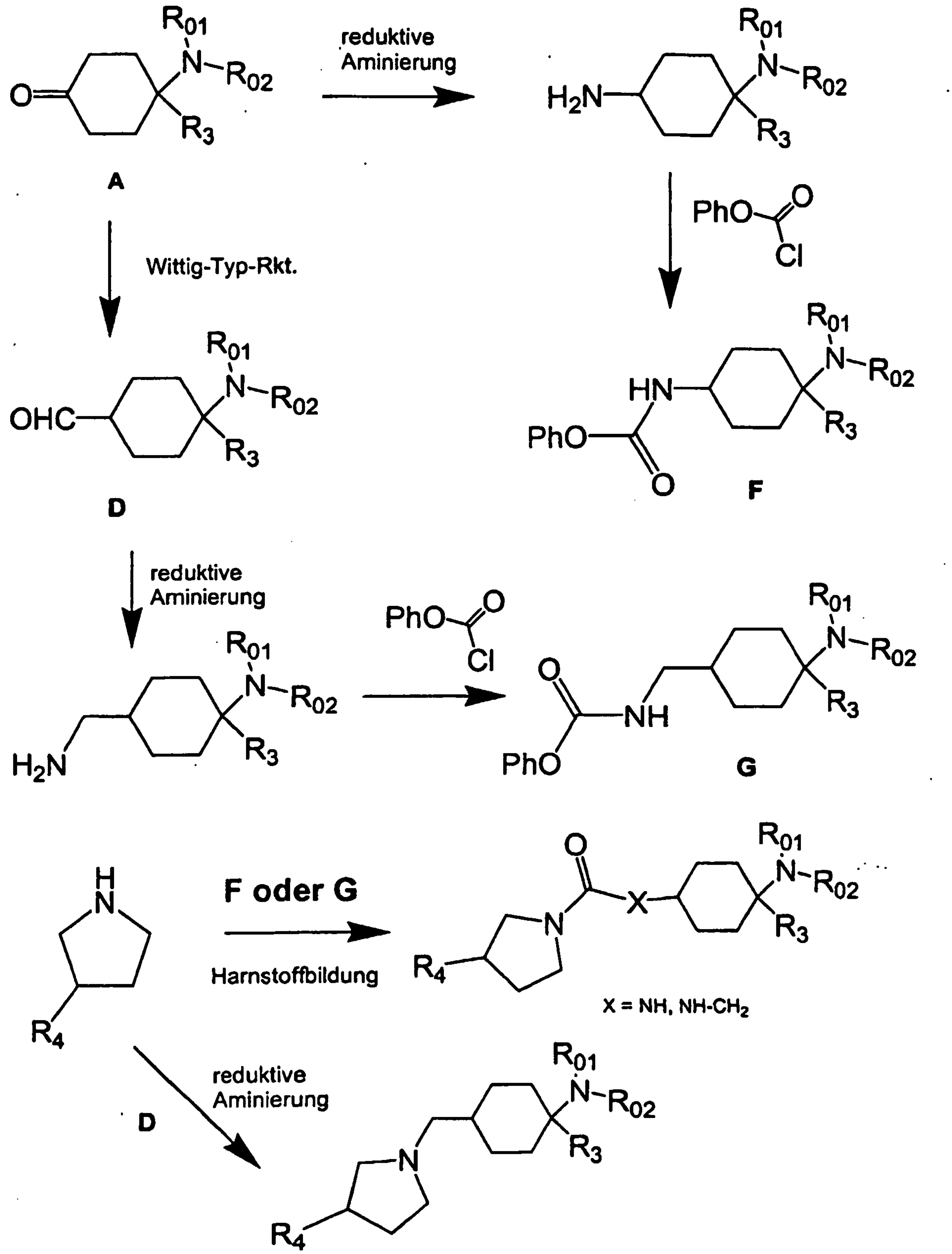


A

D

F

G

X = NH, NH-CH$_2$

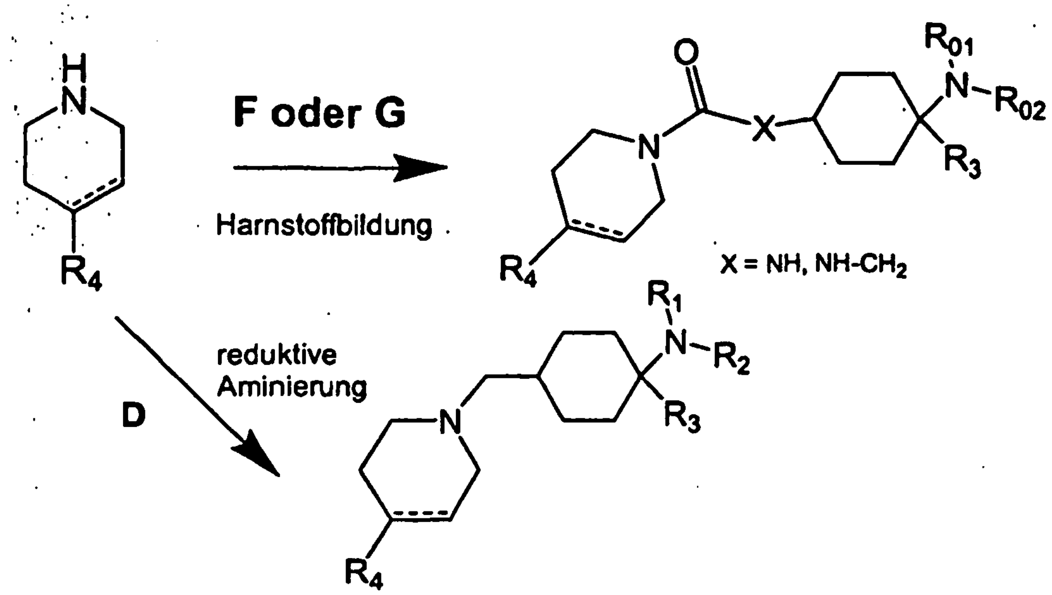


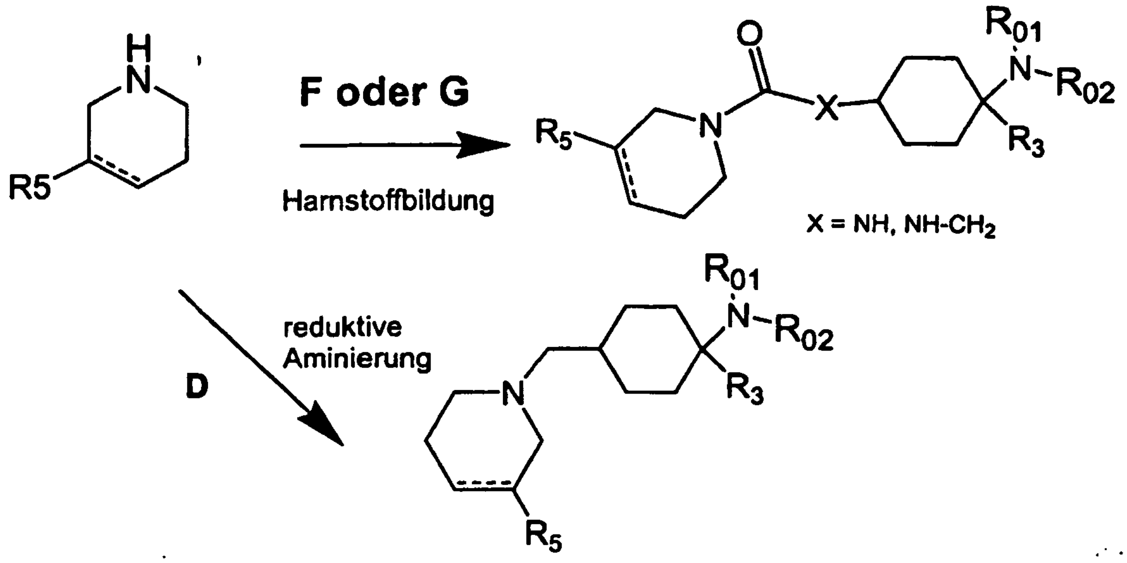


In Verfahren III werden 4-Aminocyclohexanon-Derivate bzw. die in Verfahren II beschriebenen Cyclohexancarbaldehyde mit Hydroxylamin zum Oxim umgesetzt, das dann zum Amin mit einem Reduktionsmittel, beispielsweise Lithiumaluminiumhydrid, reduziert wird. Nach Umsetzung des Amins mit Chlorameisensäureph'enylester werden die Produkte mit den in Verfahren I beschriebenen Piperidin-, Pyrrolidin- und Tetrahydropyriden-Derivaten bei einer Temperatur zwischen 50 und 130°C zu erfindungsgemäßen Harnstoffen. 4-Aminocyclohexancarbaldehyde können auch mit Piperidin-, Pyrrolidin- und Tetrahydropyriden-Derivaten unter dem Fachmann bekannten Bedingungen zur reduktiven Aminierung, beispielsweise mit Hydriden wie Natrium- oder Lithiumborhydrid, Natriumcyanoborhydrid, Natriumtriacetoxyborhydrid, Diisobutylaluminiumhydrid, Lithium-tri-(sec.-butyl)borhydrid (L-Selectride®) oder Lithiumaluminiumhydrid, zu erfindungsgemäßen Aminen umgesetzt.

Die Isolierung der erfindungsgemäßen Verbindungen durch Säulenchromatographie mit Kieselgel als stationärer Phase führt zu einer Auftrennung der unterschiedlich polaren Diastereoisomeren. Diese wurden aufgrund ihrer Laufzeit bei der Trennung als "unpolarstes Diastereoisomer" (kürzeste Laufzeit) bis "polarstes Diastereoisomer" (längste Laufzeit) charakterisiert.

## Beispiele

Die folgenden Beispiele dienen zur näheren Erläuterung der Erfindung, schränken aber den allgemeinen Erfindungsgedanken nicht ein.
Die Ausbeuten der hergestellten Verbindungen sind nicht optimiert.
Alle Temperaturen sind unkorrigiert.
Die Angabe "Ether" bedeutet Diethylether, "EE" bedeutet Ethylacetat und "DCM" bedeutet Dichlormethan. Die Angabe "Äquivalente" bedeutet Stoffmengenäquivalente, "Smp." bedeutet Schmelzpunkt bzw. Schmelzbereich, "Zers." Zersetzung, "RT" bedeutet Raumtemperatur, "abs." absolut (wasserfrei), ,"rac." racemisch, "konz." Konzentriert, "min" Minuten, "h" Stunden, "d" Tage, "Vol.%", Volumenprozent, "m%" Massenprozent und "M" ist eine Konzentrationsangabe in mol/l.
Als stationäre Phase für die Säulenchromatographie wurde Kieselgel 60 (0.040 - 0.063 mm) der Firma E. Merck, Darmstadt, eingesetzt.
Die dünnschicht-chromatographischen Untersuchungen wurden mit HPTLC-Fertigplatten, Kieselgel 60 F 254, der Firma E. Merck, Darmstadt, durchgeführt.
Die Mischungsverhältnisse von Laufmitteln für chromatographische Untersuchungen sind stets in Volumen/Volumen angegeben.
Die im folgenden eingesetzten Verbindungen waren entweder kommerziell erhältlich, oder ihre Herstellung ist aus dem Stand der Technik bekannt oder in für den Fachmann offensichtlicher Weise aus dem Stand der Technik abgeleitet worden.

### Beispiel 1:

### {4-[3-(1H-Indol-3-yl)-pyrrolidin-1-yl]-1-phenyl-cyclohexyl}-dimethyl-amin; Dihydrochlorid (unpolareres Diastereoisomer)

### 3-(1*H*-Indol-3-yl)pyrrolidin-2,5-dion

Indol (4,38g, 40 mmol) und Maleinimid (7,84g, 80 mmol) wurden in konzentrierter Essigsäure (35 ml) gelöst und 36 h unter Rückfluss gerührt. Nach Abkühlen des Ansatzes wurde langsam Wasser (10 ml) zugetropft. Anschließend wurde die Lösung 24 h in den Kühlschrank gestellt. Der dabei entstandene Feststoff wurde abfiltriert. 3-(1*H*-Indol-3-yl) pyrrolidin-2,5-dion wurde so in einer Ausbeute von 3,24 g (38 %) als brauner Feststoff erhalten.

### 3-Pyrrolidin-3-yl-1*H*-indol

Lithiumaluminiumhydrid (2,86 g, 75,6 mmol) wurde unter Ausschluss von Luftfeuchtigkeit in trockenem THF (50 ml) suspendiert. Die Suspension wurde mittels eines Eis-Kochsalzbades auf eine Innentemperatur von ca. 0 ˚C gekühlt. Anschließend wurde 3-(1*H*-Indol-3-yl)pyrrolidin-2,5-dion (3,24g, 15,12 mmol) innerhalb von 15 min portionsweise zugegeben. Nach ca. 1 h hatte das Reaktionsgemisch RT erreicht und wurde dann 32 h unter Rückfluss zum Sieden erhitzt. Nach Abkühlung wurde das Reaktionsgemisch zunächst vorsichtig mit feuchtem THF (5 ml Wasser in 25 ml THF), dann mit 5M Natronlauge (3 ml) und schließlich mit Wasser (3 ml) versetzt. Der Ansatz wurde 20 min gerührt und über Kieselgur filtriert. Das nach mehrmaligem Waschen des Filterkuchens mit Methanol erhaltene Lösungsmittelgemisch wurde zur Trockne eingeengt. Der erhaltene ölige Rückstand wurde an Kieselgel mit Methanol/30-proz. wässrigem Ammoniak (20 : 1) säulenchromatographisch gereinigt. Das Produkt wurde noch einmal über Kieselgur filtriert. Die nach mehrmaligem Waschen des Kieselgurs mit Chloroform erhaltene Lösung wurde zur Trockne eingeengt. Das gewünschte Produkt konnte so in einer Ausbeute von 670 mg (24 %) als viskoses Öl erhalten werden.

### {4-[3-(1*H*-Indol-3-yl)pyrrolidin-1-yl]-1-phenylcyclohexyl}dimethylamin

4-Dimethylamino-4-phenyl-cyclohexanon (217 mg, 1 mmol) und 3-Pyrrolidin-3-yl-1*H*-indol (186 mg, 1 mmol) wurden in einem Gemisch aus trockenem 1,2-Dichlorethan (5 ml) und Tetrahydrofuran (15 ml) gelöst. Zu diesem Gemisch wurde Eisessig (57 μl, 1 mmol) und Natriumsulfat (500 mg) hinzugefügt. Nach einer Reaktionszeit von 15 min wurde die Reaktionsmischung mit Natriumtriacetoxyborhydrid (318 mg, 1,5 mmol) versetzt und 4 d bei RT gerührt. Zur Aufarbeitung wurde das Lösungsmittel abdestilliert und der Ansatz mit gesättigter Natriumhydrogencarbonatlösung (20 ml) und Wasser (20 ml) versetzt. Diese wässrige Phase wurde mit DCM (3 × 30 ml) extrahiert. Die organische Phase wurde mit $Na_2SO_4$ getrocknet und eingeengt. Die chromatographische Reinigung des Substanzgemisches an Kieselgel erfolgte mit Methanol/30-proz. wässrigem Ammoniak (500 : 1). Beim Lösen des Substanzgemisches im Laufmittel fiel ein farbloser Feststoff aus, der abfiltriert wurde. Durch NMR-spektroskopische Analytik konnte dieser als das unpolarere Diastereoisomer von {4-[3-(1*H*-Indol-3-yl)pyrrolidin-1-yl]-1-phenylcyclohexyl}dimethylamin identifiziert werden. Das unpolarere Diastereoisomer wurde in einer Ausbeute von 79 mg (20 %) erhalten. Das restliche Substanzgemisch wurde durch Flash-Chromatographie gereinigt. Das Diastereoisomerengemisch konnte in einer Ausbeute von 136 mg (35 %) erhalten werden.

**{4-[3-(1*H*-Indol-3-yl)pyrrolidin-1-yl]-1-phenylcyclohexyl}dimethylamin-dihydrochlorid (unpolareres Diastereoisomer)**

Zur Herstellung des Dihydrochlorids wurde das unpolarere Diastereoisomer von {4-[3-(1*H*-Indol-3-yl)pyrrolidin-1-yl]-1-phenylcyclohexyl}dimethylamin (79 mg, 0,2 mmol) in Ethylmethylketon (5 ml) gelöst, mit $Me_3SiCl$ (65 μl, 0,5 mmol) versetzt und bei RT 2h gerührt. Der dabei entstandene Feststoff wurde abfiltriert. Das Dihydrochlorid der unpolareren Base konnte so in einer Ausbeute von 77 mg (82 %) als farbloser Feststoff mit einem Smp. von 181-209 ˚C erhalten werden (**Beispiel 1**).

**Beispiel 2:**

**{4-[3-(1H-Indol-3-yl)-pyrrolidin-1-yl]-1-phenyl-cyclohexyl}-dimethyl-amin; Dihydrochlorid (Diastereoisomerengemisch)**

Zur Herstellung des Dihydrochlorids von {4-[3-(1*H*-Indol-3-yl)pyrrolidin-1-yl]-1-phenylcyclohexyl}dimethylamin wurde das Diastereoisomerengemisch (136 mg, 0,35 mmol) in Ethylmethylketon (5 ml) gelöst, mit $Me_3SiCl$ (111 μl, 0,87 mmol) versetzt und bei RT 1 h gerührt. Der entstandene Feststoff wurde abfiltriert. Das Produkt konnte so in einer Ausbeute von 145 mg (90 %) als farbloser Feststoff mit einem Smp. von 182-206 ˚C erhalten werden.

**Beispiel 3:**

**{4-[4-(5-Chlor-1H-indol-3-yl)-piperidin-1-yl]-1-phenyl-cyclohexyl}-dimethyl-amin; Dihydrochlorid (unpolareres Diastereoisomer)**

**5-Chlor-3-piperidin-4-yl-1*H*-indol**

5-Chlor-3-(1,2,3,6-tetrahydropyridin-4-yl)-1*H*-indol (2 g, 9,25 mmol) wurde unter Argon in Methanol (50 ml) gelöst. Pd/Kohle (100 mg, 5 %) wurde zugegeben und das Reaktionsgemisch 8 h bei 2 bar hydriert. Zur Aufarbeitung wurde die Lösung über Kieselgel abfiltriert und mit Methanol (10 × 20 ml) gewaschen. Das Filtrat wurde eingeengt und getrocknet. 5-Chlor-3-piperidin-4-yl-1*H*-indol konnte in einer Ausbeute von 2 g (100 %) mit einem Smp. von 156-158 ˚C erhalten werden.

**{4-[4-(5-Chlor-1*H*-indol-3-yl)piperidin-1-yl]-1-phenylcyclohexyl}dimethylamin**

5-Chlor-3-(1,2,3,6-tetrahydropyridin-4-yl)-1*H*-indol (218 mg, 1 mmol) und 4-Dimethylamino-4-phenylcyclohexan (217 mg, 1 mmol) wurden in trockenem 1,2-Dichlorethan (20 ml) gelöst. Diesem Gemisch wurde Eisessig (1 mmol) und Natriumtriacetoxyborhydrid (300 mg, 1,4 mmol) zugefügt. Anschließend wurde 24 h bei RT gerührt. Zur Aufarbeitung wurde 1,2-Dichlorethan abdestilliert und der Ansatz mit Wasser (10 ml) verdünnt. Der Ansatz wurde mit 5M NaOH auf pH 11 eingestellt und mit EE (3 × 20 ml) extrahiert. Die organische Phase wurde mit $Na_2SO_4$ getrocknet und eingedampft. Das Produkt war ein Gemisch aus zwei diastereoisomeren Verbindungen, die durch Chromatographie mit Methanol getrennt werden konnten. Das unpolarere Diastereoisomer wurde als farbloses Öl in einer Ausbeute von 210 mg (48 %) erhalten, das polarere Diastereoisomer als farbloses Öl in einer Ausbeute von 80 mg (18 %).

**{4-[4-(5-Chlor-1*H*-indol-3-yl)piperidin-1-yl]-1-phenylcyclohexyl}dimethylamin-dihydrochlorid**

Zur Herstellung des Dihydrochlorids wurde das unpolarere Diastereoisomer von {4-[4-(5-Chlor-1*H*-indol-3-yl) piperidin-1-yl]-1-phenylcyclohexyl}dimethylamin (210 mg, 0,48 mmol) in Ethylmethylketon (5 ml) gelöst und mit Chlortrimethylsilan (155 μl, 1,2 mmol) versetzt. Der dabei entstandene Feststoff wurde abfiltriert und getrocknet. Das Dihydrochlorid des unpolareren Amins wurde so in einer Ausbeute von 180 mg (73 %) als farbloser Feststoff mit einem Smp. von 249-251 ˚C gewonnen (**Beispiel 3**).

**Beispiel 4:**

**{4-[4-(5-Chlor-1H-indol-3-yl)-piperidin-1-yl]-1-phenyl-cyclohexyl}-dimethyl-amin; Dihydrochlorid (polareres Diastereoisomer)**

Zur Herstellung des polareren Dihydrochlorids wurde das polarere Diastereoisomer von {4-[4-(5-Chlor-1*H*-indol-3-yl)piperidin-1-yl]-1-phenylcyclohexyl}dimethylamin (80 mg, 0,18 mmol) in Ethylmethylketon (3 ml) gelöst und mit Chlortrimethylsilan (60 µl, 0,45 mmol) versetzt. Der dabei entstandene Feststoff wurde abfiltriert und getrocknet. Das Dihydrochlorid des polareren Amins wurde so in einer Ausbeute von 80 mg (86 %) als farbloser Feststoff mit einem Smp. von 198-200 ˚C gewonnen (**Beispiel 4**).

**Beispiel 5:**

**{4-[4-(1H-Indol-3-yl)-piperidin-1-yl]-1-phenyl-cyclohexyl}-dimethyl-amin; Dihydrochlorid (polareres Diastereoisomer)**

**3-Piperidin-4-yl-1*H*-indol**

3-(1,2,3,6-tetrahydropyridin-4-yl)-1*H*-indol (3 g, 16,5 mmol) wurde unter Argon in Methanol (50 ml) gelöst. Pd/Kohle (150 mg, 5 %) wurde zugegeben und das Reaktionsgemisch 8 h bei 2 bar hydriert. Zur Aufarbeitung wurde die Lösung über Kieselgel abfiltriert und mit Methanol (10 × 20 ml) gewaschen. Das Filtrat wurde eingeengt und getrocknet. 3-Piperidin-4-yl-1*H*-indol konnte in einer Ausbeute von 3,3 g (100 %) mit einem Smp. von 190-192 ˚C erhalten werden.

**{4-[4-(1*H*-Indol-3-yl)piperidin-1-yl]-1-phenylcyclohexyl}dimethylamin**

3-Piperidin-4-yl-1*H*-indol (200 mg, 1 mmol) und 4-Dimethylamino-4-phenylcyclohexanon (217 mg, 1 mmol) wurden in trockenem 1,2-Dichlorethan (20 ml) gelöst. Diesem Gemisch wurde Eisessig (1 mmol) und Natriumtriacetoxyborhydrid (300 mg, 1,4 mmol) zugefügt. Anschließend wurde 24 h bei RT gerührt. Zur Aufarbeitung wurde 1,2-Dichlorethan abdestilliert und der Ansatz mit Wasser (10 ml) verdünnt. Der Ansatz wurde mit 5M NaOH auf pH 11 eingestellt und mit EE (3 × 20 ml) extrahiert. Die organische Phase wurde mit $Na_2SO_4$ getrocknet und eingedampft. Das Produkt war ein Gemisch aus zwei diastereoisomeren Verbindungen, die durch Chromatographie mit Methanol getrennt werden konnten. Das polarere und das unpolarere Diastereoisomer wurden jeweils als farblose Öle in einer Ausbeute von 90 mg (22 %) erhalten.

**{4-[4-(1*H*-Indol-3-yl)piperidin-1-yl]-1-phenylcyclohexyl}dimethylamin-dihydrochlorid (polareres Diastereoisomer)**

Zur Herstellung des polareren Hydrochlorids wurde das polarere Diastereoisomer von {4-[4-(1*H*-Indol-3-yl)piperidin-1-yl]-1-phenylcyclohexyl}dimethylamin (90 mg, 0,22 mmol) in Ethylmethylketon (3 ml) gelöst und mit Chlortrimethylsilan (70 µl, 0,55 mmol) versetzt. Der dabei entstandene Feststoff wurde abfiltriert und getrocknet.
Das Hydrochlorid des polareren Amins wurde so in einer Ausbeute von 106 mg (100 %) als farbloser Feststoff mit einem Smp. von 247-249 ˚C gewonnen (**Beispiel 5**).

**Beispiel 6:**

**{4-[4-(1*H*-Indol-3-yl)piperidin-1-yl]-1-phenylcyclohexyl}dimethylamin-dihydrochlorid (unpolareres Diastereoisomer)**

Zur Herstellung des unpolareren Dihydrochlorids wurde das unpolarere Diastereoisomer von {4-[4-(1*H*-Indol-3-yl)piperidin-1-yl]-1-phenylcyclohexyl}dimethylamin (90 mg, 0,22 mmol) in Ethylmethylketon (3 ml) gelöst und mit Chlortrimethylsilan (70 µl, 0,55 mmol) versetzt. Der dabei entstandene Feststoff wurde abfiltriert und getrocknet. Das Dihydrochlorid des unpolareren Amins wurde so in einer Ausbeute von 106 mg (100 %) als farbloser Feststoff mit einem Smp. von 263-265 ˚C gewonnen (**Beispiel 6**).

**Beispiel 7:**

**{4-[4-(5-Methoxy-1H-Indol-3-yl)-piperidin-1-yl)-1-phenyl-cyclohexyl)-dimethylamin; Dihydrochlorid (unpolareres Diastereoisomer)**

**5-Methoxy-3-piperidin-4-yl-1*H*-indol**

5-Methoxy-3-(1,2,3,6-tetrahydropyridin-4-yl)-1*H*-indol (2 g, 9,44 mmol) wurde unter Argon in Methanol (50 ml) gelöst. Pd/Kohle (100 mg, 5 %) wurde zugegeben und das Reaktionsgemisch 8 h bei 2 bar hydriert. Zur Aufarbeitung wurde die Lösung über Kieselgel abfiltriert und mit Methanol (10 × 20 ml) gewaschen. Das Filtrat wurde eingeengt und getrocknet. 5-Methoxy-3-piperidin-4-yl-1*H*-indol konnte in einer Ausbeute von 1,9 g (88 %) mit einem Smp. von 160-162 ˚C erhalten werden.

**{4-[4-(5-Methoxy-1*H*-indol-3-yl)piperidin-1-yl]-1-phenylcyclohexyl}dimethylamin**

5-Methoxy-3-piperidin-4-yl-1*H*-indol (461 mg, 2 mmol) und 4-Dimethylamino-4-phenylcyclohexan (435 mg, 2 mmol) wurden in trockenem 1,2-Dichlorethan (40 ml) gelöst. Diesem Gemisch wurde Eisessig (2 mmol) und Natriumtriacetoxyborhydrid (600 mg, 2,8 mmol) zugefügt. Anschließend wurde 24 h bei RT gerührt. Zur Aufarbeitung wurde 1,2-Dichlorethan abdestilliert und der Ansatz mit Wasser (20 ml) verdünnt. Der Ansatz wurde mit 5M NaOH auf pH 11 eingestellt und mit EE (3 × 20 ml) extrahiert. Die organische Phase wurde mit $Na_2SO_4$ getrocknet und eingedampft. Das Produkt war ein Gemisch aus zwei diastereoisomeren Verbindungen, die durch Chromatographie mit Methanol getrennt werden konnten. Das unpolarere Diastereoisomer wurde als farbloses Öl in einer Ausbeute von 590 mg (68 %) erhalten, das polarere Diastereoisomer als farbloses Öl in einer Ausbeute von 170 mg (20 %).

**{4-[4-(5-Methoxy-1*H*-indol-3-yl)piperidin-1-yl]-1-phenylcyclohexyl}dimethylamin-dihydrochlorid**

Zur Herstellung des Dihydrochlorids wurde das unpolarere Diastereoisomer von {4-[4-(5-Methoxy-1*H*-indol-3-yl)piperidin-1-yl]-1-phenylcyclohexyl}dimethylamin (590 mg, 1,37 mmol) in Ethylmethylketon (7 ml) gelöst und mit Chlortrimethylsilan (435 µl, 3,4 mmol) versetzt. Der dabei entstandene Feststoff wurde abfiltriert und getrocknet.
Das unpolarere {4-[4-(5-Methoxy-1*H*-indol-3-yl)piperidin-1-yl]-1-phenylcyclohexyl}dimethylamin-dihydrochlorid wurde so in einer Ausbeute von 690 mg (100 %) als farbloser Feststoff mit einem Smp. von 224-226 ˚C gewonnen (**Beispiel 7**).

Beispiel 8:

**{4-[4-(5-Methoxy-1*H*-indol-3-yl)piperidin-1-yl]-1-phenylcyclohexyl}dimethylamin-dihydrochlorid (polareres Diastereoisomer)**

Zur Herstellung des Dihydrochlorids wurde das polarere Diastereoisomer von {4-[4-(5-Methoxy-1*H*-indol-3-yl) piperidin-1-yl]-1-phenylcyclohexyl}dimethylamin (170 mg, 0,4 mmol) in Ethylmethylketon (5 ml) gelöst und mit Chlortrimethylsilan (125 µl, 1,0 mmol) versetzt. Der dabei entstandene Feststoff wurde abfiltriert und getrocknet.
Das polarere {4-[4-(5-Methoxy-1*H*-indol-3-yl)piperidin-1-yl]-1-phenylcyclohexyl}dimethylamin-dihydrochlorid wurde so in einer Ausbeute von 200 mg (100 %) als farbloser Feststoff mit einem Smp. von 201-203 ˚C gewonnen (**Beispiel 8**).

**Beispiel 9:**

**{4-[3-(1H-Indol-3-yl)-piperidin-1-yl]-1-phenyl-cyclohexyl}-dimethyl-amin; Dihydrochlorid (unpolareres Diastereoisomer)**

**3-(1-Benzyl-1,2,5,6-tetrahydro-pyridin-3-yl)-1*H*-indol**

KOH (2,12 g, 37,8 mmol), Indol (1,0 g, 8,54 mmol) und 1-Benzyl-3-piperidon-hydrochlorid (4,82 g, 21,34 mmol) wurden in Methanol (20 ml) suspendiert und unter Argon 8 h auf 65 ˚C erhitzt. Bei RT wurde dem Gemisch während einer Zeit von 40 min tropfenweise Wasser (40 ml) zugesetzt. Methanol wurde abdestilliert und die wässrige Phase mit EE (3 × 20 ml) extrahiert. Die organische Phase wurde mit $Na_2SO_4$ getrocknet und eingedampft. 3-(1-Benzyl-1,2,5,6-tetrahydro-pyridin-3-yl)-1*H*-indol konnte durch Chromatographie mit Cyclohexan/EE (1 : 1) gereinigt werden. Es wurde als gelber Feststoff in einer Ausbeute von 860 mg (35 %) und einem Smp. von 105-107 ˚C erhalten.

**3-Piperidin-3-yl-1*H*-indol**

3-(1-Benzyl-1,2,5,6-tetrahydro-pyridin-3-yl)-1*H*-indol (430 mg, 1,48 mmol) wurde unter Argon in Methanol (30 ml) gelöst. Pd/Kohle (43 mg, 10 %) wurde zugegeben und das Reaktionsgemisch 16 h bei 2 bar hydriert. Zur Aufarbeitung wurde die Lösung über Kieselgel abfiltriert und mit Methanol (10 × 10 ml) gewaschen. Das Filtrat wurde eingeengt und getrocknet. 3-Piperidin-3-yl-1*H*-indol konnte in einer Ausbeute von 233 mg (79 %) als gelbes Öl erhalten werden.

**{4-[3-(1*H*-Indol-3-yl)piperidin-1-yl]-1-phenylcyclohexyl}dimethylamin**

3-Piperidin-3-yl-1*H*-indol (233 mg, 1,16 mmol) und 4-Dimethylamino-4-phenylcyclohexanon (253 mg, 1,16 mmol) wurden in trockenem 1,2-Dichlorethan (20 ml) gelöst. Diesem Gemisch wurde Eisessig (1,16 mmol) und Natriumtriacetoxyborhydrid (350 mg, 1,63 mmol) zugefügt. Anschließend wurde 24 h bei RT gerührt. Zur Aufarbeitung wurde 1,2-Dichlorethan abdestilliert und der Ansatz mit Wasser (20 ml) verdünnt. Der Ansatz wurde mit 5M NaOH auf pH 11 eingestellt und mit EE (3 × 20 ml) extrahiert. Die organische Phase wurde mit $Na_2SO_4$ getrocknet und eingedampft. Das Produkt war ein Gemisch aus zwei diastereoisomeren Verbindungen, die durch Chromatographie mit Methanol getrennt werden konnten. Das unpolarere Diastereoisomer wurde als farbloses Öl in einer Ausbeute von 208 mg (45 %) erhalten, das polarere Diastereoisomer als farbloses Öl in einer Ausbeute von 50 mg (11%).

**{4-[3-(1*H*-Indol-3-yl)piperidin-1-yl]-1-phenylcyclohexyl}dimethylamin-dihydrochlorid (unpolareres Diastereoisomer)**

Zur Herstellung des Dihydrochlorids wurde das unpolarere Diastereoisomer von {4-[3-(1*H*-Indol-3-yl)piperidin-1-yl]-1-phenylcyclohexyl}dimethylamin (208 mg, 0,52 mmol) in Ethylmethylketon (5 ml) gelöst und mit Chlortrimethylsilan, (165 µl, 1,3 mmol) versetzt. Der dabei entstandene Feststoff wurde abfiltriert und getrocknet. Das unpolarere {4-[3-(1*H*-Indol-3-yl)piperidin-1-yl]-1-phenylcyclohexyl}dimethylamin-dihydrochlorid wurde so in einer Ausbeute von 245 mg (100 %) als farbloser Feststoff mit einem Smp. von 188-190 ˚C gewonnen (**Beispiel 9**).

**Beispiel 10:**

**{4-[3-(1*H*-Indol-3-yl)piperidin-1-yl]-1-phenylcyclohexyl)dimethylamin-dihydrochlorid (polareres Diastereoisomer)**

Zur Herstellung des polareren Dihydrochlorids wurde das polarere Diastereoisomer von {4-[3-(1*H*-Indol-3-yl) piperidin-1-yl]-1-phenylcyclohexyl}dimethylamin (50 mg, 0,12 mmol) in Ethylmethylketon (3 ml) gelöst und mit Chlortrimethylsilan (40 µl, 0,3 mmol) versetzt. Der dabei entstandene Feststoff wurde abfiltriert und getrocknet. Das polarere {4-[3-(1*H*-Indol-3-yl)piperidin-1-yl]-1-phenylcyclohexyl}dimethylamin-dihydrochlorid wurde so in einer Ausbeute von 59 mg (100 %) als farbloser Feststoff mit einem Smp. von 186-188 ˚C gewonnen (**Beispiel 10**).

**<u>Beispiel 11:</u>**

**2-(4-Dimethylamino-4-phenylcyclohexyliden)-1-[3-(1*H*-Indol-3-yl)pyrrolidin-1-yl]ethanon hydrochlorid**

**2-(4-Dimethylamino-4-phenylcyclohexyliden)-1-[3-(1*H*-indol-3-yl)pyrrolidin-1-yl]ethanon**

Zu einer Lösung von 4-Dimethylamino-4-phenylcyclohexyliden-essigsäure (891 mg, 3,0 mmol) in trockenem Dimethylformamid (15 ml) wurden unter Argon nacheinander 1-Hydroxybenzotriazol (810 mg, 6,0 mmol), 3-Pyrrolidin-3-yl-1*H*-indol (558 mg, 3,0 mmol) und *N*-Methylmorpholin (0,666 ml, 6,0 mmol) gegeben. Die Lösung wurde im Eisbad gekühlt und mit Dicyclohexylcarbodiimid (1,2 g, 6,0 mmol) versetzt. Die Reaktionsmischung wurde 4 d bei RT gerührt, wobei nach und nach der Dicyclohexylharnstoff ausfiel. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit Wasser (300 ml) verdünnt, mit 5M Natronlauge (7 ml, 35 mmol) versetzt und 3 d bei 5 ˚C aufbewahrt. Dabei fiel das Rohprodukt des Amids als beigefarbener Feststoff aus (994 mg). Nach chromatographischer Trennung an Kieselgel (80 g) mit EE/Methanol (1 : 1) wurde 2-(4-Dimethylamino-4-phenylcyclohexyliden)-1-[3-(1*H*-indol-3-yl)pyrrolidin-1-yl]ethanon in einer Ausbeute von 46 % (585 mg) mit einem Smp. von 94-97 ˚C als farbloser Feststoff isoliert. - Die Verbindung wurde als Diastereoisomerengemisch erhalten.

**2-(4-Dimethylamino-4-phenylcyclohexyliden)-1-[3-(1*H*-indol-3-yl)pyrrolidin-1-yl]ethanon hydrochlorid**

2-(4-Dimethylamino-4-phenylcyclohexyliden)-1-[3-(1*H*-indol-3-yl)pyrrolidin-1-yl]ethanon (200 mg, 0,47 mmol) wurde in Ethylmethylketon (10 ml) gelöst und mit Chlortrimethylsilan (0,09 ml, 0,7 mmol) versetzt. Nach 1,5 h wurde das Hydrochlorid als farblose Verbindung mit einem Smp. von 220-223 ˚C in einer Ausbeute von 96 % (210 mg) als Diastereoisomerengemisch erhalten (**Beispiel 11**).

**Beispiele 12 und 13:**

**{1-(4-Fluorphenyl)-4-[3-(1*H*-indol-3-yl)pyrrolidin-1-yl]cyclohexyl}dimethylamin-dihydrochlorid (polareres und unpolareres Diastereoisomer)**

{1-(4-Fluorphenyl)-4-[3-(1*H*-Indol-3-yl)pyrrolidin-1-yl]cyclohexyl}dimethylamin

4-Dimethylamino-4-(4-fluorphenyl)cyclohexanon (0,701 g, 2,98 mmol) wurde unter Argon in einer Mischung aus 1,2-Dichlorethan (20 ml) und Tetrahydrofuran (15 ml) gelöst, mit 3-Pyrrolidin-3-yl-1*H*-indol (0,554 g, 2,98 mmol) und Essigsäure (0,164 ml, 2,98 mmol) versetzt. Die klare hellbraune Lösung wurde 15 min bei RT gerührt und dann mit Natriumtriacetoxyborhydrid (0,9 g, 4,2 mmol) versetzt. Nach 3 d Rühren bei RT wurde das Reaktionsgemisch eingeengt und der feste farblose Rückstand mit 1M NaOH (50 ml) und EE (40 ml) 20 min bei RT gerührt. Dabei löste sich ein Teil des Feststoffes nicht. Er wurde abfiltriert und mit EE (10 ml) gewaschen. Es wurde so das unpolarere Diastereoisomer als farbloser Feststoff (629 mg) erhalten. Vom Filtrat wurden die Phasen getrennt und die wässrige mit EE ($2 \times 40$ ml) extrahiert. Die vereinigten organischen Extrakte wurden mit Wasser (20 ml) gewaschen, über Natriumsulfat getrocknet und eingeengt. Es wurde ein fester hellbrauner Rückstand (0,558 g) erhalten, der säulenchromatographisch mit Methanol/ 30-proz. wässrigem Ammoniak (75 : 1) in die beiden Diastereoisomere getrennt wurde. Das unpolarere Diastereoisomer von {1-(4-Fluorphenyl)-4-(3-(1*H*-indol-3-yl)pyrrolidin-1-yl]cyclohexyl}dimethylamin konnte so als farbloser Feststoff mit einem Smp. von 248-250 ˚C in einer Gesamtausbeute von 0,682 g (57 %) und das polarere ebenfalls als farbloser Feststoff (0,331 g, 27 %, Fp. 201-205 ˚C) diastereoisomerenrein erhalten werden.

**{1-(4-Fluorphenyl)-4-[3-(1*H*-indol-3-yl)pyrrolidin-1-yl]cyclohexyl}dimethylamin-dihydrochlorid**

Das sehr schwer lösliche unpolarere Diastereoisomer von {1-(4-Fluorphenyl)-4-[3-(1*H*-indol-3-yl)pyrrolidin-1-yl]cyclohexyl}dimethylamin (300 mg, 0,74 mmol) wurde in einer Mischung aus Methanol (80 ml), Ethylmethylketon (30 ml) und Chloroform (20 ml) unter Erwärmen gelöst und mit 5,5M isopropanolischer Salzsäure (0,4 ml, 2,22 mmol) versetzt. Die vorher trübe Lösung wurde sofort klar. Nach 2 h wurde auf ca. 5 ml eingeengt. Nach Zugabe von Ether (10 ml) wurde über Nacht gerührt. Nach 18 h konnte das unpolarere Dihydrochlorid (**Beispiel 13**) abfiltriert werden. Es wurde als farblose, kristalline Substanz mit einem Smp. von 208-210 ˚C in einer Ausbeute von 96 % (338,8 mg) isoliert.

Zu einer Lösung des polareren Diastereoisomers von {1-(4-Fluorphenyl)-4-[3-(1*H*-indol-3-yl)pyrrolidin-1-yl]cyclohexyl}dimethylamin (330 mg, 0,81 mmol) in Ethylmethylketon (25 ml) und Methanol (5 ml) wurde 5,5M isopropanolische Salzsäure (0,44 ml, 2,43 mmol) gegeben. Nach einer Reaktionszeit von 2 h konnte das polarere Dihydrochlorid (**Beispiel 12**) in einer Ausbeute von 92 % (358 mg) mit einem Smp. von 223-230 ˚C abfiltriert werden.

**Beispiele 14 und 15:**

**4-(5-Methoxy-1*H*-indol-3-yl)-3,6-dihydro-2*H*-pyridin-1-carbonsäure-(4-dimethylamino-4-phenylcyclohexylmethyl)amid-citrat**

**4-(5-Methoxy-1*H*-indol-3-yl)-3,6-dihydro-2*H*-pyridin-1-carbonsäure-(4-dimethylamino-4-phenylcyclohexylmethyl)amid**

Zu einer Lösung von 5-Methoxy-3-(1,2,3,6-tetrahydro-pyridin-4-yl)-1H-indol (317,9 mg, 1,5 mmol) in Dioxan (10 ml) wurde (4-Dimethylamino-4-phenylcyclohexylmethyl)-carbaminsäure phenylester (528,7 mg, 1,5 mmol) gegeben. Anschließend wurde 20 h unter Rückfluss gekocht. Die Reaktionsmischung war bei RT eine Suspension. Der Feststoff wurde abfiltriert, mit kaltem Dioxan ($3 \times 1$ ml) gewaschen und getrocknet. Der Feststoff war ein Teil des polareren Diastereomers von 4-(5-Methoxy-1H-indol-3-yl)-3,6-dihydro-2H-pyridine-1-carbonsäure (4-dimethylamino-4-phenyl-cyclohexylmethyl)-amid (99 mg, Fp. 120-125 ˚C, 14 %). Die Reaktionslösung (Filtrat und Waschphasen) wurde eingeengt. Der Rückstand enthielt Phenol sowie das unpolarere und den Rest des polareren Produktes. Durch Flash-Chromatographie an Kieselgel (75 g) wurden die Diastereoisomeren getrennt und gereinigt. Als Eluent wurde Methanol (900 ml) eingesetzt. Das unpolarere Diastereoisomer von 4-(5-Methoxy-1H-indol-3-yl)-3,6-dihydro-2H-pyridine-1-carbonsäure

(4-dimethylamino-4-phenyl-cyclohexylmethyl)-amid (234 mg, Fp. 98-104 ˚C, 32 %) und ein weiterer Teil des polareren Diastereoisomers (120 mg, Fp. 108-111 ˚C, 16 %) wurden so in reiner Form gewonnen.

**4-(5-Methoxy-1*H*-indol-3-yl)-3,6-dihydro-2*H*-pyridin-1-carbonsäure-(4-dimethylamino-4-phenylcyclohexylmethyl)amid-citrat**

Das unpolarere Diastereoisomer von 4-(5-Methoxy-1H-indol-3-yl)-3,6-dihydro-2H-pyridine-1-carbonsäure (4-dimethylamino-4-pheriyl-cyclohexylmethyl)-amid (230 mg, 0,47 mmol) wurde in abs. Ethanol (8 ml) gelöst. Unter Rühren wurde bei RT die in heißem Ethanol (1 ml) gelöste Zitronensäure (91 mg, 0,47 mmol) tropfenweise zugegeben. Nach einstündigem Rühren bei RT war nur wenig Niederschlag ausgefallen. Die Reaktionsmischung wurde mit 25 ml Diethylether versetzt und erneut ca. 1h bei RT gerührt. Der Niederschlag wurde abfiltriert, mit Diethylether (3 × 2 ml) gewaschen und im Vakuum getrocknet. Das unpolarere Diastereoisomer von 4-(5-Methoxy-1*H*-indol-3-yl)-3,6-dihydro-2*H*-pyridin-1-carbonsäure-(4-dimethylamino-4-phenylcyclohexylmethyl)amid-citrat (237 mg, Fp. 142-145˚C, 74 %, **Beispiel 14**) war ein hellgelber Feststoff.

Das polarere Diastereoisomer von 4-(5-Methoxy-1*H*-indol-3-yl)-3,6-dihydro-2*H*-pyridin-1-carbonsäure-(4-dimethylamino-4-phenylcyclohexylmethyl)amid-citrat (186 mg, 0,38 mmol) wurde in abs. Ethanol (10 ml) gelöst. Unter Rühren wurde bei RT die in heißem Ethanol (1 ml) gelöste Zitronensäure (73,4 mg, 0.38 mmol) tropfenweise zugegeben. Es fiel spontan ein kräftig oranger Niederschlag aus. Der Niederschlag wurde nach 1 h abfiltriert, mit $Et_2O$ (4 × 2 ml) gewaschen und im Vakuum getrocknet. Das polarere Diastereoisomer von 4-(5-Methoxy-1*H*-indol-3-yl)-3,6-dihydro-2*H*-pyridin-1-carbonsäure-(4-dimethylamino-4-phenylcyclohexylmethyl)amid-citrat (190 mg, Fp. 110-130 ˚C, 73 %, **Beispiel 15**) war ein oranger Feststoff.

**Beispiel 16-18:**

**4-(1*H*-Indol-3-yl)piperidin-1-carbonsäure-(4-dimethylamino-4-phenylcyclo-hexylmethyl)amid-hemicitrat (Beispiel 16) bzw. -citrat (Beispiele 17 und 18)**

**4-(1*H*-Indol-3-yl)piperidin-1-carbonsäure-(4-dimethylamino-4-phenylcyclohexylmethyl)amid**

Zu einer Lösung von 3-Piperidin-4-yl-1H-indol (340,5 mg, 1,7 mmol) in Dioxan (12 ml) wurde das Diastereomerengemisch von (4-Dimethylamino-4-phenylcyclohexylmethyl)-carbaminsäure-phenylester (599 mg, 1,7 mmol) gegeben. Das Reaktionsgemisch war ab 40 ˚C eine klare Lösung. Diese wurde 24 h unter Rückfluss gekocht. Auch bei RT zeigte sich kein Niederschlag. Das Lösungsmittel wurde im Vakuum abdestilliert. Der Rückstand enthielt neben Phenol die beiden Diastereoisomeren von 4-(1*H*-Indol-3-yl)piperidin-1-carbonsäure-(4-dimethylamino-4-phenylcyclohexylmethyl)amid. Durch Flash-Chromatographie an Kieselgel (75 g) wurden die Diastereoisomeren getrennt und gereinigt. Als Eluent wurden Methanol/EE (1 : 1; 800 ml) und Methanol (500 ml) eingesetzt. Das unpolarere Diastereoisomer (281 mg, Fp. 89-93 ˚C, 36 %) und das polarere Diastereoisomer von 4-(1*H*-Indol-3-yl)piperidin-1-carbonsäure-(4-dimethylamino-4-phenylcyclohexylmethyl)amid (262 mg, Fp. 104-107 ˚C, 34 %) wurden so in reiner Form gewonnen.

**4-(1*H*-Indol-3-yl)piperidin-1-carbonsäure-(4-dimethylamino-4-phenylcyclohexylmethyl)amid-hemicitrat (Beispiel 16) bzw. -citrat (Beispiele** 17 und 18)

Das unpolarere Diastereoisomer von 4-(1*H*-Indol-3-yl)piperidin-1-carbonsäure-(4-dimethylamino-4-phenylcyclohexylmethyl)amid (280 mg, 0,61 mmol) wurde in abs. Ethanol (16 m!) und DCM (6 ml) gelöst. Unter Rühren wurde bei ca. 40 ˚C Zitronensäure (118 mg, 0,616 mmol) in einer Portion zugegeben. Nach 20 min begann ein farbloser Niederschlag auszufallen. Die Suspension wurde bei RT 24 h gerührt. Danach wurde der Niederschlag abfiltriert, mit kaltem Ethanol (2 × 1 ml) und Diethylether (4 × 2 ml) gewaschen. Man erhielt das unpolarere Diastereoisomer von 4-(1*H*-Indol-3-yl)piperidin-1-carbonsäure-(4-dimethylamino-4-phenylcyclohexylmethyl)amid-hemicitrat (241 mg, Fp. 165-168 ˚C, 61 %, **Beispiel 16**) als farblosen Feststoff.

Das Filtrat wurde auf 3 ml Lösung eingeengt und portionsweise mit Diethylether (12 ml) versetzt. Der entstandene Niederschlag wurde abfiltriert, mit Diethylether (3 × 2 ml) gewaschen und getrocknet. Es handelte sich um das unpolarere Diastereoisomer von 4-(1*H*-Indol-3-yl)piperidin-1-carbonsäure-(4-dimethylamino-4-phenylcyclohexylmethyl)amid-citrat (78 mg, Fp. 120-128 ˚C, 20 %, **Beispiel 17**) als ein grauweißer Feststoff.

Das polarere Diastereoisomer von 4-(1*H*-Indol-3-yl)piperidin-1-carbonsäure-(4-dimethylamino-4-phenylcyclohexylmethyl)amid (262 mg, 0,57 mmol) wurde in abs. Ethanol (7 ml) und DCM (2 ml) gelöst. Unter Rühren wurde bei ca. 40˚C Zitronensäure (111 mg, 0,575 mmol) in einer Portion zugegeben. Nach mehrstündigem Rühren bei RT war kein Niederschlag ausgefallen. Das Lösungsmittel wurde im Vakuum bis auf ca. 2 ml abdestilliert. Durch Zugabe von Diethylether (15 ml) fiel ein Niederschlag aus. Dieser wurde abfiltriert, mit Diethylether (3 × 2 ml) gewaschen und getrocknet. Der

cremefarbene Feststoff war das polarere Diastereoisomer von 4-(1*H*-Indol-3-yl)piperidin-1-carbonsäure-(4-dimethylamino-4-phenylcyclohexylmethyl)amid-citrat (348 mg, 94 %, **Beispiel 18**).

**Beispiele 19 und 20:**

**4-(5-Chlor-1*H*-indol-3-yl)piperidin-1-carbonsäure-(4-dimethylamino-4-phenylcyclohexylmethyl)amid-citrat (polareres Diastereoisomer; Beispiel 19) bzw. -hemicitrat (unpolareres Diastereoisomer; Beispiel 20)**

**4-(5-Chlor-1*H*-indol-3-yl)piperidin-1-carbonsäure-(4-dimethylamino-4-phenylcyclohexylmethyl)amid**

Zur Suspension von 5-Chlor-3-piperidin-4-yl-1H-indol (516 mg, 2,2 mmol) in Dioxan (20 ml) wurde das Diastereomerengemisch von (4-Dimethylamino-4-phenylcyclohexylmethyl)-carbaminsäure-phenylester (774 mg, 2,2 mmol) gegeben. Das Reaktionsgemisch war auch bei 100 °C eine Suspension. Diese wurde 40 h unter Rückfluss gekocht. Zur Aufarbeitung wurde der vorhandene Niederschlag abfiltriert, mit Dioxan (1 × 1 ml) und Diethylether (3 × 2 ml) gewaschen und im Vakuum getrocknet. Der isolierte Feststoff (183 mg, 17 %, Fp. 145-150 °C) war ein noch verunreinigter Teil des polareren Diastereoisomers von 4-(5-Chlor-1H-indol-3-yl)piperidin-1-carbonsäure-(4-dimethylamino-4-phenylcyclohexylmethyl)amid, das durch Flash-Chromatographie an Kieselgel [20 g, Eluent: Methanol (500 ml)] gereinigt wurde. Die Mutterlauge und die Waschphasen wurden im Vakuum eingeengt. Der Rückstand enthielt neben Phenol auch die beiden Diastereoisomere von 4-(5-Chlor-1*H*-indol-3-yl)piperidin-1-carbonsäure-(4-dimethylamino-4-phenylcyclohexylmethyl)amid. Durch Flash-Chromatographie an Kieselgel (75 g) wurden die Diastereoisomere getrennt und gereinigt. Als Eluent wurde Methanol/EE (1:1; 900 ml) eingesetzt. Das unpolarere Diastereoisomer (335 mg, Fp. 205-207 °C, 31 %) und das polarere Diastereoisomer von 4-(5-Chlor-1H indol-3-yl)piperidin-1-carbonsäure-(4-dimethylamino-4-phenylcyclohexylmethyl)amid (115 mg, Fp. 140-143°C, 11 %) wurden so als hellgelbe Feststoffe gewonnen.

**4-(5-Chlor-1*H*-indol-3-yl)piperidin-1-carbonsäure-(4-dimethylamino-4-phenylcyclohexylmethyl)amid-citrat (polareres Diastereoisomer; Beispiel 19) bzw. -hemicitrat (unpolareres Diastereoisomer; Beispiel 20)**

Das unpolarere Diastereoisomer von 4-(5-Chlor 1*H*-indol-3-yl)piperidin-1-carbonsäure-(4-dimethylamino-4-phenylcyclohexylmethyl)amid (327 mg, 0,66 mmol) wurde in abs. Ethanol (10 ml) und DCM (4 ml) gelöst. Unter Rühren wurde bei ca. 40 °C die Zitronensäure (129 mg, 0,67 mmol) in einer Portion zugegeben. Nach einstündigem Rühren bei RT fiel ein Niederschlag aus. Die Suspension rührte 18 h bei RT. Danach wurde diese mit Diethylether (35 ml) versetzt und erneut 2 h gerührt. Der farblose Niederschlag wurde abfiltriert, mit Diethylether (3 × 2 ml) gewaschen und im Vakuum getrocknet. Das unpolarere Hemicitrat von 4-(5-Chlor-1H-indol-3-yl)piperidin-1-carbonsäure-(4-dimethylamino-4-phenylcyclohexylmethyl)amid (355 mg, Fp. 152-156 °C, 79 %, **Beispiel 20**) war ein farbloser Feststoff.

Das polarere Diastereoisomer von 4-(5-Chlor-1*H*-indot-3-yl)piperidin-1-carbonsäure-(4-dimethylamino-4-phenylcyclohexylmethyl)amid (267 mg, 0,54 mmol) wurde in abs.

Ethanol (7 ml) gelöst. Unter Rühren wurde bei ca. 35 °C die Zitronensäure (105,1 mg, 0.55 mmol) in einer Portion zugegeben. Nach mehrstündigem Rühren bei RT war kein Niederschlag sichtbar. Die Reaktionsmischung wurde auf ca. 1 ml Lösung reduziert und portionsweise mit Diethylether (25 ml) versetzt. Der entstandene Niederschlag wurde nach 18 h abfiltriert, mit $Et_2O$ (3 × 2 ml) gewaschen und im Vakuum getrocknet. Das polarere Citrat von 4-(5-Chlor-1*H*-indol-3-yl)piperidin-1-carbonsäure-(4-dimethylamino-4-phenylcyclohexylmethyl)amid (324 mg, Fp. 125-130 °C, 88 %, **Beispiel 19**) war ein farbloser Feststoff.

Beispiele 21 und 22:

**4-(1*H*-Indol-3-yl)-3,6-dihydro-2*H*-pyridin-1-carbonsäure-(4-dimethylamino-4-phenylcyclohexylmethyl)amid-citrat**

**(4-Dimethylamino-4-phenylcyclohexylmethyl)carbaminsäure-4-nitrophenylester**

Als Alternative zum (4-Dimethylamino-4-phenylcyclohexylmethyl)carbaminsäurephenylester konnte auch (4-Dimethylamino-4-phenylcyclohexylmethyl)-carbaminsäure-4-nitrophenylester für die Synthese der Harnstoffe aus den Piperidin- und Pyrrolidin-Derivaten verwendet werden. Dieser wurde analog aus (4-Aminomethyl-1-phenyl-cyclohexyl)-dimethylamin und Chlorameisensäure-4-nitrophenylester in DCM/Pyridin hergestellt.

Zu einer Lösung von (4-Aminomethyl-1-phenyl-cyclohexyl)-dimethyl-amin (1 g, 4,3 mmol) in abs. DCM (15 ml) und Pyridin (766 µl, 9,49 mmol), wurde unter Eiswasserkühlung langsam der in abs. DCM (15 ml) gelöste Chlorameisensäure-4-nitrophenylester (913,5 mg, 4,53 mmol) getropft. Anschließend wurde 20 h bei RT gerührt. Zur Aufarbeitung

wurde die rote Reaktionsmischung mit Wasser (3 × 10 ml), mit 1 M HCl (3 × 10 ml) und mit 1 M NaOH (2 × 10 ml) gewaschen. Die organische Phase wurde mit $Na_2SO_4$ getrocknet und anschließend eingedampft. Der Rückstand war ein Gemisch der Diastereoisomere von (4-Dimethylamino-4-phenylcyclohexylmethyl)carbaminsäure-4-nitrophenylester (1,25 g, rotbraunes Öl, 73 %).

**4-(1*H*-indol-3-yl)-3,6-dihydro-2*H*-pyridin-1-carbonsäure-(4-dimethylamino-4-phenylcyclohexylmethyl)amid**

Zu einer Lösung von 3-(1,2,3,6-Tetrahydro-pyridin-4-yl)-1H-indol (411,8 mg, 2,08 mmol) in Dioxan (10 ml) wurde das Diastereoisomerengemisch von (4-Dimethylamino-4-phenylcyclohexylmethyl)carbaminsäure-4-nitrophenylester (825,5 mg, 2,08 mmol) gegeben. Das Reaktionsgemisch war ab 60 ˚C eine klare, rotbraune Lösung. Diese wurde 12 h unter Rückfluss gekocht. Zur Aufarbeitung wurde das Lösungsmittel im Vakuum abdestilliert. Der Rückstand enthielt neben Nitrophenol die beiden Diastereoisomeren von 4-(1*H*-Indol-3-yl)-3,6-dihydro-2*H*-pyridin-1-carbonsäure-(4-dimethylamino-4-phenylcyclohexylmethyl)amid. Durch Flash-Chromatographie an Kieselgel (120 g) wurden die Diastereoisomeren getrennt und gereinigt. Als Eluent wurde Methanol (2000 ml) eingesetzt. Das unpolarere Diastereoisomer (351 mg, Fp. 204-206 ˚C, 37 %) und das polarere Diastereoisomer von 4-(1*H*-Indol-3-yl)-3,6-dihydro-2*H*-pyridin-1-carbonsäure-(4-dimethylamino-4-phenylcyclohexylmethyl)amid (270 mg, Fp. 112-115 ˚C, 28 %) wurden so als hellgelbe Feststoffe gewonnen.

**4-(1*H*-Indol-3-yl)-3,6-dihydro-2*H*-pyridin-1-carbonsäure-(4-dimethylamino-4-phenylcyclohexylmethyl)amid-citrat**

Das unpolarere Diastereoisomer von 4-(1*H*-Indol-3-yl)-3,6-dihydro-2*H*-pyridin-1-carbonsäure-(4-dimethylamino-4-phenylcyclohexylmethyl)amid (337 mg, 0,738 mmol) wurde in abs. Ethanol (22 ml) und DCM (4 ml) gelöst. Unter Rühren wurde bei ca. 40 ˚C die Zitronensäure (143 mg, 0,745 mmol) in einer Portion zugegeben. Nach 10 min fiel wenig hellgelber Niederschlag aus. Die Suspension wurde 4 h bei RT gerührt. Die Lösungsmittelmenge wurde im Vakuum auf ca. 10 ml reduziert. Danach wurde die Suspension mit Diethylether (70 ml) versetzt und erneut 2 h gerührt. Der Niederschlag wurde abfiltriert, mit Diethylether (3 × 3 ml) gewaschen und im Vakuum getrocknet. Das unpolarere Diastereoisomer von 4-(1*H*-Indol-3-yl)-3,6-dihydro-2*H*-pyridin-1-carbonsäure-(4-dimethylamino-4-phenylcyclohexylmethyl)amid-citrat (393 mg, Fp. 152-155 ˚C, 82 %, **Beispiel 21**) war ein beiger Feststoff.

Das polarere Diastereoisomer von 4-(1*H*-Indol-3-yl)-3,6-dihydro-2*H*-pyridin-1-carbonsäure-(4-dimethylamino-4-phenylcyclohexylmethyl)amid (256 mg, 0,56 mmol) wurde in abs. Ethanol (10 ml) und DCM (2 ml) gelöst. Unter Rühren wurde bei ca. 35 ˚C die Zitronensäure (108,8 mg, 0,57 mmol) in einer Portion zugegeben. Nach vierstündigem Rühren bei RT waren nur braune Tropfen an der Kolbenwand sichtbar. Die überstehende Lösung wurde dekantiert und im Vakuum auf ca. 5 ml eingeengt. Bei RT wurde portionsweise Diethylether (50 ml) zugesetzt. Der entstandene Niederschlag wurde nach 2 h abfiltriert, mit Ether (2 × 3 ml) gewaschen und im Vakuum getrocknet. Das polarere Diastereoisomer von 4-(1*H*-Indol-3-yl)-3,6-dihydro-2*H*-pyridin-1-carbonsäure-(4-dimethylamino-4-phenylcyclohexylmethyl)amid-citrat (222 mg, Fp. 125-130 ˚C, 61 %, **Beispiel 22**) war ein hellbrauner Feststoff.

**Beispiele 23 und 24:**

**4-(5-Fluor-1*H*-indol-3-yl)piperidin-1-carbonsäure-(4-dimethylamino-4-phenylcyclohexylmethyl)amid-hemicitrat (Beispiel 23) bzw. -citrat (Beispiel 24)**

**4-(5-Fluor-1*H*-indol-3-yl)piperidin-1-carbonsäure-(4-dimethylamino-4-phenylcyclohexylmethyl)amid**

Zu einer Lösung von 5-Fluor-3-piperidin-4-yl-1H-indol (240,1 mg, 1,1 mmol) in Dioxan (11 ml) wurde das Diastereoisomerengemisch von (4-Dimethylamino-4-phenyl-cyclohexylmethyl)-carbaminsäure-phenylester (387,7 mg, 1,1 mmol) gegeben. Die Lösung wurde 16 h unter Rückfluss gekocht. Zur Aufarbeitung wurde das Lösungsmittel im Vakuum abdestilliert. Der Rückstand enthielt neben Phenol die beiden Diastereoisomeren von 4-(5-Fluor-1*H*-indol-3-yl)piperidin-1-carbonsäure-(4-dimethylamino-4-phenylcyclohexylmethyl)amid. Durch Flash-Chromatographie an Kieselgel (25 g) wurden die Diastereoisomere getrennt und gereinigt. Als Eluent wurde Methanol/EE (1 : 1; 900 ml) eingesetzt. Das unpolarere Diastereoisomer (150 mg, Fp. 95-98 ˚C, 29 %) und das polarere Diastereoisomer von 4-(5-Fluor-1*H*-indol-3-yl)piperidin-1-carbonsäure-(4-dimethylamino-4-phenylcyclohexylmethyl)amid (120 mg, Fp. 206-208 ˚C, 23 %) wurden so in reiner Form gewonnen.

**4-(5-Fluor-1*H*-indol-3-yl)piperidin-1-carbonsäure-(4-dimethylamino-4-phenylcyclohexylmethyl)amid-hemici-**

**trat bzw. -citrat**

Das unpolarere Diastereoisomer von 4-(5-Fluor-1*H*-indol-3-yl)piperidin-1-carbonsäure-(4-dimethylamino-4-phenylcyclohexylmethyl)amid (150 mg, 0,314 mmol) wurde in abs. Ethanol (11 ml) und DCM (2 ml) gelöst. Unter Rühren wurde bei ca. 40 ˚C die Zitronensäure (61,1 mg, 0,318 mmol) in einer Portion zugegeben. Nach 10 min fiel ein farbloser Niederschlag aus. Nach fünfstündigem Rühren bei RT wurde die Suspension über Nacht im Kühlschrank gekühlt. Nach 20 h wurde der Niederschlag abfiltriert, mit Diethylether (3 × 3 ml) gewaschen und im Vakuum getrocknet. Das unpolarere Diastereoisomer von 4-(5-Fluor-1*H*-indol-3-yl)piperidin-1-carbonsäure-(4-dimethylamino-4-phenylcyclohexylmethyl) amid-hemicitrat (117 mg, Fp. 162-166 ˚C, 56 %, **Beispiel 23**) war ein farbloser Feststoff.

Das polarere Diastereoisomer von 4-(5-Fluor-1*H*-indol-3-yl)piperidin-1-carbonsäure-(4-dimethylamino-4-phenylcyclohexylmethyl)amid (120 mg, 0,252 mmol) wurde in abs. Ethanol (2 ml) gelöst. Unter Rühren wurde bei ca. 40 ˚C die Zitronensäure (48,9 mg, 0,254 mmol) in einer Portion zugegeben. Das Reaktionsgemisch wurde 20 h bei RT gerührt. Während dieser Zeit fiel kein Niederschlag aus. Ethanol wurde bis zur Trockene abdestilliert und der Rückstand im Vakuum getrocknet. Das polarere Diastereoisomer von 4-(5-Fluor-1*H*-indol-3-yl)piperidin-1-carbonsäure-(4-dimethylamino-4-phenylcyclohexylmethyl)amid-citrat (168 mg, Fp. 110-115 ˚C, 99 %, **Beispiel 24**) war ein cremefarbener fester Schaum.

**Beispiel 25:**

**2-(4-Dimethylamlno-4-phenylcyclohexyliden)-1-[4-(1*H*-indol-3-yl)-3,6-dihydro-2*H*-pyridin-1-yl]ethanon-hydrochlorid**

**2-(4-Dimethylamino-4-phenylcyclohexyliden)-1-[4-(1*H*-indol-3-yl)-3,6-dihydro-2*H*-pyridin-1-yl]ethanon**

Zu einer Lösung von (4-Dimethylamino-4-phenyl-cyclohexyliden)essigsäure (296 mg, 1,0 mmol) in trockenem Dimethylformamid (10 ml) wurden unter Argon nacheinander 1-Hydroxybenzotriazol (270 mg, 2,0 mmol), 3-(1,2,3,6-Tetrahydropyridin-4-yl)-1*H*-indol (199 mg, 1,0 mmol) und N-Methyl-morpholin (0,222 ml, 2,0 mmol) gegeben. Die Lösung wurde im Eisbad gekühlt und mit Dicyclohexylcarbodiimid (413 mg, 2,0 mmol) versetzt. Die Reaktionsmischung wurde 7 d bei RT gerührt, wobei nach und nach der Dicyclohexylharnstoff ausfiel. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit Wasser (300 ml) verdünnt, mit 5M Natronlauge (7 ml, 35 mmol) versetzt und 16 h bei 5 ˚C aufbewahrt. Dabei fiel das Rohprodukt von 2-(4-Dimethylamino-4-phenylcyclohexyliden)-1-[4-(1*H*-indol-3-yl)-3,6-dihydro-2*H*-pyridin-1-yl]ethanon als gelber Feststoff aus. (213 mg, 49 %). Nach chromatographischer Reinigung an Kieselgel (30 g) mit EE/Methanol (3 : 1) wurde 2-(4-Dimethylamino-4-phenylcyclohexyliden)-1-[4-(1*H*-indol-3-yl)-3,6-dihydro-2*H*-pyridin-1-yl]ethanon in einer Ausbeute von 35 % (153 mg) als gelber Feststoff isoliert.

**2-(4-Dimethylamino-4-phenylcyclohexyliden)-1-[4-(1*H*-indol-3-yl)-3,6-dihydro-2*H*-pyridin-1-yl]ethanon-hydrochlorid**

2-(4-Dimethylamino-4-phenylcyclohexyliden)-1-[4-(1*H*-indol-3-yl)-3,6-dihydro-2*H*-pyridin-1-yl]ethanon (150 mg, 0,34 mmol) wurde in Ethylmethylketon (3 ml) gelöst und mit Chlortrimethylsilan (0,063 ml, 0,5 mmol) versetzt. Nach 1 h wurde das Hydrochlorid als rotbrauner Feststoff mit einem Smp. von 177-182 ˚C in einer Ausbeute von 69 % (112 mg) isoliert (**Beispiel 25**).

**Beispiel 26:**

**2-(4-Dimethylamino-4-phenylcyclohexyliden)-1-[4-(1*H*-indol-3-yl)-piperidin-1-yl]ethanon-hydrochlorid**

**2-(4-Dimethylamino-4-phenylcyclohexyliden)-1-[4-(1*H*-indol-3-yl)-piperidin-1-yl]ethanon**

Zu einer Lösung von (4-Dimethylamino-4-phenyl-cyclohexyliden)essigsäure (592 mg, 2,0 mmol) in trockenem Dimethylformamid (10 ml) wurden unter Argon nacheinander 1-Hydroxybenzotriazol (540 mg, 4,0 mmol), 3-Piperidin-4-yl-1H-indol (400 mg, 2,0 mmol) und N-Methylmorpholin (0,444 ml, 4,0 mmol) gegeben. Die Lösung wurde im Eisbad gekühlt und mit Dicyclohexylcarbodiimid (826 mg, 4,0 mmol) versetzt. Die Reaktionsmischung wurde 4 d bei RT gerührt, wobei nach und nach der Dicyclohexylharnstoff ausfiel. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fielen ölige Produkte aus, die durch Filtration nicht abgetrennt werden

konnten. Das Reaktionsgemisch wurde mit DCM (3 × 70 ml) extrahiert, die Extrakte vereinigt und die organische Phase mit Wasser (2 × 50 ml) gewaschen. Nach dem Trocknen wurde die organische Phase eingeengt, wobei als braunes, öliges. Rohprodukt 2-(4-Dimethylamino-4-phenylcyclohexyllden)-1-[4-(1*H*-indol-3-yl)-piperidin-1-yl]ethanon erhalten wurde. Nach chromatographischer Trennung an Kieselgel (70 g) mit EE/Methanol (2 : 1) wurde 2-(4-Dimethylamino-4-phenylcyclohexyliden)-1-[4-(1*H*-indol-3-yl)-piperidin-1-yl]ethanon in einer Ausbeute von 41 % (359 mg) als gelber schaumiger Feststoff isoliert.

**2-(4-Dimethylamino-4-phenylcyclohexyliden)-1-[4-(1*H*-indol-3-yl)-piperidin-1-yl]ethanon-hydrochlorid**

2-(4-Dimethylamino-4-phenylcyclohexyliden)-1-[4-(1*H*-indol-3-yl)-piperidin-1-yl]ethanon (344 mg, 0,779 mmol) wurde in Ethylmethylketon (10 ml) gelöst und mit Chlortrimethylsilan (0,15 ml, 1,2 mmol) versetzt. Nach 45 min wurde das Hydrochlorid als beigefarbener Feststoff mit einem Smp. von 178-180 ˚C in einer Ausbeute von 65 % (242 mg) isoliert (**Beispiel 26**).

**Beispiel 27:**

**1-[4-(5-Chlor-1*H*-indol-3-yl)piperidin-1-yl]-2-(4-dimethylamino-4-phenylcyclohexyliden)ethanon-hydrochlorid**

**1-[4-(5-Chlor-1*H*-indol-3-yl)piperidin-1-yl]-2-(4-dimethylamino-4-phenylcyclohexyliden)ethanon**

Zu einer Lösung von (4-Dimethylamino-4-phenyl-cyclohexyliden)essigsäure. (444 mg, 1,5 mmol) in trockenem Dimethylformamid (10 ml) wurden unter Argon nacheinander 1-Hydroxybenzotriazol (405 mg, 3,0 mmol), 5-Chlor-3-piperidin-4-yl-1*H*-indol (352 mg, 1,5 mmol) und *N*-Methylmorpholin (0,333 ml, 3,0 mmol) gegeben. Die Lösung wurde im Eisbad gekühlt und mit Dicyclohexylcarbodiimid (618 mg, 3,0 mmol) versetzt. Die Reaktionsmischung wurde 7 d bei RT gerührt, wobei nach und nach der Dicyclohexylharnstoff ausfiel. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fielen ölige Produkte aus, die durch Filtration nicht abgetrennt werden konnten. Das Reaktionsgemisch wurde mit DCM (3 × 50 ml) extrahiert, die wässrige Phase mit Wasser (300 ml) und 5M Natronlauge (7 ml, 35 mmol) versetzt und 16 h bei 5 ˚C aufbewahrt. Dabei fiel 1-[4-(5-Chlor-1*H*-indol-3-yl) piperidin-1-yl]-2-(4-dimethylamino-4-phenylcyclohexyliden)ethanon als gelber Feststoff (167 mg, 23 %) mit einem Smp. von 115-119 ˚C aus.

**1-[4-(5-Chlor-1*H*-indol-3-yl)piperidin-1-yl]-2-(4-dimethylamino-4-phenylcyclohexyliden)ethanon-hydrochlorid**

1-[4-(5-Chlor-1*H*-indol-3-yl)piperidin-1-yl]-2-(4-dimethylamino-4-phenylcyclohexyliden)ethanon (167 mg, 0,35 mmol) wurde in Ethylmethylketon (5 ml) gelöst und mit Chlortrimethylsilan (0,067 ml, 0,53 mmol) versetzt. Nach 50 min wurde das Hydrochlorid als beigefarbene Verbindung mit einem Smp. von 188-190 ˚C in einer Ausbeute von 73 % (131 mg) isoliert (**Beispiel 27**).

**Beispiel 28:**

**2-(4-Dimethylamino-4-phenylcyclohexyliden)-1-[4-(5-methoxy-1*H*-indol-3-yl)-3,6-dihydro-2*H*-pyridin-1-yl] ethanon-hydrochlorid**

**2-(4-Dimethylamino-4-phenylcyclohexyliden)-1-[4-(5-methoxy-1*H*-indol-3-yl)-3,6-dihydro-2*H*-pyridin-1-yl] ethanon**

Zu einer Lösung von (4-Dimethylamino-4-phenyl-cyclohexyliden)-essigsäure (444 mg, 1,5 mmol) in trockenem Dimethylformamid (10 ml) wurden unter Argon nacheinander 1-Hydroxybenzotriazol (405 mg, 3,0 mmol), 5-Methoxy-3-(1,2,3,6-tetrahydropyridin-4-yl)-1*H*-indol (345 mg, 1,5 mmol) und *N*-Methylmorpholin (0,333 ml, 3,0 mmol) gegeben. Die Lösung wurde im Eisbad gekühlt und mit Dicyclohexylcarbodiimid (618 mg, 3,0 mmol) versetzt. Die Reaktionsmischung wurde 4 d bei RT gerührt, wobei nach und nach der Dicyclohexylharnstoff zusammen mit 2-(4-Dimethylamino-4-phenytcyclohexyliden)-1-[4-(5-methoxy-1*H*-indol-3-yl)-3,6-dihydro-2H-pyridin-1-yl]ethanon ausfiel. Die Aufarbeitung des Ansatzes erfolgte durch Abtrennen der ausgefallenen Reaktionsprodukte (605 mg) durch Filtration und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit Wasser (400 ml) verdünnt, mit 5M Natronlauge (7 ml, 35 mmol) versetzt und 16 h bei 5 ˚C aufbewahrt. Dabei fiel weiteres 2-(4-Dimethylamino-4-phenylcyclohexyliden)-1-[4-(5-methoxy-1*H*-indol-3-yl)-3,6-dihydro-2*H*-pyridin-1-yl]ethanon als gelbes festes Rohprodukt aus

(283 mg).

Nach chromatographischer Trennung des Gemisches aus 2-(4-Dimethylamino-4-phenylcyclohexyliden)-1-[4-(5-methoxy-1*H*-indol-3-yl)-3,6-dihydro-2*H*-pyridin-1-yl]ethanon und Dicyclohexylharnstoff an Kieselgel (40 g) mit EE/Methanol (4 : 1) und Methanol wurden 2-(4-Dimethylamino-4-phenylcyclohexyliden)-1-[4-(5-methoxy-1*H*-indol-3-yl)-3,6-dihydro-2*H*-pyridin-1-yl]ethanon in einer Ausbeute von 24 % (172 mg) als farbloser Feststoff mit einem Smp. von 215-220 ˚C erhalten. Die Amidfraktion, die aus der wässrigen Phase isoliert wurde, ließ sich durch Auswaschen mit Methanol (40 ml) reinigen. 2-(4-Dimethylamino-4-phenylcyclohexyliden)-1-[4-(5-methoxy-1*H*-indol-3-yl)-3,6-dihydro-2*H*-pyridin-1-yl]ethanon blieb als farbloser Feststoff zurück (120 mg, 17 %).

**2-(4-Dimethylamino-4-phenylcyclohexyliden)-1-[4-(5-methoxy-1*H*-indol-3-yl)-3,6-dihydro-2*H*-pyridin-1-yl] ethanon-hydrochlorid**

2-(4-Dimethylamino-4-phenylcyohexyliden)1-[4-(5-methoxy-1*H*-indol-3-yl)3,6-dihydro-2H-pyridin-1-yl]ethanon (252 mg, 0,536 mmol) wurde in Ethanol (20 ml) suspendiert und mit 5M isopropanolischer Salzsäure (0,214 ml, 1,07 mmol) versetzt. Aus der klaren Lösung fiel das Hydrochlorid aus und konnte nach 1 h als farbloser Feststoff (222 mg, 82 %) mit einem Smp. von 175-178 ˚C isoliert werden (**Beispiel 28**).

**Beispiele 29 und 30:**

**4-(1*H*-Indol-3-yl)piperidin-1-carbonsäure-(4-dimethylamino-4-phenylcyclohexyl)-amid-hydrochlorid**

**4-(1*H*-Indol-3-yl)piperidin-1-carbonsäure-(4-dimethylamino-4-phenylcyclohexyl)amid**

Zu einer Lösung von 3-Piperidin-4-yl-1H-indol (200 mg, 1,0 mmol) in Dioxan (10 ml) wurde das unpolarere Diastereoisomer von 4-(1*H*-Indol-3-yl)piperidin-1-carbonsäure-(4-dimethylamino-4-phenylcyclohexyl)amid (338 mg, 1,0 mmol) gegeben.

Anschließend wurde 32 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 × 20 ml) extrahiert. Die organische Phase wurde mit $Na_2SO_4$ getrocknet und eingedampft. Das unpolarere 4-(1*H*-Indol-3-yl)piperidin-1-carbonsäure-(4-dimethylamino-4-phenylcyclohexyl)amid konnte aus Methanol (5 ml) umkristallisiert werden. Es wurde in einer Ausbeute von 125 mg (28 %) als farbloser Feststoff erhalten.

Zu einer Lösung von 3-Piperidin-4-yl-1H-indol (187 mg, 0,93 mmol) in Dioxan (10 ml) wurde das polarere Diastereoisomer von (4-Dimethylamino-4-phenyl-cyclohexyl)-carbaminsäure-phenylester (316 mg, 0,93 mmol) gegeben. Anschließend wurde 32 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 × 15 ml) extrahiert. Die organische Phase wurde mit $Na_2SO_4$ getrocknet und eingedampft. Das polarere Diastereoisomer von 4-(1*H*-Indol-3-yl)piperidin-1-carbonsäure-(4-dimethylamino-4-phenylcyclohexyt)amid konnte aus Methanol (5 ml) umkristallisiert werden. Es wurde in einer Ausbeute von 210 mg (51 %) als farbloser Feststoff erhalten.

**4-(1*H*-Indol-3-yl)piperidin-1-carbonsäure-(4-dimethylamino-4-phenylcyclohexyl)-amid-hydrochlorid**

Zur Herstellung des unpolareren Diastereoisomers von 4-(1H-Indol-3-yl)piperidin-1-carbonsäure-(4-dimethylamino-4-phenylcyclohexyl)amid-hydrochlorid wurde das unpolarere Diastereoisomer von 4-(1*H*-Indol-3-yl)piperidin-1-carbonsäure-(4-dimethylamino-4-phenylcyclohexyl)amid (125 mg, 0,28 mmol) in Ethylmethylketon (3 ml) gelöst und mit Chlortrimethylsilan (54 µl, 0,42 mmol) versetzt. Der dabei entstandene Feststoff wurde abfiltriert und getrocknet. Das Hydrochlorid des unpolareren Amids wurde so in einer Ausbeute von 135 mg (100 %) als farbloser Feststoff (**Beispiel 29**) mit einem Smp. von 210-214 ˚C gewonnen.

Zur Herstellung des polareren Diastereoisomers von 4-(1*H*-Indol-3-yl)piperidin-1-carbonsäure-(4-dimethylamino-4-phenylcyclohexyl)amid-hydrochlorid wurde das polarere 4-(1*H*-Indol-3-yl)piperidin-1-carbonsäure-(4-dimethylamino-4-phenylcyclohexyl)amid(210 mg, 0,47 mmol) in Ethylmethylketon (5 ml) gelöst und mit Chlortrimethylsilan (91 µl, 0,71 mmol) versetzt. Der dabei entstandene Feststoff wurde abfiltriert und getrocknet. Das Hydrochlorid des polareren Amids wurde so in einer Ausbeute von 227 mg (100 %) als farbloser Feststoff (**Beispiel 30**) mit einem Smp. von 175-177 ˚C gewonnen.

**Beispiel 31:**

**4-(5-Chlor-1*H*-indol-3-yl)piperidin-1-carbonsäure-(4-dimethylamino-4-phenylcyclohexyl)amid-hydrochlorid**

**4-(5-Chor-1*H*-indol-3-yl)piperidin-1-carbonsäure-(4-dimethylamino-4-phenylcyclohexyl)amid (unpolareres Diastereoisomer)**

Zu einer Lösung von 5-Chlor-3-piperidin-4-yl-1H-indol (218 mg, 1,0 mmol) in Dioxan (10 ml) wurde das unpolarere Diastereoisomer von (4-Dimethylamino-4-phenylcyclohexyl)-carbaminsäure-phenylester (338 mg, 1,0 mmol) gegeben. Anschließend wurde 32 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 × 20 ml) extrahiert. Die organische Phase wurde mit $Na_2SO_4$ getrocknet und eingedampft. Das unpolarere Diastereoisomer von 4-(5-Chor-1*H*-indol-3-yl)piperidin-1-carbonsäure-(4-dimethylamino-4-phenylcyclohexyl)-amid konnte aus Methanol (5 ml) umkristallisiert werden. Es wurde in einer Ausbeute von 120 mg (25 %) als farbloser Feststoff erhalten.

**4-(5-Chlor-1*H*-indol-3-yl)piperidin-1-carbonsäure-(4-dimethylamino-4-phenylcyclohexyl)amid-hydrochlorid**

Zur Herstellung von 4-(5-Chlor-1*H*-indol-3-yl)piperidin-1-carbonsäure-(4-dimethylamino-4-phenylcyclohexyl) amid-hydrochlorid wurde das unpolarere Diastereoisomer von 4-(5-Chlor-1*H*-indol-3-yl)piperidin-1-carbonsäure-(4-dimethylamino-4-phenylcyclohexyl)amid (120 mg, 0,25 mmol) in Ethylmethylketon (3 ml) gelöst und mit Chlortrimethylsilan (48 µl, 0,38 mmol) versetzt. Der dabei entstandene Feststoff wurde abfiltriert und getrocknet. Das unpolarere Diastereoisomer von 4-(5-Chlor-1H-indol-3-yl)piperidin-1-carbonsäure-(4-dimethylamino-4-phenylcyclohexyl)amid-hydrochlorid wurde so in einer Ausbeute von 129 mg (100 %) als farbloser Feststoff (**Beispiel 31**) mit einem Smp. von 198-200 ˚C gewonnen.

**Beispiel 32:**

**4-(5-Methoxy-1*H*-indol-3-yl)piperidin-1-carbonsäure-(4-dimethylamino-4-phenylcyclohexyl)amid-hydrochlorid**

**4-(5-Methoxy-1*H*-indol-3-yl)piperidin-1-carbonsäure-(4-dimethylamino-4-phenylcyclohexyl)amid (unpolareres Diastereoisomer)**

Zu einer Lösung von 5-Methoxy-3-piperidin-4-yl-1H-indol (230 mg, 1,0 mmol) in Dioxan (10 ml) wurde das unpolarere Diastereoisomer von (4-Dimethylamino-4-phenyl-cyclohexyl)-carbaminsäure-phenylester (338 mg, 1,0 mmol) gegeben. Anschließend wurde 32 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 × 20 ml) extrahiert. Die organische Phase wurde mit $Na_2SO_4$ getrocknet und eingedampft. Das unpolarere Diastereoisomer von 4-(5-Methoxy-1*H*-indol-3-yl)piperidin-1-carbonsäure-(4-dimethylamino-4-phenylcyclohexyl)amid konnte aus Methanol (5 ml) umkristallisiert werden. Es wurde in einer Ausbeute von 170 mg (36 %) als farbloser Feststoff erhalten.

**4-(5-Methoxy-1*H*-indol-3-yl)piperidin-1-carbonsäure-(4-dimethylamino-4-phenylcyclohexyl)amid-hydrochlorid (unpolareres Diastereoisomer)**

Zur Herstellung des Hydrochlorids wurde das unpolarere Diastereoisomer von 4-(5-Methoxy-1*H*-indol-3-yl) piperidin-1-carbonsäure-(4-dimethylamino-4-phenylcyclohexyl)amid (170 mg, 0,36 mmol) in Ethylmethylketon (4 ml) gelöst und mit Chlortrimethylsilan (68 µl, 0,54 mmol) versetzt. Der dabei entstandene Feststoff wurde abfiltriert und getrocknet. Das Hydrochlorid des unpolareren Diastereoisomers von 4-(5-Methoxy-1*H*-indol-3-yl)piperidin-1-carbonsäure-(4-dimethylamino-4-phenylcyclohexyl)amid wurde so in einer Ausbeute von 183 mg (100 %) als farbloser Feststoff (**Beispiel 32**) mit einem Smp. von 163-165 ˚C gewonnen.

**Beispiele 33 und 34:**

**4-(5-Fluor-1*H*-indol-3-yl)piperidin-1-carbonsäure-(4-dimethylamino-4-phenylcyclohexyl)amid-hydrochlorid**

**4-(5-Fluor-1*H*-indol-3-yl)piperidin-1-carbonsäure-(4-dimethylamino-4-phenylcyclohexyl)amid**

Zu einer Lösung von 5-Fluor 3-piperidin-4-yl-1H-indol (208 mg, 0,95 mmol) in Dioxan (10 ml) wurde das unpolarere Diastereoisomer von (4-Dimethylamino-4-phenylcyclohexyl)-carbaminsäure-phenylester (322 mg, 0,95 mmol) gegeben. Anschließend wurde 32 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 × 20 ml) extrahiert. Die organische Phase wurde mit $Na_2SO_4$ getrocknet und eingedampft. Das unpolarere Diastereoisomer von 4-(5-Fluor-1*H*-indol-3-yl)piperidin-1-carbonsäure-(4-dimethylamino-4-phenylcyclohexyl)-amid konnte aus Methanol (5 ml) umkristallisiert werden. Es wurde in einer Ausbeute von 220 mg (50 %) als farbloser Feststoff erhalten.

Zu einer Lösung von 5-Fluor-3-piperidin-4-yl-1H-indol (208 mg, 0,95 mmol) in Dioxan (10 ml) wurde das polarere Diastereoisomer von (4-Dimethylamino-4-phenylcyclohexyl)-carbaminsäure-phenylester (322 mg, 0,95 mmol) gegeben. Anschließend wurde 32 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 × 15 ml) extrahiert. Die organische Phase wurde mit $Na_2SO_4$ getrocknet und eingedampft. Das polarere Diastereoisomer von 4-(5-Fluor-1*H*-indol-3-yl)piperidin-1-carbonsäure-(4-dimethylamino-4-phenylcyclohexyl)amid konnte aus Methanol (5 ml) umkristallisiert werden. Es wurde in einer Ausbeute von 230 mg (52 %) als farbloser Feststoff erhalten.

**4-(5-Fluor-1*H*-indol-3-yl)piperidin-1-carbonsäure-(4-dimethylamino-4-phenylcyclohexyl)amid-hydrochlorid (Beispiele 33 und 34)**

Zur Herstellung des Hydrochlorids wurde das unpolarere Diastereoisomer von 4-(5-Fluor-1*H*-indol-3-yl)piperidin-1-carbonsäure-(4-dimethylamino-4-phenylcyclohexyl)-amid (220 mg, 0,48 mmol) in Ethylmethylketon (5 ml) gelöst und mit Chlortrimethylsilan (92 µl, 0,71 mmol) versetzt. Der dabei entstandene Feststoff wurde abfiltriert und getrocknet. Das Hydrochlorid des unpolareren Diastereoisomers von 4-(5-Fluor-1*H*-indol-3-yl)piperidin-1-carbonsäure-(4-dimethylamino-4-phenylcyclohexyl)amid wurde so in einer Ausbeute von 237 mg (100 %) als farbloser Feststoff (**Beispiel 33**) mit einem Smp. von 167-170 ˚C gewonnen.

Zur Herstellung des Hydrochlorids wurde das polarere Diastereoisomer von 4-(5-Fluor-1*H*-indol-3-yl)piperidin-1-carbonsäure-(4-dimethylamino-4-phenylcyclohexyl)-amid (230 mg, 0,5 mmol) in Ethylmethylketon (5 ml) gelöst und mit Chlortrimethylsilan (96 µl, 0,75 mmol) versetzt. Der dabei entstandene Feststoff wurde abfiltriert und getrocknet. Das Hydrochlorid des polareren Diastereoisomers von 4-(5-Fluor-1*H*-indol-3-yl)piperidin-1-carbonsäure-(4-dimethylamino-4-phenylcyclohexyl)amid wurde so in einer Ausbeute von 248 mg (100 %) als gelber Feststoff (**Beispiel 34**) mit einem Smp. von 170-172 ˚C gewonnen.

**Beispiele 35 und 36:**

**3-(5-Chlor-1*H*-indol-3-yl)piperidin-1-carbonsäure-(4-dimethylamino-4-phenylcyclohexyl)amid-hydrochlorid**

**3-(5-Chor-1*H*-Indol-3-yl)piperidin-1-carbonsäure-(4-dimethylamino-4-phenylcyclohexyl)amid**

Zu einer Lösung von 5-Chlor-3-piperidin-3-yl-1H-indol (200 mg, 0,85 mmol) in Dioxan (10 ml) wurde das unpolarere Diastereoisomer von (4-Dimethylamino-4-phenylcyclohexyl)-carbaminsäure-phenylester (288 mg, 0,85 mmol) gegeben. Anschließend wurde 32 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 × 15 ml) extrahiert. Die organische Phase wurde mit $Na_2SO_4$ getrocknet und eingedampft. Das unpolarere Diastereoisomer von 3-(5-Chor-1*H*-indol-3-yl)piperidin-1-carbonsäure-(4-dimethylamino-4-phenylcyclohexyl)amid konnte durch Säulenchromatographie mit Methanol gereinigt werden. Es wurde in einer Ausbeute von 217 mg (53 %) als farbloser Feststoff erhalten.

Zu einer Lösung von 5-Chlor-3-piperidin-3-yl-1H-indol (200 mg, 0,85 mmol) in Dioxan (10 ml) wurde das polarere Diastereoisomer von (4-Dimethylamino-4-phenylcyclohexyl)-carbaminsäure-phenylester (288 mg, 0,85 mmol) gegeben. Anschließend wurde 32 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 × 15 ml) extrahiert. Die organische Phase wurde mit $Na_2SO_4$ getrocknet und eingedampft. Das polarere Diastereoisomer von 3-(5-Chor-1*H*-indol-3-yl)piperidin-1-carbonsäure-(4-dimethylamino-4-phenylcyclohexyl)amid konnte durch Säulenchromatographie mit Methanol gereinigt werden. Es wurde in einer Ausbeute von 86 mg (21 %) als farbloser Feststoff erhalten.

**3-(5-Chlor-1*H*-indol-3-yl)piperidin-1-carbonsäure-(4-dimethylamino-4-phenylcyclohexyl)amid-hydrochlorid**

Zur Herstellung des Hydrochlorids wurde das unpolarere Diastereoisomer von 3-(5-Chor-1*H*-indol-3-yl)piperidin-1-carbonsäure-(4-dimethylamino-4-phenylcyclohexyl)amid (217 mg, 0,45 mmol) in Ethylmethylketon (5 ml) gelöst und mit Chlortrimethylsilan (86 µl, 0,68 mmol) versetzt. Der dabei entstandene Feststoff wurde abfiltriert und getrocknet. Das Hydrochlorid des unpolareren Diastereoisomers von 3-(5-Chor-1*H*-indol-3-yl)piperidin-1-carbonsäure-(4-dimethylamino-4-phenylcyclohexyl)amid wurde so in einer Ausbeute von 233 mg (100 %) als farbloser Feststoff (**Beispiel 35**)

mit einem Smp. von 195-198 °C gewonnen.

Zur Herstellung des Hydrochlorids wurde das polarere Diastereoisomer von 3-(5-Chor-1*H*-indol-3-yl)piperidin-1-carbonsäure-(4-dimethylamino-4-phenylcyclohexyl)amid (86 mg, 0,18 mmol) in Ethylmethylketon (3 ml) gelöst und mit Chlortrimethylsilan (34 µl, 0,27 mmol) versetzt. Der dabei entstandene Feststoff wurde abfiltriert und getrocknet. Das Hydrochlorid des polareren Diastereoisomers von 3-(5-Chor-1*H*-indol-3-yl)piperidin-1-carbonsäure-(4-dimethylamino-4-phenylcyclohexyl)amid wurde so in einer Ausbeute von 92 mg (100 %) als farbloser Feststoff (**Beispiel 36**) mit einem Smp. von 158-160 °C gewonnen.

**Beispiele 37 und 38:**

**3-(5-Methoxy-1*H*-indol-3-yl)piperidin-1-carbonsäure-(4-dimethylamino-4-phenylcyclohexyl)amid-hydrochlorid**

**3-(5-Methoxy-1*H*-indol-3-yl)piperidin-1-carbonsäure-(4-dimethylamino-4-phenylcyclohexyl)amid**

Zu einer Lösung von 5-Methoxy-3-piperidin-3-yl-1 H-indol (225 mg, 0,98 mmol) in Dioxan (10 ml) wurde das unpolarere Diastereoisomer (4-Dimethylamino-4-phenylcyclohexyl)-carbaminsäure-phenylester (330 mg, 0,98 mmol) gegeben. Anschließend wurde 32 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 × 15 ml) extrahiert. Die organische Phase wurde mit $Na_2S0_4$ getrocknet und eingedampft. Das unpolarere Diastereoisomer von 3-(5-Methoxy-1*H*-indol-3-yl)piperidin-1-carbonsäure-(4-dimethylamino-4-phenylcyclohexyl)amid konnte durch Säulenchromatographie mit Methanol gereinigt werden. Es wurde in einer Ausbeute von 244 mg (53 %) als farbloser Feststoff erhalten.

Zu einer Lösung von 5-Methoxy-3-piperidin-3-yl-1 H-indol (225 mg, 0,98 mmol) in Dioxan (10 ml) wurde das polarere Diastereoisomer von (4-Dimethylamino-4-phenylcyclohexyl)-carbaminsäure-phenylester (330 mg, 0,98 mmol) gegeben. Anschließend wurde 32 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 × 15 ml) extrahiert. Die organische Phase wurde mit $Na_2SO_4$ getrocknet und eingedampft. Das polarere Diastereoisomer von 3-(5-Methoxy-1*H*-indol-3-yl)piperidin-1-carbonsäure-(4-dimethylamino-4-phenylcyclohexyl)amid konnte durch Säulenchromatographie mit Methanol gereinigt werden. Es wurde in einer Ausbeute von 220 mg (47 %) als farbloser Feststoff erhalten.

**3-(5-Methoxy-1*H*-indol-3-yl)piperidin-1-carbonsäure-(4-dimethylamino-4-phenylcyclohexyl)amid-hydrochlorid**

Zur Herstellung des Hydrochlorids wurde das unpolarere Diastereoisomer von 3-(5-Methoxy-1*H*-indol-3-yl)piperidin-1-carbonsäure-(4-dimethylamino-4-phenylcyclohexyl)amid (244 mg, 0,51 mmol) in Ethylmethylketon (5 ml) gelöst und mit Chlortrimethylsilan (98 µl, 0,77 mmol) versetzt. Der dabei entstandene Feststoff wurde abfiltriert und getrocknet. Das Hydrochlorid des unpolareren Diastereoisomers von 3-(5-Methoxy-1*H*-indol-3-yl)piperidin-1-carbonsäure-(4-dimethylamino-4-phenylcyclohexyl)amid wurde so in einer Ausbeute von 262 mg (100 %) als farbloser Feststoff (**Beispiel 37**) mit einem Smp. von 160-162 °C gewonnen.

Zur Herstellung des Hydrochlorids wurde das polarere Diastereoisomer von 3-(5-Methoxy-1*H*-indol-3-yl)piperidin-1-carbonsäure-(4-dimethylamino-4-phenylcyclohexyl)amid (220 mg, 0,46 mmol) in Ethylmethylketon (5 ml) gelöst und mit Chlortrimethylsilan (89 µl, 0,70 mmol) versetzt. Der dabei entstandene Feststoff wurde abfiltriert und getrocknet. Das Hydrochlorid des polareren Diastereoisomers von 3-(5-Methoxy-1*H*-indol-3-yl)piperidin-1-carbonsäure-(4-dimethylamino-4-phenylcyclohexyl)amid wurde so in einer Ausbeute von 236 mg (100 %) als farbloser Feststoff (**Beispiel 38**) mit einem Smp. von 175-177 °C gewonnen.

**Beispiel 39:**

**2-(4-Dimethylamino-4-phenylcylohexyl)-1 -[3-(1*H*-indol-3-yl)pyrro-lidin-1-yl]ethanon-hydrochlorid**

**2-(4-Dimethylamino-4-phenylcyclohexyl)-1-[3-(1*H*-indol-3-yl)pyrro-lidin-1-yl]ethanon**

Zu einer Lösung von (4-Dimethylamino-4-phenyl-cyclohexyl)-essigsäure (897 mg, 3,0 mmol) in trockenem Dimethylformamid (15 ml) wurden unter Argon nacheinander 1-Hydroxybenzotriazol (810 mg, 6,0 mmol), 3-Pyrrolidin-3-yl-1*H*-indol (558 mg, 3,0 mmol) und *N*-Methylmorpholin (0,666 ml, 6,0 mmol) gegeben. Die Lösung wurde im Eisbad gekühlt und mit Dicyclohexylcarbodiimid (1,2 g, 6,0 mmol) versetzt. Die Reaktionsmischung wurde 4 d bei RT gerührt, wobei nach und nach der Dicyclohexylharnstoff ausfiel. Die Aufarbeitung des Ansatzes erfolgte durch Abtrennen des ausgefallenen Harnstoffes und das Eintragen des Filtrats in eine Mischung aus gesättigter NaCl-Lösung (40 ml) und

gesättigter $NaHCO_3$-Lösung (10 ml). Dabei fiel noch weiterer Harnstoff aus, der abgetrennt wurde. Die wässrige Phase wurde mit Wasser (300 ml) verdünnt, mit 5M Natronlauge (7 ml, 35 mmol) versetzt und 3 d bei 5 ˚C aufbewahrt. Dabei fiel das Rohprodukt von 2-(4-Dimethylamino-4-phenylcyclohexyl)-1-[3-(1*H*-indol-3-yl)pyrro-lidin-1-yl]ethanon als beigefarbener Feststoff aus (1,11 g). Nach chromatographischer Trennung an Kieselgel (80 g) mit Methanol wurde 2-(4-Dimethylamino-4-phenylcyclohexyl)-1-[3-(1*H*-indol-3-yl)pyrro-lidin-1-yl]ethanon in einer Ausbeute von 40 % (521 mg) mit einem Smp. von 98-100 ˚C als farbloser Feststoff isoliert.

**2-(4-Dimethylamino-4-phenylcyclohexyl)-1-[3-(1*H*-indol-3-yl)pyrro-lidin-1-yl]ethanon-hydrochlorid**

2-(4-Dimethylamino-4-phenylcyclohexyl)-1-[3-(1*H*-indo)-3-yl)pyrro-lidin-1-yl]ethanon (510 mg, 1,19 mmol) wurde in Ethylmethylketon (15 ml) gelöst und mit Chlortrimethylsilan (0,226 ml, 1,8 mmol) versetzt. Nach 1 h wurde das Hydrochlorid als farblose Verbindung mit einem Smp. von 180-191 ˚C in-einer Ausbeute von 94 % (523 mg) isoliert (**Beispiel 39**).

**Beispiele 40 und 41:**

**2-[4-Dimethylamino-4-(4-fluorphenyl)cyclohexyl]-1-[3-(1*H*-indol-3-yl)pyrrolidin-1-yl]ethanon-hydrochlorid**

**2-[4-Dimethylamino-4-(4-fluorphenyl)cyclohexyl]-1-[3-(1*H*-indol-3-yl)pyrrolidin-1-yl]ethanon**

Zu einer Lösung von 2-[4-Dimethylamino-4-(4-fluor-phenyl)-cyclohexylidene]-1-[3-(1H-indol-3-yl)-pyrrolidin-1-yl]-ethanone (605 mg, 1,36 mmol) in abs. Methanol (100 ml) wurde Palladium auf Kohle (5 %, 60 mg) gegeben. Die Reaktionsmischung wurde 23 h bei einem Druck von 3 bar bei RT hydriert. Der Katalysator wurde über Celite abgetrennt und das Filtrat eingeengt. Nach chromatographischer Auftrennung des Rückstandes (579 mg) an Kieselgel (50 g) mit EE/Methanol (2 : 1) wurde das unpolarere Diastereoisomer von 2-[4-Dimethylamino-4-(4-fluorphenyl)cyclohexyl]-1-[3-(1H-indol-3-yl)pyrrolidin-1-yl]ethanon in einer Ausbeute von 31 % (190 mg) und das polarere Diastereoisomer von 2-[4-Dimethylamino-4-(4-fluorphenyl)cyclohexyl]-1-[3-(1*H*-indol-3-yl)pyrrolidin-1-yl]ethanon in einer Ausbeute von 64 % (386 mg) isoliert. Beide Verbindungen waren farblose Öle.

**2-[4-Dimethylamino-4-(4-fluorphenyl)cyclohexyl]-1 -[3-(1*H*-indol-3-yl)pyrrolidin-1-yl]ethanon-hydrochlorid**

Das unpolarere Diastereoisomer von 2-[4-Dimethylamino-4-(4-fluorphenyl)cyclohexyl]-1-[3-(1*H*-indol-3-yl)pyrrolidin-1-yl]ethanon (190 mg, 0,43 mmol) wurde in Ethylmethylketon (10 ml) gelöst und mit Chlortrimethylsilan (0,08 ml, 0,63 mmol) versetzt. Nach einer Reaktionszeit von 1,5 h wurde das Hydrochlorid als farbloser Feststoff in einer Ausbeute von 100 % (208 mg) mit einem Smp. von 168-173 ˚C erhalten (**Beispiel 40**).

Das polarere Diastereoisomer von 2-[4-Dimethylamino-4-(4-fluorphenyl)cyclohexyl]-1-[3-(1H-indol-3-yl)pyrrolidin-1-yl]ethanon (329 mg, 0,73 mmol) wurde in Ethylmethylketon (10 ml) gelöst, mit Trimethylchlorsilan (0,14 ml, 1,1 mmol) versetzt und 1,5 h bei RT gerührt. Das ausgefallene Hydrochlorid wurde in einer Ausbeute von 93 % (329 mg) und einem Smp. von 170-177 ˚C isoliert (**Beispiel 41**).

**Beispiel 42:**

**3-(5-Fluor-1*H*-Indol-3-yl)piperidin-1-carbonsäure-(4-dimethylamino-4-phenylcyclohexyl)amid-hydrochlorid (polareres Diastereoisomer)**

**3-(5-Fluor-1*H*-Indol-3-yl)piperidin-1-carbonsäure-(4-dimethylamino-4-phenylcyclohexyl)amid (polareres Diastereoisomer)**

Zu einer Lösung von 5-Fluor-3-piperidin-3-yl-1 H-indol (218 mg, 1 mmol) in Dioxan (10 ml) wurde das polarere Diastereoisomer von (4-Dimethylamino-4-phenylcyclohexyl)-carbaminsäure-phenylester (338 mg, 1 mmol) gegeben. Anschließend wurde 32 h unter Rückfluss gekocht. Zur Aufarbeitung wurde Dioxan abdestilliert und der Ansatz mit Wasser (10 ml) verdünnt. Der Ansatz wurde mit 5M NaOH auf pH 11 eingestellt und mit EE (3 $\times$ 15 ml) extrahiert. Die organische Phase wurde mit $Na_2SO_4$ getrocknet und eingedampft. Das polarere Diastereoisomer von 3-(5-Fluor-1*H*-Indol-3-yl)piperidin-1-carbonsäure-(4-dimethylamino-4-phenylcyclohexyl)amid konnte durch Säulenchromatographie [Kieselgel 60 (25 g); Methanol (250 ml)] gereinigt werden. Es wurde in einer Ausbeute von 200 mg (43 %) als farbloser Feststoff erhalten.

**3-(5-Fluor-1*H*-Indol- 3-yl) piperidin- 1-carbonsäure-(4-dimethylamino- 4-**phenylcyclohexyl)amid-hydrochlorid (polareres Diastereoisomer)

Zur Herstellung des Hydrochlorids wurde das polarere Diastereoisomer von 3-(5-Fluor-1*H*-Indol-3-yl)piperidin-1-carbonsäure-(4-dimethylamino-4-phenylcyclohexyl)-amid (200 mg, 0,43 mmol) in Ethylmethylketon (5 ml) gelöst und mit Chlortrimethylsilan (82 μl, 0,65 mmol) versetzt. Der dabei entstandene Feststoff wurde abfiltriert und getrocknet. Das Hydrochlorid des polareren Diastereoisomers von 3-(5-Fluor-1*H*-Indol-3-yl)piperidin-1-carbonsäure-(4-dimethylamino-4-phenylcyclohexyl)amid wurde so in einer Ausbeute von 215 mg (100 %) als farbloser Feststoff (**Beispiel 42**) mit einem Smp. von 160-164 ˚C gewonnen.

**Beispiele 43 und 44:**

**4-(5-Fluor-1*H*-Indol-3-yl)-3,6-dihydro-2*H*-pyridin-1-carbonsäure-(4-dimethylamino-4-phenylcyclohexylmethyl) amid-citrat**

**4-(5-Fluor-1*H*-indol-3-yl)-3,6-dihydro-2*H*-pyridin-1-carbonsäure-(4-dimethylamino-4-phenylcyclohexylmethyl) amid**

Zu einer Lösung von 5-Fluor-3-(1,2,3,6-tetrahydro-pyridin-4-yl)-1H-indol (428,2 mg, 1,98 mmol) in Dioxan (10 ml) wurde das Diastereoisomerengemisch von (4-Dimethylamino-4-phenyl-cyclohexylmethyl)-carbaminsäure-phenylester (700 mg, 1,98 mmol) gegeben. Anschließend wurde 12 h unter Rückfluss gekocht. Die Reaktionsmischung war bei RT leicht trüb. Zur Aufarbeitung wurde das Lösungsmittel abdestilliert. Der Rückstand enthielt neben Phenol die beiden Diastereoisomeren von 4-(5-Fluor-1*H*-indol-3-yl)-3,6-dihydro-2*H*-pyridin-1-carbonsäure-(4-dimethylamino-4-phenylcyclohexylmethyl)amid. Durch Flash-Chromatographie an Kieselgel (50 g) wurden die Diastereoisomeren getrennt und gereinigt. Als Eluent wurde Methanol/EE (1:1:1000 ml) eingesetzt. Das unpolarere Diastereoisomer von 4-(5-Fluor-1*H*-indol-3-yl)-3,6-dihydro-2*H*-pyridin-1-carbonsäure-(4-dimethylamino-4-phenylcyclohexylmethyl)amid (300 mg, hellgelb, Fp. 105-109 ˚C, 32 %) und das polarere Diastereoisomer von 4-(5-Fluor-1*H*-indol-3-yl)-3,6-dihydro-2*H*-pyridin-1-carbonsäure-(4-dimethylamino-4-phenylcyclohexylmethyl)amid (142 mg, 15 %) wurden so in reiner Form gewonnen.

**4-(5-Fluor-1*H*-indol-3-yl)-3,6-dihydro-2*H*-pyridin-1-carbonsäure-(4-dimethylamino-4-phenylcyclohexylmethyl) amid-citrat**

Das unpolarere Diastereoisomer von 4-(5-Fluor-1*H*-indol-3-yl)-3,6-dihydro-2*H*-pyridin-1-carbonsäure-(4-dimethylamino-4-phenylcyclohexylmethyl)amid (288 mg, 0,606 mmol) wurde in abs. Ethanol (6 ml) und DCM (5 ml) gelöst. Unter Rühren wurde bei RT die Zitronensäure (216 mg, 1,12 mmol) im Überschuss zugegeben. Nach Rühren über Nacht bei RT war kein Niederschlag ausgefallen. Die Lösungsmittel wurden abdestilliert. Das unpolarere Diastereoisomer von 4-(5-Fluor-1*H*-indol-3-yl)-3,6-dihydro-2*H*-pyridin-1-carbonsäure-(4-dimethylamino-4-phenylcyclohexylmethyl) amid-citrat (516 mg, **Beispiel 43**) war ein oranger hygroskopischer Feststoff.

Das polarere Diastereoisomer von 4-(5-Fluor-1*H*-indol-3-yl)-3,6-dihydro-2*H*-pyridin-1-carbonsäure-(4-dimethylamino-4-phenylcyclohexylmethyl)amid (140 mg, 0,294 mmol) wurde in abs. Ethanol (10 ml) gelöst. Die Zitronensäure (105,7 mg, 0.55 mmol) wurde als Feststoff zugegeben. Die Mischung wurde bei 40 ˚C solange gerührt, bis die Zitronensäure völlig gelöst war. Bei RT bildete sich an der Kolbenwand ein klebriger Niederschlag. Der Alkohol wurde abdestilliert und der Rückstand im Vakuum getrocknet. Das polarere Diastereoisomer von 4-(5-Fluor-1*H*-indol-3-yl)-3,6-dihydro-2*H*-pyridin-1-carbonsäure-(4-dimethylamino-4-phenylcyclohexylmethyl)amid-citrat (255 mg, hygroskopisch, **Beispiel 44**) war ein orangebrauner Feststoff.

**Beispiel 45:**

**4-(5-Methoxy-1*H*-indol-3-yl)piperidin-1-carbonsäure-(4-dimethylamino-4-phenylcyclohexylmethyl)amid-citrat**

**4-(5-Methoxy-1*H*-indol-3-yl)piperidin-1-carbonsäure-(4-dimethylamino-4-phenylcyclohexylmethyl)amid**

In zwei Mikrowellenreaktionsgefäßen wurden jeweils 5-Methoxy-3-piperidin-4-yl-1 H-indol (169,3 mg, 0,735 mmol) in Dioxan (4.5 ml) und das Diastereoisomerengemisch von (4-Dimethylamino-4-phenyl-cyclohexylmethyl)-carbaminsäure-phenylester (259,0 mg, 0,735 mmol) gelöst. Das Reaktionsgemisch wurde in der Mikrowelle wie folgt behandelt: Versuch 1 (150˚C, 52 min) und Versuch 2 (200 ˚C, 2 min). Zur Aufarbeitung wurde von jedem Versuch das Lösungsmittel im Vakuum abdestilliert. Der jeweilige Rückstand enthielt neben Phenol die beiden Diastereoisomeren von 4-(5-Methoxy-1*H*-indol-3-yl)piperidin-1-carbonsäure-(4-dimethylamino-4-phenylcyclohexylmethyl)amid. Durch

Flash-Chromatographie an Kieselgel (30 g bzw. 40 g) wurden die Diastereoisomeren getrennt und gereinigt. Als Eluent wurde Methanol/EE (1:1; ca. 600 ml) eingesetzt. Das unpolarere Diastereoisomer von 4-(5-Methoxy-1*H*-indol-3-yl) piperidin-1-carbonsäure-(4-dimethylamino-4-phenylcyclohexylmethyl)amid [Versuch 1 (116 mg, Fp. 106-107 ˚C, 32 %) bzw. Versuch 2 (89 mg, Fp. 110-112 ˚C, 25 %)] und das polarere Diastereoisomer von 4-(5-Methoxy-1*H*-indol-3-yl) piperidin-1-carbonsäure-(4-dimethylamino-4-phenylcyclohexylmethyl)amid [Versuch 1 (108 mg, Fp. 98-100 ˚C, 30 %) bzw. Versuch 2 (92 mg, Fp. 92-97 ˚C, 26 %)] wurden so in reiner Form gewonnen.

**4-(5-Methoxy-1*H*-indol-3-yl)piperidin-1-carbonsäure-(4-dimethylamino-4-phenylcyclohexylmethyl)amid-citrat**

Das unpolarere Diastereoisomer von 4-(5-Methoxy-1*H*-indol-3-yl)piperidin-1-carbonsäure-(4-dimethylamino-4-phenylcyclohexylmethyl)amid (203 mg, 0,415 mmol) wurde in abs. Ethanol (2 ml) und DCM (3 ml) gelöst. Unter Rühren wurde bei ca. 40 ˚C die Zitronensäure (80,7 mg, 0,419 mmol) in einer Portion zugegeben. Bei RT fiel sofort ein farbloser Niederschlag aus. Nach zweistündigem Rühren bei RT wurde die Suspension mit Diethylether (25 ml) versetzt und über Nacht weitergerührt. Nach 20 h wurde der Niederschlag abfiltriert, mit Diethylether (3 × 1 ml) gewaschen und im Vakuum getrocknet. Das unpolarere Diastereoisomer von 4-(5-Methoxy-1*H*-indol-3-yl)piperidin-1-carbonsäure-(4-dimethylamino-4-phenylcyclohexylmethyl)amid-citrat (249,5 mg, Fp. 155-158 ˚C, 88 %, **Beispiel 45**) war ein farbloser Feststoff.

Das polarere Diastereoisomer von 4-(5-Methoxy-1*H*-indol-3-yl)piperidin-1-carbonsäure-(4-dimethylamino-4-phenylcyclohexylmethyl)amid (197 mg, 0,403 mmol) wurde in abs. Ethanol (3 ml) und DCM (5 ml) gelöst. Unter Rühren wurde bei ca. 40 ˚C die Zitronensäure (78,3 mg, 0,407 mmol) in einer Portion zugegeben. Das Reaktionsgemisch wurde 2 h bei RT gerührt. Während dieser Zeit fiel kein Niederschlag aus. Die Lösungsmittel wurden bis auf 2 ml abdestilliert. Der Rückstand wurde mit Diethylether (30 ml) versetzt. Die Suspension wurde 20 h bei RT gerührt. Der Niederschlag wurde abfiltriert, mit Diethylether (3 × 1,5 ml) gewaschen und im Vakuum getrocknet. Das polarere Diastereoisomer von 4-(5-Methoxy-1*H*-indol-3-yl)piperidin-1-carbonsäure-(4-dimethylamino-4-phenylcyclohexylmethyl)amid-citrat (239 mg, Fp. 139-143 ˚C, 87 %, **Beispiel 46**) war ein farbloser Feststoff.

**Beispiele 47 und 48:**

**3-(1*H*-indol-3-yl)piperidin-1-carbonsäure-(4-dimethylamino-4-phenylcyclohexylmethyl)amid-citrat**

**3-(1*H*-Indol-3-yl)piperidin-1-carbonsäure-(4-dimethylamino-4-phenylcyclohexylmethyl)amid**

Zu einer Lösung von 3-Piperidin-3-yl-1 H-indol (300 mg, 1,5 mmol) in Dioxan (10 ml) wurde das Diastereoisomerengemisch von (4-Dimethylamino-4-phenylcyclohexylmethyl)-carbaminsäure-phenylester (529 mg, 1,5 mmol) gegeben. Anschließend wurde 20 h unter Rückfluss gekocht. Zur Aufarbeitung wurde das Lösungsmittel abdestilliert. Der Rückstand enthielt neben Phenol die beiden Diastereoisomeren von 3-(1*H*-Indol-3-yl)piperidin-1-carbonsäure-(4-dimethylamino-4-phenylcyclohexylmethyl)amid. Durch Flash-Chromatographie an Kieselgel (50 g) wurden die Diastereoisomeren getrennt und gereinigt. Als Eluent wurde Methanol/EE (1 : 1; 900 ml) eingesetzt. Das unpolarere Diastereoisomer von 3-(1*H*-Indol-3-yl)piperidin-1-carbonsäure-(4-dimethylamino-4-phenylcyclohexylmethyl)amid (220,6 mg, Fp. 165-170 ˚C, 32 %) und das polarere Diastereoisomervon 3-(1*H*-Indol-3-yl)piperidin-1-carbonsäure-(4-dimethylamino-4-phenylcyclohexylmethyl)amid (142 mg, Fp. 95-99 ˚C, 31 %) wurden so in reiner Form gewonnen.

**3-(1*H*-Indol-3-yl)piperidin-1-carbonsäure-(4-dimethylamino-4-phenylcyclohexylmethyl)amid-citrat**

Das unpolarere Diastereoisomer von 3-(1*H*-Indol-3-yl)piperidin-1-carbonsäure-(4-dimethylamino-4-phenylcyclohexylmethyl)amid (220 mg, 0,48 mmol) wurde in abs. Ethanol (3 ml) und DCM (3 ml) gelöst. Unter Rühren wurde bei ca. 40 ˚C die Zitronensäure (93,1 mg, 0,484 mmol) in einer Portion zugegeben. Bei RT fiel zunächst kein Niederschlag aus. Die Lösungsmittelmenge wurde im Vakuum auf 1 ml reduziert und mit Diethylether (20 ml) versetzt. Nach zweistündigem Rühren bei RT wurde der Niederschlag abfiltriert, mit Diethylether (3 × 1 ml) gewaschen und im Vakuum getrocknet. Das unpolarere Diastereoisomer von 3-(1*H*-Indol-3-yl)piperidin-1-carbonsäure-(4-dimethylamino-4-phenylcyclohexylmethyl)ämid-citrat (292 mg, Fp. 150-158 ˚C, 93 %, **Beispiel 47**) war ein farbloser Feststoff.

Das polarere Diastereoisomer von 3-(1*H*-Indol-3-yl)piperidin-1-carbonsäure-(4-dimethylamino-4-phenylcyclohexylmethyl)amid (214 mg, 0,467 mmol) wurde in abs. Ethanol (3 ml) gelöst. Unter Rühren wurde bei ca. 40 ˚C die Zitronensäure (90,6 mg, 0,471 mmol) in einer Portion zugegeben. Das Reaktionsgemisch wurde 2 h bei RT gerührt. Während dieser Zeit fiel kein Niederschlag aus. Ethanol wurde bis auf 1 ml abdestilliert. Der Rückstand wurde mit Diethylether (10 ml) versetzt. Die Suspension wurde 20 h bei RT gerührt. Der Niederschlag wurde abfiltriert, mit Diethylether (3 × 1,5 ml) gewaschen und im Vakuum getrocknet. Das polarere Diastereoisomer von 3-(1*H*-Indol-3-yl)piperidin-1-carbonsäure-(4-dimethylamino-4-phenylcyclohexylmethyl)amid-citrat (297 mg, Fp. 116-120 ˚C, 98 %, **Beispiel 48**) war ein cremefarbener Feststoff.

**Beispiele 49 und 50:**

**3-(5-Fluor-1*H*-indol-3-yl)piperidin-1-carbonsäure-(4-dimethylamino-4-phenylcyclohexylmethyl)amid-citrat**

**3-(5-Fluor-1*H*-indol-3-yl)piperidin-1-carbonsäure-(4-dimethylamino-4-phenylcyclohexylmethyl)amid**

Zu einer Lösung von 5-Fluor-3-piperidin-3-yl-1 H-indol (220 mg, 1 mmol) in Dioxan (8 ml) wurde das Diastereoisomerengemisch von (4-Dimethylamino-4-phenylcyclohexylmethyl)-carbaminsäure-4-nitrophenylester (400 mg, 1 mmol) gegeben. Anschließend wurde 8 h unter Rückfluss gekocht. Zur Aufarbeitung wurde das Lösungsmittel abdestilliert. Der Rückstand enthielt neben Nitrophenol die beiden Diastereoisomeren von 3-(5-Fluor-1*H*-indol-3-yl)piperidin-1-carbonsäure-(4-dimethylamino-4-phenylcyclohexylmethyl)amid. Durch Flash-Chromatographie an Kieselgel (30 g) wurden die Diastereoisomeren getrennt und gereinigt. Als Eluent wurde Methanol/EE (1:1; 850 ml) eingesetzt. Das unpolarere Diastereoisomer von 3-(5-Fluor-1*H*-indol-3-yl)piperidin-1-carbonsäure-(4-dimethylamino-4-phenylcyclohexylmethyl)amid (143 mg, Fp. 110-115 ˚C, 30 %) und das polarere Diastereoisomer von 3-(5-Fluor-1*H*-indol-3-yl)piperidin-1-carbonsäure-(4-dimethylamino-4-phenylcyclohexylmethyl)amid (118 mg, Fp. 98-101 ˚C, 25 %) wurden so in reiner Form gewonnen.

**3-(5-Fluor-1*H*-indol-3-yl)piperidin-1-carbonsäure-(4-dimethylamino-4-phenylcyclohexylmethyl)amid-citrat**

Das unpolarere Diastereoisomer von 3-(5-Fluor-1*H*-indol-3-yl)piperidin-1-carbonsäure-(4-dimethylamino-4-phenylcyclohexylmethyl)amid (140 mg, 0,294 mmol) wurde in abs. Ethanol (2 ml) gelöst. Unter Rühren wurde bei ca. 40 ˚C die Zitronensäure (57,1 mg, 0,297 mmol) in einer Portion zugegeben. Bei RT fiel kein Niederschlag aus. Die Reaktionslösung wurde langsam mit Diethylether (50 ml) versetzt. Die entstandene Suspension wurde bei RT 1 h gerührt. Der Niederschlag wurde abfiltriert, mit Diethylether (3 × 2 ml) gewaschen und im Vakuum getrocknet. Das unpolarere Diastereoisomer von 3-(5-Fluor-1*H*-indol-3-yl)piperidin-1-carbonsäure-(4-dimethylamino-4-phenylcyclohexylmethyl) amid-citrat (178 mg, Fp. 127-132 ˚C, 91 %, **Beispiel 49**) war ein farbloser Feststoff.

Das polarere Diastereoisomer von 3-(5-Fluor-1*H*-indol-3-yl)piperidin-1-carbonsäure-(4-dimethylamino-4-phenylcyclohexylmethyl)amid (115 mg, 0,241 mmol) wurde in abs. Ethanol (1,5 ml) gelöst. Unter Rühren wurde bei ca. 40˚C die Zitronensäure (46,8 mg, 0,244 mmol) in einer Portion zugegeben. Bei RT fiel kein Niederschlag aus. Die Reaktionslösung wurde langsam mit Diethylether (50 ml) versetzt. Die entstandene Suspension wurde bei RT 1 h gerührt. Der Niederschlag wurde abfiltriert, mit Diethylether (3 × 2 ml) gewaschen und im Vakuum getrocknet. Das polarere Diastereoisomer von 3-(5-Fluor-1*H*-indol-3-yl)piperidin-1-carbonsäure-(4-dimethylamino-4-phenylcyclohexylmethyl)amid-citrat (133 mg, Fp. 128-130 ˚C, 83 %, **Beispiel 50**) war ein farbloser Feststoff.

**Beispiele 51 und 52:**

**3-(1*H*-indol-3-yl)pyrrolidin-1-carbonsäure-(4-dimethylamino-4-phenylcyclohexylmethyl)amid-citrat**

**3-(1*H*-indol-3-yl)pyrrolidin-1-carbonsäure-(4-dimethylamino-4-phenylcyclohexylmethyl)amid**

Zu einer Lösung von 3-Pyrrolidin-3-yl-1H-indol (274 mg, 1,47 mmol) in Dioxan (12 ml) wurde das Diastereoisomerengemisch von (4-Dimethylamino-4-phenylcyclohexylmethyl)-carbaminsäure-phenylester (519 mg, 1,47 mmol) gegeben. Anschließend wurde 12 h unter Rückfluss gekocht. Zur Aufarbeitung wurde das Lösungsmittel abdestilliert. Der Rückstand enthielt neben Phenol die beiden Diastereoisomeren von 3-(1 H-Indol-3-yl)pyrrolidin-1 -carbonsäure-(4-dimethylamino-4-phenyl-cyclohexylmethyl)amid. Durch Flash-Chromatographie an Kieselgel (50 g) wurden die Diastereoisomeren getrennt und gereinigt. Als Eluent wurde Methanol/EE (1 : 1; 1000 ml) eingesetzt. Das unpolarere Diastereoisomer von 3-(1 H-Indol-3-yl)pyrrolidin-1-carbonsäure-(4-dimethylamino-4-phenyl-cyclohexylmethyl)amid (234 mg, Fp. 101-103 ˚C, 36 %) und das polarere Diastereoisomeren von 3-(1*H*-)ndot-3-yl)pyrrolidin-1-carbonsäure-(4-dimethylamino-4-phenyl-cyclohexylmethyl)amid (244 mg, Fp. 103-106 ˚C. 37 %) wurden so in reiner Form gewonnen.

**3-(1*H*-Indol-3-yl)pyrrolidin-1-carbonsäure-(4-dimethylamino-4-phenylcyclohexylmethyl)amid-citrat**

Das unpolarere Diastereoisomer von 3-(1*H*-Indol-3-yl)pyrrolidin-1-carbonsäure-(4-dimethylamino-4-phenylcyclohexylmethyl)amid (222 mg, 0,499 mmol) wurde in abs. Ethanol (5 ml) gelöst. Unter Rühren wurde bei ca. 40 ˚C die Zitronensäure (96,9 mg, 0,504 mmol) in einer Portion zugegeben. Bei RT fiel kein Niederschlag aus. Die Lösungsmittelmenge wurde im Vakuum auf 2 ml reduziert und dann mit Diethylether (20 ml) versetzt. Nach Rühren über Nacht bei RT wurde der Niederschlag abfiltriert, mit Diethylether (3 × 3 ml) gewaschen und im Vakuum getrocknet. Das unpolarere Diastereoisomer von 3-(1*H*-Indol-3-yl)pyrrolidin-1-carbonsäure-(4-dimethylamino-4-phenyl-cyclohexylmethyl)amid-citrat (292 mg, 92 %, **Beispiel 51**) war ein hellbeiger Feststoff.

Das polarere Diastereoisomer von 3-(1*H*-Indol-3-yl)pyrrolidin-1-carbonsäure-(4-dimethylamino-4-phenyl-cyclohexylmethyl)amid (115 mg, 0,241 mmol) wurde in abs. Ethanol (1,5 ml) gelöst. Unter Rühren wurde bei ca. 40°C die Zitronensäure (46,8 mg, 0,244 mmol) in einer Portion zugegeben. Bei RT fiel kein Niederschlag aus. Die Lösungsmittelmenge wurde im Vakuum auf 2 ml reduziert und dann mit Diethylether (20 ml) versetzt. Nach Rühren über Nacht bei RT wurde der Niederschlag abfiltriert, mit Diethylether (3 × 3 ml) gewaschen und im Vakuum getrocknet. Das unpolarere Diastereoisomer von 3-(1*H*-Indol-3-yl)pyrrolidin-1-carbonsäure-(4-dimethylamino-4-phenyl-cyclohexylmethyl)amid-citrat (312 mg, 94 %, **Beispiel 52**) war ein hellbeiger Feststoff.

**Beispiel 53:**

**2-(4-Dimethylamino-4-phenylcyclohexyl)-1-[3-(1*H*-indol-3-yl)piperi-din-1-yl]ethanon-hydrochlorid**

**2-(4-Dimethylamino-4-phenylcyclohexyl)-1-[3-(1*H*-indol-3-yl)piperidin-1-yl]ethanon**

Zu einer Lösung von 2-(4-Dimethylamino-4-phenyl-cyclohexyliden)-1-[3-(1H-indol-3-yl)-piperidin-1-yl]-ethanon (220 mg, 0,498 mmol) in abs. Methanol (30 ml) wurde Palladium auf Kohle (5 %, 60 mg) gegeben. Die Reaktionsmischung wurde 24 h bei einem Druck von 3 bar bei RT hydriert. Der Katalysator wurde über Celite abgetrennt und das Filtrat eingeengt. Nach chromatographischer Auftrennung des Rückstandes (210 mg) an Kieselgel (20 g) mit EE/Methanol (2 : 1) wurde 2-(4-Dimethylamino-4-phenylcyclohexyl)-1-[3-(1*H*-indol-3-yl)piperidin-1-yl]ethanon als farbloses Öl in einer Ausbeute von 30 % (66 mg) isoliert. - Die Verbindung wurde als Diastereoisomerengemisch erhalten.

**2-(4-Dimethylamino-4-phenylcyclohexyl)-1-[3-(1*H*-indol-3-yl)piperi-din-1-yl]ethanon-hydrochlorid**

2-(4-Dimethylamino-4-phenylcyclohexyl)-1-[3-(1*H*-indol-3-yl)piperidin-1-yl]ethanon (66 mg, 0,148 mmol) wurde in Ethylmethylketon (4 ml) gelöst und mit Chlortrimethylsilan (0,03 ml, 0,23 mmol) versetzt. Nach 1 h konnte das Hydrochlorid als farbloser Feststoff in einer Ausbeute von 65 % (46 mg) isoliert werden (**Beispiel 53**). Ein Smp. konnte nicht bestimmt werden. - Das Hydrochlorid wurde als Diastereoisomerengemisch erhalten.

**Beispiel 54**:

**2-(4-Dimethylamino-4-phenylcyclohexyliden)-1-[3-(1*H*-indol-3-yl)-piperidin-1-yl]ethanon-hydrochlorid**

**2-(4-Dimethylamino-4-phenylcyclohexyliden)-1-[3-(1*H*-indol-3-yl)-piperidin-1-yl]ethanon**

Zu einer Lösung von (4-Dimethylamino-4-phenyl-cyclohexyliden)-essigsäure (739 mg, 2,5 mmol) in trockenem Dimethylformamid (10 ml) wurden unter Argon nacheinander 1-Hydroxybenzotriazol (675 mg, 5,0 mmol), 3-Piperidin-3-yl-1*H*-indol (500 mg, 2,5 mmol) und *N*-Methylmorpholin (0,555 ml, 5,0 mmol) gegeben. Die Lösung wurde im Eisbad gekühlt und mit Dicyclohexylcarbodiimid (1,03 g, 5,0 mmol) versetzt. Die Reaktionsmischung wurde 5 d bei RT gerührt, wobei nach und nach der Dicyclohexylharnstoff ausfiel. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff zusammen mit 2-(4-Dimethylamino-4-phenylcyclohexyliden)-1-[3-(1*H*-indol-3-yl)-piperidin-1-yl]ethanon aus (1,5 g). Die wässrige Phase wurde mit Wasser (300 ml) verdünnt, mit 5M Natronlauge (7 ml, 35 mmol) versetzt und 16 h bei 5 °C aufbewahrt. Dabei fiel das Rohprodukt als farbloser Feststoff aus (464 mg). Das Rohprodukt von 2-(4-Dimethylamino-4-phenylcyclohexyliden)-1-[3-(1*H*-indol-3-yl)-piperidin-1-yl]ethanon wurde an Kieselgel (60 g) mit EE/Methanol (4 : 1) und (2 : 1) chromatographisch gereinigt und in einer Ausbeute von 30 % (322 mg) gewonnen - 2-(4-Dimethylamino-4-phenylcyclohexyliden)-1-[3-(1*H*-indol-3-yl)-piperidin-1-yl]ethanon wurde als Diastereoisomerengemisch erhalten. Eine Trennung war nicht möglich.

**2-(4-Dimethylamino-4-phenylcyclohexyliden)-1-[3-(1*H*-indol-3-yl)-piperidin-1-yl]ethanon-hydrochlorid**

2-(4-Dimethylamino-4-phenylcyclohexyliden)-1-[3-(1*H*-indol-3-yl)-piperidin-1-yl]ethanon (316 mg, 0,716 mmol) wurde in Ethylmethylketon (4 ml) gelöst und mit Chlortrimethylsilan (0,14 ml, 1,1 mmol) versetzt. Nach 1,5 h wurde das 2-(4-Dimethylamino-4-phenylcyclohexyliden)-1-[3-(1*H*-indol-3-yl)-piperidin-1-yl]ethanon-hydrochlorid als farblose Verbindung mit einem Smp. von 177-180 °C in einer Ausbeute von 89 % (305 mg) als Diastereoisomerengemisch erhalten (**Beispiel 54**).

Beispiel 55:

**4-[4-(5-Chlor-1*H*-indol-3-yl)-3,6-dihydro-2*H*-pyridin-1-yl]-1-phenylcyclohexyldimethylamin-dihydrochlorid**

**5-Chlor-3-(1,2,3,6-tetrahydropyridin-4-yl)-1*H*-indol**

KOH (8,19 g, 146 mmol), 5-Chlorindol (5,0g, 33 mmol) und Piperidin-4-on-hydrochlorid (10,22 g, 85,7 mmol) wurden in Methanol (50 ml) suspendiert und unter Argon 8 h auf 65˚C erhitzt. Bei RT wurde dem Gemisch während einer Zeit von 40 min tropfenweise Wasser (50 ml) zugesetzt. Der dabei entstandene Feststoff wurde abfiltriert, mit Wasser (3 × 20 ml) gewaschen und aus Methanol (20 ml) umkristallisiert. 5-Chlor-3-(1,2,3,6-tetrahydropyridin-4-yl)-1*H*-indol wurde in einer Ausbeute von 6,4 g (90 %) als gelber Feststoff mit einem Smp. von 166-168 ˚C erhalten.

**4-[4-(5-Chlor-1*H*-indol-3-yl)-3,6-dihydro-2*H*-pyridin-1-yl]-1-phenylcyclohexyldimethylamin**

5-Chlor-3-(1,2,3,6-tetrahydropyridin-4-yl)-1*H*-indol (170 mg, 0,79 mmol) und 4-Dimethylamino-4-phenylcyclohexanon (171 mg, 0,79 mmol) wurden in trockenem 1,2-Dichlorethan (10 ml) gelöst. Diesem Gemisch wurde Eisessig (0,79 mmol) und Natriumtriacetoxyborhydrid (300 mg, 1,4 mmol) zugefügt. Anschließend wurde 24 h bei RT gerührt. Zur Aufarbeitung wurde 1,2-Dichlorethan abdestilliert und der Ansatz mit Wasser (10 ml) verdünnt. Der Ansatz wurde mit 5M NaOH auf pH 11 eingestellt und mit EE (4 × 20 ml) extrahiert. Die organische Phase wurde mit $Na_2SO_4$ getrocknet und eingedampft. Das Diastereoisomerengemisch wurde als farbloser Feststoff in einer Ausbeute von 340 mg (100 %) erhalten.

**4-[4-(5-Chlor-1*H*-indol-3-yl)-3,6-dihydro-2*H*-pyridin-1-yl]-1-phenylcyclohexyldimethylamin-dihydrochlorid**

Zur Herstellung der Hydrochloride wurde das Diastereoisomerengemisch von 4-[4-(5-Chlor-1*H*-indol-3-yl)-3,6-dihydro-2*H*-pyridin-1-yl]-1-phenylcyclohexyl-dimethylamin (340 mg, 0,79 mmol) in Ethylmethylketon (5 ml) gelöst und mit Chlortrimethylsilan (255 µl, 2,0 mmol) versetzt. Der dabei entstandene Feststoff wurde abfiltriert und getrocknet. Das Hydrochlorid wurde so in einer Ausbeute von 170 mg (46 %) als farbloser Feststoff (**Beispiel 55**) mit einem Smp. von 210-212 ˚C gewonnen.

Beispiel 56:

**{4-[4-(1*H*-Indol-3-yl)-3,6-dihydro-2*H*-pyridin-1-yl]-1-phenylcyclohexyl}-dimethylamin-dihydrochlorid**

**3-(1,2,3,6-tetrahydropyridin-4-yl)-1*H*-indol**

KOH (8,19 g, 146 mmol), Indol (3,87 g, 33 mmol) und Piperidin-4-on-hydrochlorid (10,22 g, 85,7 mmol) wurden in Methanol (50 ml) suspendiert und unter Argon 8 h auf 65 ˚C erhitzt. Bei RT wurde dem Gemisch während einer Zeit von 40 min tropfenweise Wasser (50 ml) zugesetzt. Der dabei entstandene Feststoff wurde abfiltriert, mit Wasser (3 × 20 ml) gewaschen und aus Methanol (20 ml) umkristallisiert. 3-(1,2,3,6-Tetrahydropyridin-4-yl)-1 H-indol wurde in einer Ausbeute von 4,26 g (71 %) als gelber Feststoff mit einem Smp. von 172-174 ˚C erhalten.

**{4-[4-(1*H*-Indol-3-yl)-3,6-dihydro-2*H*-pyridin-1-yl]-1-phenylcyclohexyl}-dimethylamin**

3-(1,2,3,6-Tetrahydropyridin-4-yl)-1*H*-indol (182 mg, 1 mmol) und 4-Dimethylamino-4-phenylcyclohexanon (217 mg, 1 mmol) wurden in trockenem 1,2-Dichlorethan (10 ml) gelöst. Diesem Gemisch wurde Eisessig (1 mmol) und Natriumtriacetoxyborhydrid (300 mg, 1,4 mmol) zugefügt. Anschließend wurde 24 h bei RT gerührt. Zur Aufarbeitung wurde 1,2-Dichlorethan abdestilliert und der Ansatz mit Wasser (10 ml) verdünnt. Der Ansatz wurde mit 5M NaOH auf pH 11 eingestellt und mit EE (4 × 20 ml) extrahiert. Die organische Phase wurde mit $Na_2SO_4$ getrocknet und eingedampft. Das Produkt war ein Gemisch aus zwei diastereoisomeren Verbindungen, die durch Chromatographie nicht getrennt werden konnten. Das Diastereoisomerengemisch wurde als farbloser Feststoff in einer Ausbeute von 320 mg (80 %) erhalten.

**{4-[4-(1*H*-Indol-3-yl)-3,6-dihydro-2*H*-pyridin-1-yl]-1-phenylcyclohexyl}-dimethylamin-dihydrochlorid**

Zur Herstellung der Hydrochloride wurde das Diastereoisomerengemisch von {4-[4-(1*H*-Indol-3-yl)-3,6-dihydro-2*H*-pyridin-1-yl]-1-phenylcyclohexyl}-dimethylamin (320 mg, 0,8 mmol) in Ethylmethylketon (5 ml) gelöst und mit Chlortrimethylsilan (255 µl, 2,0 mmol) versetzt. Der dabei entstandene Feststoff wurde abfiltriert und getrocknet. Das Hydrochlorid wurde so in einer Ausbeute von 378 mg (100 %) als farbloser Feststoff (**Beispiel 56**) mit einem Smp. von 188-191 ˚C gewonnen.

**Beispiel 57:**

**{4-[4-(5-Methoxy-1*H*-indol-3-yl)-3,6-dihydro-2*H*-pyridin-1-yl]-1-phenylcyclohexyl}dimethylamin-dihydrochlorid**

**5-Methoxy-3-(1,2,3,6-tetrahydropyridin-4-yl)-1*H*-indol**

KOH (8,44 g, 150 mmol), 5-Methoxyindol (5,0 g, 34 mmol) und Piperidin-4-on-hydrochlorid (10,53 g, 88,3 mmol) wurden in Methanol (50 ml) suspendiert und unter Argon 8 h auf 65˚C erhitzt. Bei RT wurde dem Gemisch während einer Zeit von 40 min tropfenweise Wasser (50 ml) zugesetzt. Der dabei entstandene Feststoff wurde abfiltriert, mit Wasser (3 × 20 ml) gewaschen und aus Methanol (20 ml) umkristallisiert. 5-Methoxy-3-(1,2,3,6-tetrahydropyridin-4-yl)-1H indol wurde in einer Ausbeute von 4,66 g (75 %) als gelber Feststoff mit einem Smp. von 173-175 ˚C erhalten.

**{4-[4-(5-Methoxy-1*H*-indol-3-yl)-3,6-dihydro-2*H*-pyridin-1-yl]-1-phenylcyclohexyl)dimethylamin**

5-Methoxy-3-(1,2,3,6-tetrahydropyridin-4-yl)-1*H*-indol (212 mg, 1,0 mmol) und 4-Dimethylamino-4-phenylcyclohexanon (217 mg, 1,0 mmol) wurden in trockenem 1,2-Dichlorethan (20 ml) gelöst. Diesem Gemisch wurde Eisessig (1,0 mmol) und Natriumtriacetoxyborhydrid (300 mg, 1,4 mmol) zugefügt. Anschließend wurde 24 h bei RT gerührt. Zur Aufarbeitung wurde 1,2-Dichlorethan abdestilliert und der Ansatz mit Wasser (10 ml) verdünnt. Der Ansatz wurde mit 5M NaOH auf pH 11 eingestellt und mit EE (4 × 20 ml) extrahiert. Die organische Phase wurde mit $Na_2SO_4$ getrocknet und eingedampft. Das Produkt war ein Gemisch aus zwei diastereoisomeren Verbindungen. Das Diastereoisomerengemisch wurde als farbloser Feststoff in einer Ausbeute von 343 mg (80 %) erhalten.

**{4-[4-(5-Methoxy-1*H*-Indol-3-yl)-3,6-dihydro-2*H*-pyridin-1-yl]-1-phenylcyclohexyl}dimethylamin-dihydrochlorid**

Zur Herstellung der Hydrochloride wurde das Diastereoisomerengemisch von {4-[4-(5-Methoxy-1*H*-indol-3-yl)-3,6-dihydro-2*H*-pyridin-1-yl]-1-phenylcyclohexyl}-dimethylamin (343 mg, 0,8 mmol) in Ethylmethylketon (5 ml) gelöst und mit Chlortrimethylsilan (255 µl, 2,0 mmol) versetzt. Der dabei entstandene Feststoff wurde abfiltriert und getrocknet. Das Hydrochlorid wurde so in einer Ausbeute von 400 mg (100 %) als farbloser Feststoff (**Beispiel 57**) mit einem Smp. von 201-203 ˚C gewonnen.

**Beispiele 58 und 59:**

**4-(5-Chlor-1*H*-indol-3-yl)-3,6-dihydro-2*H*-pyridin-1-carbonsäure-(4-dimethylamino-4-phenyl-cyclohexylmethyl)amid-hydrochlorid**

**(4-Dimethylamino-4-phenylcyclohexylmethyl)carbaminsäure-phenylester**

Zu einer Lösung von (4-Aminomethyl-1-phenyl-cyclohexyl)-dimethyl-amin (2,32 g, 10 mmol) in abs. DCM (25 ml) und Pyridin (888 pl. 11 mmol), wurde der in abs. DCM (25 ml) gelöste Chlorameisensäure-phenylester (1,32 ml, 10,5 mmol) unter Eiswasserkühlung langsam zugetropft. Anschließend wurde 20 h bei RT gerührt. Zur Aufarbeitung wurde die Reaktionsmischung mit Wasser (3x10 ml), mit 1M HCl (3 × 10 ml) und mit 1 M NaOH (2 × 10 ml) extrahiert. Die organische Phase wurde mit $Na_2SO_4$ getrocknet und anschließend eingedampft. Der Rückstand war das Diastereoisomerengemisch von (4-Dimethylamino-4-phenylcyclohexylmethyl)-carbaminsäure-phenylester. Dieser wurde als farbloser Feststoff in einer Ausbeute von 3,18 g (Fp. 108-124 ˚C, 90 %) erhalten.

**4-(5-Chlor-1*H*-indol-3-yl)-3,6-dihydro-2*H*-pyridin-1-carbonsäure-(4-dimethylamino-4-phenyl-cyclohexylmethyl)amid**

Zu einer Lösung von 5-Chlor-3-(1,2,3,6-tetrahydro-pyridin-4-yl)-1H-indol (465,4 mg, 2 mmol) in Dioxan (12 ml) wurde das Diastereoisomerengemisch von (4-Dimethylamino-4-phenyl-cyclohexylmethyl)-carbaminsäure-phenylester (705 mg, 2 mmol) gegeben. Anschließend wurde 20 h unter Rückfluß gekocht. Die Reaktionsmischung war bei RT eine Suspension. Der Feststoff wurde abfiltriert, mit kaltem Dioxan (3 × 2 ml) gewaschen und getrocknet. Der Feststoff war das polarere Diastereoisomer von 4-(5-Chlor-1*H*-indol-3-yl)-3,6-dihydro-2*H*-pyridin-1-carbonsäure-(4-dimethylamino-4-phenyl-cyclohexylmethyl)amid (259 mg, Fp. 120-130 °C, 26 %). Die Reaktionslösung (Filtrat und Waschphasen) wurde eingeengt und der Rückstand mit Wasser (10 ml) versetzt. Der Ansatz wurde mit 5M NaOH auf pH 11 eingestellt und mit EE (3 × 20 ml) extrahiert. Die vereinigten EE-Extrakte wurden mit 1 M NaOH (1 × 5 ml) gewaschen und mit $Na_2SO_4$ getrocknet. Das Lösungsmittel wurde im Vakuum abdestilliert. Der Rückstand enthielt Phenol und überwiegend das unpolarere Diastereoisomer von 4-(5-Chlor-1*H*-indol-3-yl)-3,6-dihydro-2*H*-pyridin-1-carbonsäure-(4-dimethylamino-4-phenyl-cyclohexylmethyl)amid. Durch Flash-Chromatographie an Kieselgel (80 g) wurden die Diastereoisomeren getrennt und gereinigt. Als Eluent wurde Ethanol (1000 ml) eingesetzt. Das unpolarere Diastereoisomer von 4-(5-Chlor-1*H*-indol-3-yl)-3,6-dihydro-2*H*-pyridin-1-carbonsäure-(4-dimethylamino-4-phenyl-cyclohexylmethyl)amid (304 mg, Fp. 216-22 °C, 31 %) und ein weiterer Teil des polareren Diastereoisomers von 4-(5-Chlor-1*H*-indol-3-yl)-3,6-dihydro-2*H*-pyridin-1-carbonsäure-(4-dimethylamino-4-phenylcyclohexylmethyl)amid (85 mg, Fp. 108-111 °C, 9 %) wurden so isoliert.

**4-(5-Chlor-1 *H*-indol-3-yl)-3,6-dihydro-2*H*-pyridin-1-carbonsäure-(4-dimethylamino-4-phenyl-cyclohexylmethyl)amid-hydrochlorid**

Das unpolarere Diastereoisomer von 4-(5-Chlor-1*H*-indol-3-yl)-3,6-dihydro-2*H*-pyridin-1-carbonsäure-(4-dimethylamino-4-phenyl-cyclohexylmethyl)amid (288 mg, 0,59 mmol) wurde in Aceton (25 ml) und Ethanol (25 ml) gelöst. Unter Rühren wurde bei RT die 5M isopropanolische Salzsäure (176 µl, 0,88 mmol) tropfenweise zur Suspension zugegeben. Spontan entstand eine klare Lösung, aus der nach einstündigem Rühren bei RT kein Niederschlag ausfiel. Die Reaktionslösung wurde im Vakuum auf 2 ml reduziert und langsam mit Diethylether (50 ml) versetzt. Danach wurde bei RT 2 h kräftig gerührt. Es bildete sich ein oranger Niederschlag. Der Feststoff wurde abfiltriert, mit Diethylether (3 × 3 ml) gewaschen und im Vakuum getrocknet. Das unpolarere Diastereoisomer von 4-(5-Chlor-1*H*-indol-3-yl)-3,6-dihydro-2*H*-pyridin-1-carbonsäure-(4-dimethylamino-4-phenyl-cyclohexylmethyl)amid wurde so in einer Ausbeute von 308 mg (Fp. 191-193 °C, 99 %) als zimtfarbener Feststoff (**Beispiel 58**) erhalten.

Das polarere Diastereoisomer von 4-(5-Chlor-1*H*-indol-3-yl)-3,6-dihydro-2*H*-pyridin-1-carbonsäure-(4-dimethylamino-4-phenyl-cyclohexylmethyl)amid (246 mg, 0,5 mmol) wurde in Ethylmethylketon (10 ml) gelöst. Unter Rühren wurde bei RT Chlortrimethylsilan (95 µl, 0,75 mmol) tropfenweise zugegeben. Nach einstündigem Rühren war wenig Niederschlag ausgefallen. Die Reaktionslösung wurde mit Diethylether (35 ml) versetzt. Danach wurde bei RT 2h kräftig gerührt. Es bildete sich ein oranger Niederschlag. Der Feststoff wurde abgesaugt, mit Diethylether (3 × 2 ml) gewaschen und im Vakuum getrocknet. Das Hydrochlorid des polareren Diastereoisomers von 4-(5-Chlor-1*H*-indol-3-yl)-3,6-dihydro-2*H*-pyridin-1-carbonsäure-(4-dimethylamino-4-phenyl-cyclohexylmethyl)amid (207 mg, Fp. 183-185 °C, 78 %) wurde so als zimtfarbener Feststoff (**Beispiel 59**) erhalten.

**Beispiel 60:**

**2-(4-Dimethylamino-4-(4-fluorphenyl)cyclohexyliden)-1-[3-(1*H*-indol-3-yl)pyrrolidin-1-yl]ethanon-hydrochlorid**

**2-(4-Dimethylamino-4-(4-fluorphenyl)cyclohexyliden)-1-[3-(1*H*-indol-3-yl)pyrrolidin-1-yl]ethanon**

Zu einer Lösung von [4-Dimethylamino-4-(4-fluorphenyl)-cyclohexylidene]-essigsäure (1,055 g, 4 mmol) in trockenem Dimethylformamid (40 ml) wurden unter Argon nacheinander 1-Hydroxybenzotriazol (1,08 g, 8 mmol), 3-Pyrrolidin-3-yl-1*H*-indol (744 mg, 4 mmol) und *N*-Methylmorpholin (0,888 ml, 8 mmol) gegeben. Die klare Lösung wurde im Eisbad abgekühlt und mit Dicyclohexylcarbodiimid (1,65 g, 8 mmol) versetzt. Die Reaktionsmischung wurde 5 d bei Raumtemperatur gerührt, wobei nach und nach der Dicyclohexylharnstoff ausfiel. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Das Filtrat wurde mit Wasser verdünnt (300 ml) und mit 5M Natronlauge (7 ml, 35 mmol) versetzt. Dabei fiel das Rohprodukt von 2-(4-Dimethylamino-4-(4-fluorphenyl)-cyclohexyliden)-1-[3-(1*H*-indol-3-yl)pyrrolidin-1-yl]ethanon als beigefarbener Feststoff aus (1,67 g). Die chromatographische Trennung an Kieselgel (80 g) erfolgte mit EE/Methanol (1 : 1). 2-(4-Dimethylamino-4-(4-fluorphenyl)cyclohexyliden)-1-[3-(1*H*-indol-3-yl)pyrrolidin-1-yl]ethanon wurde dabei als Diastereoisomerengemisch in einer Ausbeute von 52 % (916 mg) als farbloser Feststoff mit einem Schmelzpunkt von 109-112 °C erhalten.

**2-(4-Dimethylamino-4-(4-fluorphenyl)cyclohexyliden)-1-[3-(1*H*-indol-3-yl)pyrrolidin-1-yl]ethanon-hydrochlorid**

Zu einer Lösung von 2-(4-Dimethylamino-4-(4-fluorphenyl)cyclohexyliden)-1-[3-(1*H*-indol-3-yl)pyrrolidin-1-yl]ethanon (300 mg, 0,67 mmol) in Ethylmethylketon (25 ml) wurde Chlortrimethylsilan (0,13 ml, 1,01 mmol) hinzugefügt

und 2 h bei RT gerührt. Dabei fiel das Hydrochlorid (300 mg, 93 %) als Diastereoisomerengemisch mit einem Schmelzpunkt von 189-194 ˚C als farbloser Feststoff aus (**Beispiel 60**).

**Beispiel 61:**

**Dimethyl(1-phenyl-4-piperidin-1-ylcyclohexyl)amin-dihydrochlorid (polareres Diastereoisomer)**

**Dimethyl(1-phenyl-4-piperidin-1-ylcyclohexyl)amin**

Piperidin (400 µl, 4 mmol) und 4-Dimethylamino-4-phenylcyclohexanon (434 mg, 2 mmol) wurden in trockenem 1,2-Dichlorethan (10 ml) gelöst. Diesem Gemisch wurde Eisessig (2 mmol) und Natriumtriacetoxyborhydrid (600 mg, 2,8 mmol) zugefügt und 24 h bei RT gerührt. Zur Aufarbeitung wurde 1,2-Dichlorethan abdestilliert und der Ansatz mit 5M NaOH auf pH 11 eingestellt. Die alkalische Phase wurde mit Wasser (10 ml) verdünnt und mit EE (3 × 20 ml) extrahiert. Die organische Phase wurde mit $Na_2SO_4$ getrocknet und eingedampft. Dimethyl(1-phenyl-4-piperidin-1-ylcyclohexyl)-amin konnte durch Chromatographie mit Ethanol gereinigt werden. Das unpolarere Diastereoisomer (15 mg) enthielt noch Verunreinigungen, die auch durch weitere Reinigungsversuche nicht beseitigt werden konnten. Das polare Diastereoisomer wurde als farbloses Öl mit einer Ausbeute von 92 mg (16 %) erhalten.

**Dimethyl(1-phenyl-4-piperidin-1-ylcyclohexyl)amin-dihydrochlorid (polareres Diastereoisomer)**

Zur Herstellung des Hydrochlorids wurde das polarere Diastereoisomer von Dimethyl(1-phenyl-4-piperidin-1-ylcyclohexyl)amin (92 mg, 0,32 mmol) in Ethylmethylketon (4 ml) gelöst und mit Trimethylchlorsilan (102 µl, 0,8 mmol) versetzt. Der dabei entstandene Feststoff wurde abfiltriert und getrocknet. Das Hydrochlorid von Dimethyl(1-phenyl-4-piperidin-1-ylcyclohexyl)amin wurde so in einer Ausbeute von 103 mg (100 %) als farbloser Feststoff (**Beispiel 61**) mit einem Smp. von 260-261 ˚C erhalten.

**Beispieltabelle:**

| Beispiel Nr. | Struktur | Salzform | Kommentare |
|---|---|---|---|
| 1 | 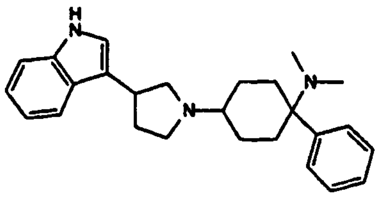 | Dihydrochlorid | Unpolareres Diastereoisomer |
| 2 | 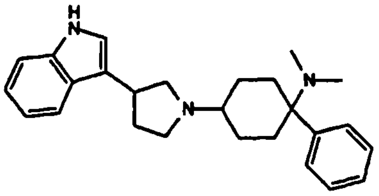 | Dihydrochlorid | Gemisch |
| 3 | 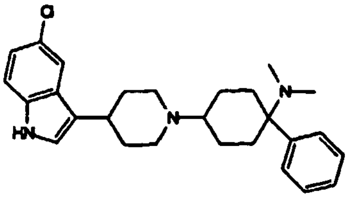 | Dihydrochlorid | Unpolareres Diastereoisomer |

(fortgesetzt)

| Beispiel Nr. | Struktur | Salzform | Kommentare |
|---|---|---|---|
| 4 | 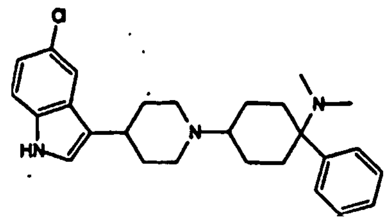 | Dihydrochlorid | Polareres Diastereoisomer |
| 5 | 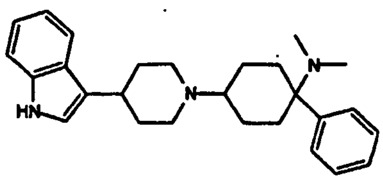 | Dihydrochlorid | Polareres Diastereoisomer |
| 6 | 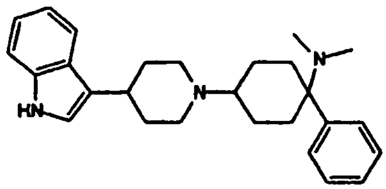 | Dihydrochlorid | Unpolareres Diastereoisomer |
| 7 | 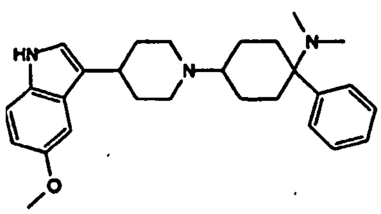 | Dihydrochlorid | Unpolareres Diastereoisomer |
| 8 | 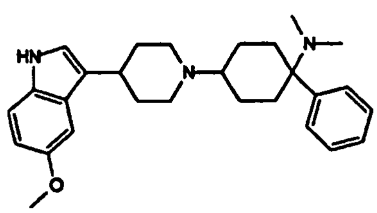 | Dihydrochlorid | Polareres Diastereoisomer |
| 9 | 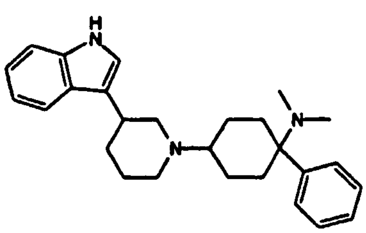 | Dihydrochlorid | Unpolareres Diastereoisomer |
| 10 | 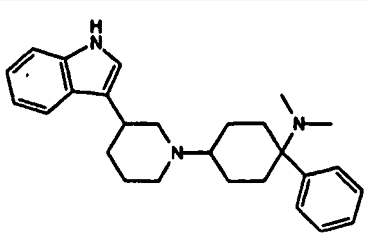 | Dihydrochlorid | Polareres Diastereoisomer |

(fortgesetzt)

| Beispiel Nr. | Struktur | Salzform | Kommentare |
|---|---|---|---|
| 11 | 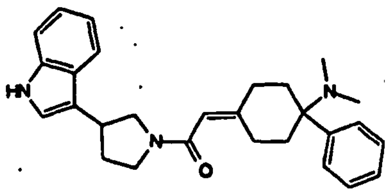 | Hydrochlorid | Diastereoisomerengemisch |
| 12 | 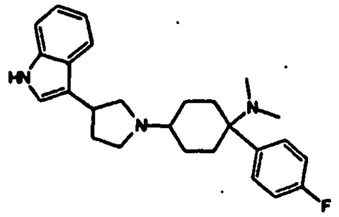 | Dihydrochlorid | Polareres Diastereoisomer |
| 13 | 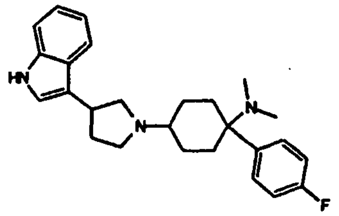 | Dihydrochlorid | Unpolareres Diastereoisomer |
| 14 | 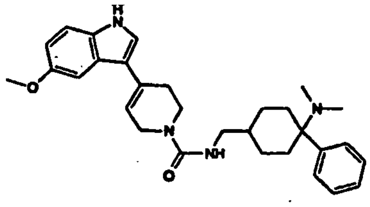 | Citrat | Unpolareres Diastereoisomer |
| 15 | 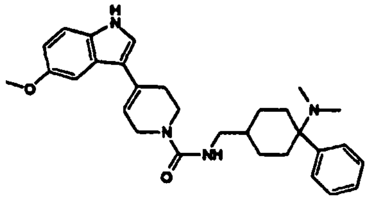 | Citrat | Polareres Diastereoisomer |
| 16 | 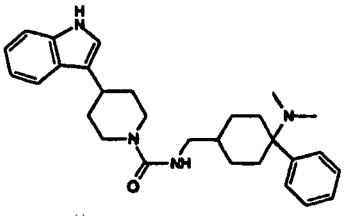 | Hemicitrat | Unpolareres Diastereoisomer |
| 17 | 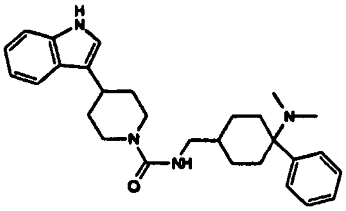 | Citrat | Unpolareres Diastereoisomer |

(fortgesetzt)

| Beispiel Nr. | Struktur | Salzform | Kommentare |
|---|---|---|---|
| 18 | 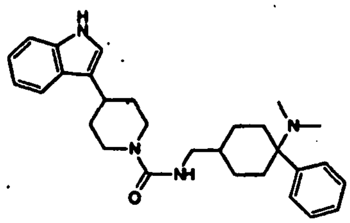 | Citrat | Polareres Diastereoisomer |
| 19 | 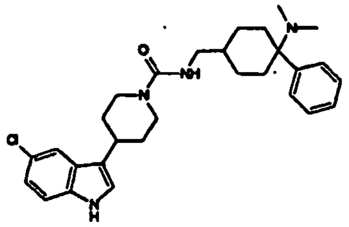 | Citrat | Polarers Diastereoisomer |
| 20 | 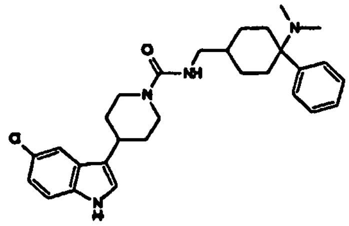 | Hemicitrat | Unpolareres Diastereoisomer |
| 21 | 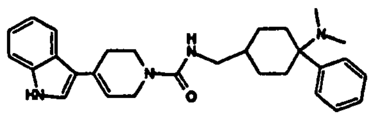 | Citrat | Unpolareres Diastereoisomer |
| 22 | 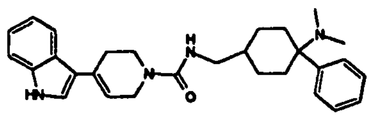 | Citrat | Polareres Diastereoisomer |
| 23 | 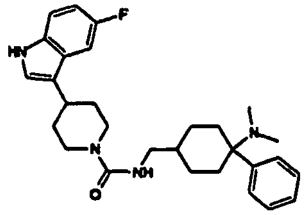 | Hemicitrat | Unpolareres Diastereoisomer |
| 24 | 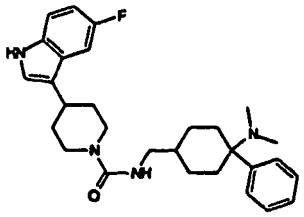 | Citrat | Polareres Diastereoisomer |

(fortgesetzt)

| Beispiel Nr. | Struktur | Salzform | Kommentare |
|---|---|---|---|
| 25 | 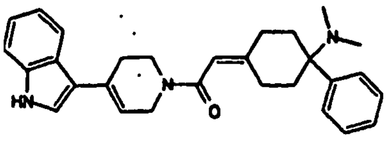 | Hydrochlorid | |
| 26 | 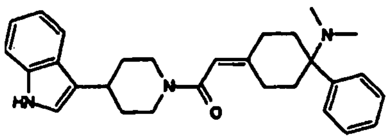 | Hydrochlorid | |
| 27 | 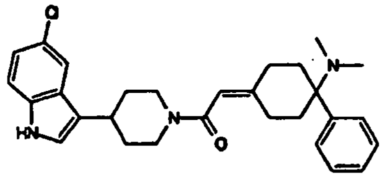 | Hydrochlorid | |
| 28 | 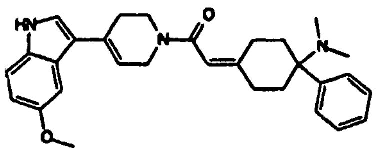 | Hydrochlorid | |
| 29 | 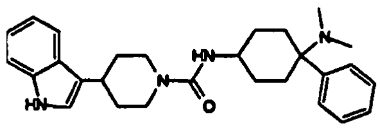 | Hydrochlorid | Unpolareres Diastereoisomer |
| 30 | 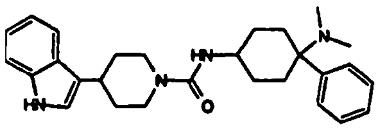 | Hydrochlorid | Polareres Diastereoisomer |
| 31 | 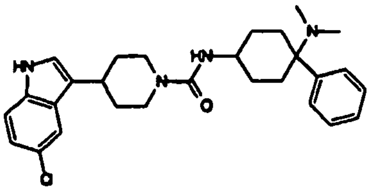 | Hydrochlorid | Unpolareres Diastereoisomer |
| 32 | 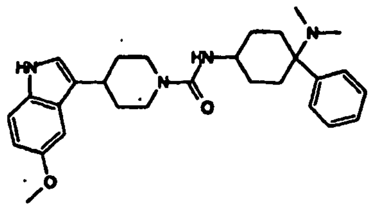 | Hydrochlorid | Unpolareres Diastereoisomer |

(fortgesetzt)

| Beispiel Nr. | Struktur | Salzform | Kommentare |
|---|---|---|---|
| **33** | 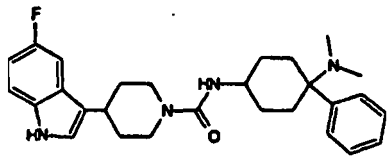 | Hydrochlorid | Unpolareres Diastereoisomer |
| **34** | 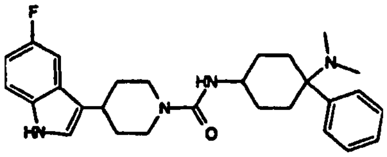 | Hydrochlorid | Polareres Diastereoisomer |
| **35** | 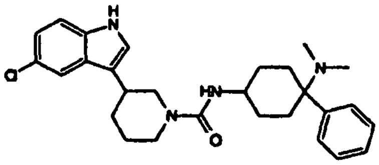 | Hydrochlorid | Unpolareres Diastereoisomer |
| **36** | 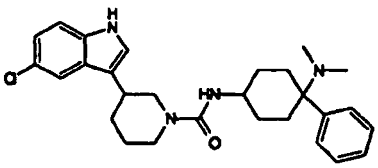 | Hydrochlorid | Polareres Diastereoisomer |
| **37** | 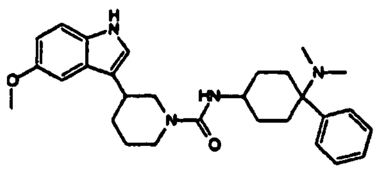 | Hydrochlorid | Unpolareres Diastereoisomer |
| **38** | 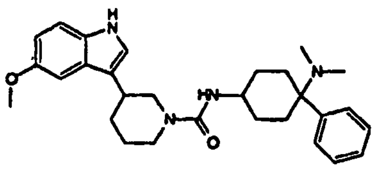 | Hydrochlorid | Polareres Diastereoisomer |
| **39** | 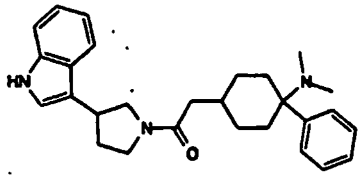 | Hydrochlorid | Eins von zwei Diastereoisomeren |
| **40** | 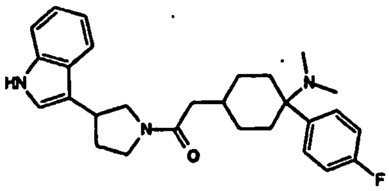 | Hydrochlorid | Unpolareres Diastereoisomer |

(fortgesetzt)

| Beispiel Nr. | Struktur | Salzform | Kommentare |
|---|---|---|---|
| 41 | 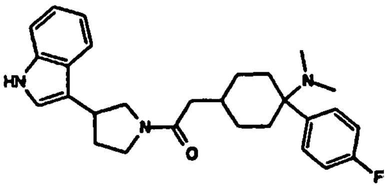 | Hydrochlorid | Polareres Diastereoisomer |
| 42 | 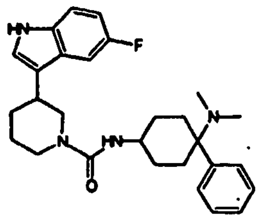 | Hydrochlorid | Polareres Diastereoisomer |
| 43 | 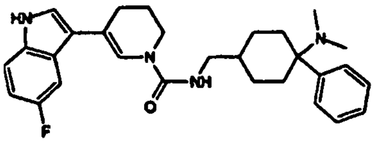 | Citrat | Unpolareres Diastereoisomer |
| 44 | 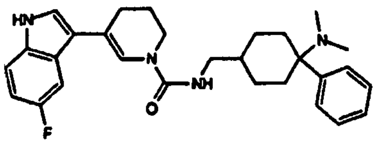 | Citrat | Polareres Diastereoisomer |
| 45 | 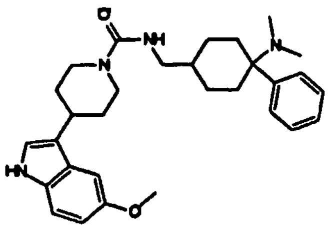 | Citrat | Unpolareres Diastereoisomer |
| 46 | 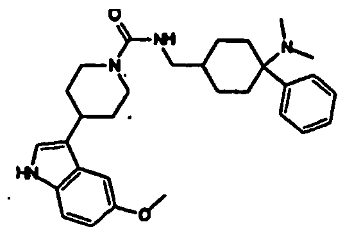 | Citrat | Polareres Diastereoisomer |
| 47 | 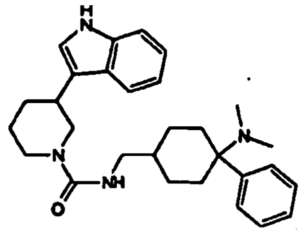 | Citrat | Unpolareres Diastereoisomer |

(fortgesetzt)

| Beispiel Nr. | Struktur | Salzform | Kommentare |
|---|---|---|---|
| 48 | 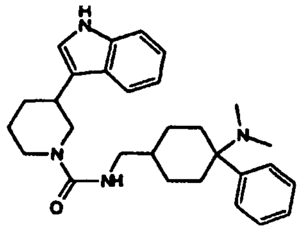 | Citrat | Polareres Diastereoisomer |
| 49 | 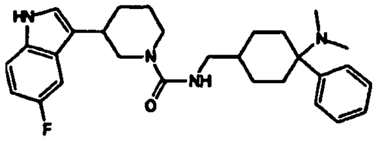 | Citrat | Unpolareres Diastereoisomer |
| 50 | 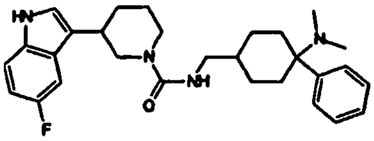 | Citrat | Polareres Diastereoisomer |
| 51 | 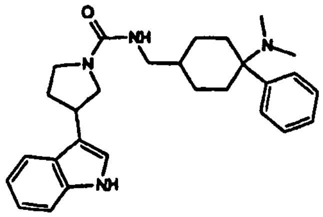 | Citrat | Unpolareres Diastereoisomer |
| 52 | 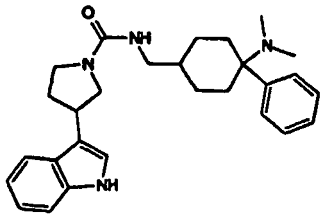 | Citrat | Polareres Diastereoisomer |
| 53 | 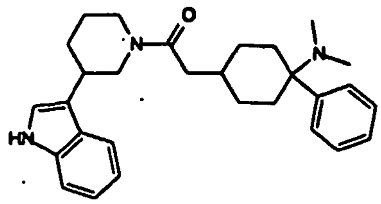 | Hydrochlorid | Diastereoisomerengemisch |
| 54 | 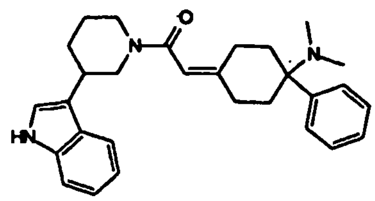 | Hydrochlorid | Diastereoisomerengemisch |

(fortgesetzt)

| Beispiel Nr. | Struktur | Salzform | Kommentare |
|---|---|---|---|
| **55** | 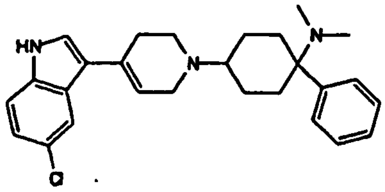 | Dihydrochlorid | Diastereoisomerengemisch |
| **56** | 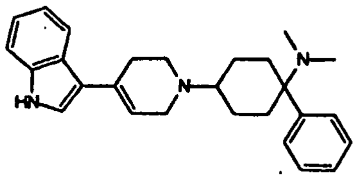 | Dihydrochlorid | Diastereoisomerengemisch |
| **57** | 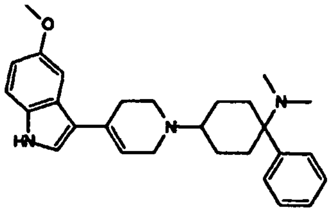 | Dihydrochlorid | Diastereoisomerengemisch |
| **58** | 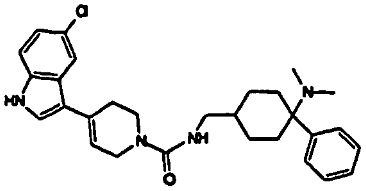 | Hydrochlorid | Unpolareres Diastereoisomer |
| **59** | 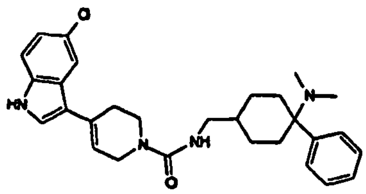 | Hydrochlorid | Polareres Diastereoisomer |
| **60** | 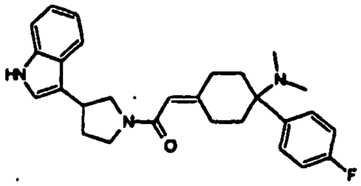 | Hydrochlorid | Diastereoisomerengemisch |
| **61** | 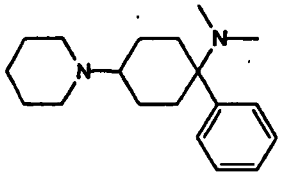 | Dihydrochlorid | Polareres Diastereoisomer |

**Untersuchungen zur Wirksamkeit der erfindungsgemäßen Verbindungen:**

Die in den folgenden Assays und Modellen erhobenen Daten sind in Tabelle 1 zusammengefaßt.

**Messung der ORL1-Bindung**

Die Cyclohexan-Derivate der allgemeinen Formel I wurden in einem Rezeptorbindungsassay-mit $^3$H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3$H-Nociceptin/Orphanin FQ betrug bei diesen Versuchen 0.5 nM. Die Bindungsassays wurden mit je 20 μg Membranprotein je 200 μl Ansatz in 50 mM Hepes, pH 7,4, 10 mM $MgCl_2$ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer $K_i$-Wert in oder % Inhibition bei c=1 μM angegeben.

**Messung der μ-Bindung**

Die Rezeptoraffinität zum humanen μ-Opiatrezeptor wurde in einem homogenen Ansatz in Mikrotiterplatten bestimmt. Hierzu wurden Verdünnungsreihen des jeweils zu prüfenden substituierten spirocyclischen Cyclohexan-Derivates mit einer Rezeptormembranpräparation (15-40 μg Protein pro 250 μl Inkubationsansatz) von CHO-K1-Zellen, welche den humanen μ-Opiatrezeptor exprimieren (RB-HOM-Rezeptormembran-Präparation der Firma NEN, Zaventem, Belgien) in Gegenwart von 1 nmol/l des radioaktiven Liganden [$^3$H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 μl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 μmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen μ-Opiatrezeptor bei einer Konzentration der Prüfsubstanzen von 1 μmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I $IC_{50}$ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten. In einzelnen Fällen (in Tabelle 2 durch DA gekennzeichnet) wurde wurde gegen [D-Ala2,MePhe4,Gly-ol5]enkephalin ("DAMGO") anstelle von Naloxon gemessen.

**Analgesierprüfung im Tail-Flick-Test an der Maus**

Die Mäuse wurden jeweils einzeln in einen Testkäfig gesetzt und die Schwanzbasis dem fokussierten Wärmestrahl einer elektrischen Lampe (Tall-flick-Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Mäusen 3 bis 5 Sekunden betrug. Vor der Applikation der Lösungen enthaltend die erfindungsgemäße Verbindung bzw. der jeweiligen Vergleichslösungen wurden die Mäuse innerhalb von fünf Minuten zweimal vorgetestet und der Mittelwert dieser Messungen als Vortestmittelwert berechnet.

Die Lösungen der erfindungsgemäßen Verbindung der allgemeinen Formel I sowie die Vergleichslösungen wurden dann intravenös appliziert. Die Schmerzmessung wurde jeweils 10, 20, 40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$[(T_1-T_0)/(T_2-T_0)] \times 100$$

Hierbei ist die Zeit $T_0$ die Latenzzeit vor der Applikation, die Zeit $T_1$ die Latenzzeit nach der Applikation der Wirkstoffkombination und die Zeit $T_2$ die maximale Expositionsdauer (12 Sekunden).

**Tabelle 2:**

| **Beispiel Nr.** | **ORL1** Ki [μM] oder % Hemmung [1 μM] | μ Ki [μM] Oder % Hemmung [1 μM] | **TaU Flick** (Maus, i.v.) %Hemmung (Dosis [mg/kg]) |
|---|---|---|---|
| **1** | **0,0038** | **0,0028** | |
| **2** | **0,012** | **0,0025** | |
| **3** | **0,032** | **0,019** | |
| **4** | **0,054** | **53 %** | |
| **5** | **0,069** | **36 %** | |
| **6** | **0,047** | **0,1** | |
| **7** | **0,065** | **0,039** | |
| **8** | **54 %** | **0,54** | |
| **9** | **0,0029** | **0,013** | |
| **10** | **0,1** | **61 %** | |
| **11** | **0,02** | **0,0008** | |
| **12** | **10 %** | **66 %** | |
| **13** | **0,087** | **0,022** | |
| **14** | **63 %** | **52 % (DA)** | |
| **15** | **0,1** | **0,03 (DA)** | |
| **16** | **57 %** | **0,027** | |
| **17** | **0,21** | **0,0685** | |
| **18** | **0,068** | **0,024** | |
| **19** | **0,075** | **0,021** | |
| **20** | **46 %** | **0,026** | |
| **21** | **47 %** | **0,083 (DA)** | |
| **22** | **0,083** | **0,11 (DA)** | |
| **23** | **51 %** | **0,048** | |
| **24** | **0,058** | **0,027** | |
| **25** | **0,039** | **0,031 (DA)** | |
| **26** | **0,095** | **0,041** | |
| **27** | **0,11** | **0,021** | |
| **28** | **0,067** | **0,024 (DA)** | |
| **29** | **46 %** | **0,0071** | |
| **30** | **47 %** | **0,28** | |
| **31** | **59 %** | **0,013** | |
| **32** | **39 %** | **0,031** | |
| **33** | **62 %** | **0,0083** | |
| **34** | **66 %** | **0,12** | |
| **35** | **0,033** | **0,0081** | |
| **36** | **26 %** | **56 %** | |
| **37** | **0,083** | **0,019** | |

(fortgesetzt)

| **Beispiel Nr.** | **ORL1** Ki [µM] oder % Hemmung [1 µM] | µ Ki [µM] Oder % Hemmung [1 µM] | **TaU Flick** (Maus, i.v.) %Hemmung (Dosis [mg/kg]) |
|---|---|---|---|
| **38** | **13 %** | **0,41** | |
| **39** | **0,021** | **0,0022** | |
| **40** | **14 %** | **0,022** | |
| **41** | **55 %** | **0,0059** | |
| **42** | **34 %** | **57 %** | |
| **43** | **0,16** | **0,015 (DA)** | |
| **44** | **0,11** | **0,0057 (DA)** | |
| **45** | **52 %** | **0,015** | |
| **46** | **0,092** | **0,07** | |
| **47** | **56 %** | **0,032** | |
| **48** | **0,091** | **0,0021** | **100 (21,5)** |
| **49** | **61 %** | **0,022** | |
| **50** | **0,068** | **0,0033** | |
| **51** | **69 %** | **0,018** | |
| **52** | **0,045** | **0,01** | |
| **53** | **0,056** | **0,0031** | |
| **54** | **0,043** | **0,0079** | **100 % (10)** |
| **55** | **0,095** | **85 %** | |
| **56** | **0,17** | **90 %** | |
| **57** | **0,15** | **91 %** | |
| **58** | **0,12** | **0,031 (DA)** | |
| **59** | **0,082** | **0,038 (DA)** | |
| **60** | **45 %** | **0,022** | |
| **61** | **0,11** | **23 % (DA)** | |

**Parenterale Lösung eines erfindungsgemäßen 4-substituierte 1-Aminocyclohexan-Derivats**

38 g eines der erfindungsgemäßen 4-substituierte 1-Aminocyclohexan-Derivate, hier Beispiel 1, wird in 1 l Wasser für Injektionszwecke bei Raumtemperatur gelöst und anschließend durch Zugabe von wasserfreier Glukose für Injektionszwecke auf isotone Bedingungen eingestellt.

## Patentansprüche

**1.** 4-substituierte 1-Aminocyclohexan-Derivate der allgemeinen Formel I,

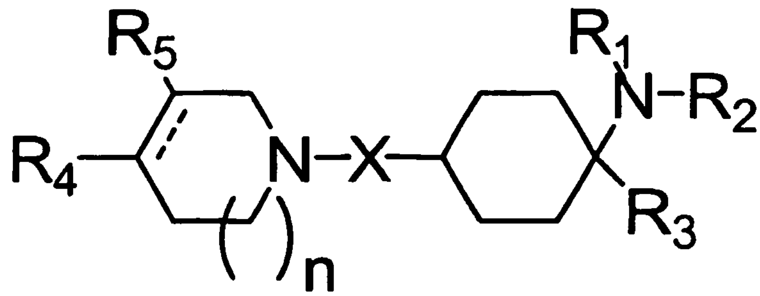

, worin

n = 0 oder 1,
X = eine Bindung, oder C(O), C(O)NH, C(O)$CH_2$, C(O)CH= oder C(O)NH$CH_2$,
$R^1$ und $R^2$ unabhängig voneinander für H; Methyl oder Ethyl stehen oder die Reste $R^1$ und $R^2$ zusammen einen Ring bilden und $(CH_2)_5$ bedeuten,
$R^3$ für $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert, steht;
$R^4$ und $R^5$ unabhängig voneinander für H; oder $(CH_2)_mR^7$ stehen
wobei m = 0-6 und $R^7$ ausgewählt aus H; $C_{3-8}$-Cycloalkyl, Aryl oder Heteroaryl, jeweils unsubstituiert oder einfach oder mehrfach substituiert, bedeutet, wobei nur einer der Reste $R^4$ und $R^5$ H sein kann,
mit der Maßgabe, dass wenn n = 1, einer der Reste $R^1$ oder $R^2$ $CH_3$ und der andere Rest $R^1$ oder $R^2$ H bedeutet, $R^3$ Phenyl und $R^5$ H bedeutet, der Rest $R^4$ nicht 4-Fluorphenyl bedeutet,

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

**2.** 4-substituierte 1-Aminocyclohexan-Derivate gemäß Anspruch 1, **dadurch gekennzeichnet, dass** $R^1$ und $R^2$ unabhängig voneinander für $CH_3$ oder H stehen, wobei $R^1$ und $R^2$ nicht gleichzeitig H bedeuten.

**3.** 4-substituierte 1-Aminocyclohexan-Derivate gemäß einem der Ansprüche 1-2, **dadurch gekennzeichnet, dass** $R^3$ für $C_{3-8}$-Cycloalkyl, Aryl oder Heteroaryl, jeweils unsubstituiert oder einfach oder mehrfach substituiert: über eine gesättigte oder ungesättigte, unverzweigte, substituierte oder unsubstituierte $C_{1-2}$-Alkyl-Gruppe gebundenen Aryl, $C_{3-8}$-Cycloalkyl oder Heteroaryl, jeweils unsubstituiert oder einfach oder mehrfach substituiert, steht;
vorzugsweise
$R^3$ Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert: über eine gesättigte, unverzweigte $C_{1-2}$-Alkyl-Gruppe gebundenen $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, bedeutet;
insbesondere
$R^3$ Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl oder Benzothiophenyl, jeweils unsubstituiert oder einfach oder mehrfach substituiert; über eine gesättigte, unverzweigte $C_{1-2}$-Alkyl-Gruppe gebundenen Phenyl, Furyl oder Thiophenyl, jeweils unsubstituiert oder einfach oder mehrfach substituiert, bedeutet

**4.** 4-substituierte 1-Aminocyclohexan -Derivate gemäß einem der Ansprüche 1 bis 2, **dadurch gekennzeichnet, dass** $R^3$ für Phenyl, Thiophenyl, Pyridyl oder Benzyl, jeweils substituiert oder unsubstituiert, steht, besonders bevorzugt für Phenyl.

5. 4-Substituierte 1-Aminocyclohexan-Derivate gemäß einem der Ansprüche 1 bis 4, worin $R^7$ H; Cyclopentyl, Cyclohexyl, Cycloheptyl, Cyclooctyl, Indolyl, Naphthyl, Benzofuranyl, Benzothiophenyl, Indanyl, Acenaphthyl, Phenyl, Thiophenyl, Furyl, Pyridyl, Pyrrolyl, Pyrazinyl oder Pyrimidyl, jeweils unsubstituiert oder einfach oder mehrfach substituiert, bedeutet.

6. 4-Substituierte 1-Aminocyclohexan-Derivate gemäß einem der Ansprüche 1 bis 5, **dadurch gekennzeichnet, dass** $R^7$ für Indolyl, unsubstituiert oder einfach oder mehrfach substituiert, steht.

7. 4-Substituierte 1-Aminocyclohexan-Derivate gemäß einem der Ansprüche 1 bis 6 6 aus der Gruppe
{4-[3-(1*H*-Indol-3-yl)-pyrrolidin-1-yl]-1-phenyl-cyclohexyl}-dimethylamin; dihydrochlorid (unpolareres Diastereoisomer)
{4-[3-(1*H*-Indol-3-yl)-pyrrolidin-1-yl]-1-phenyl-cyclohexyl}-dimethylamin; dihydrochlorid (Diastereoisomerengemisch)
{4-[4-(5-Chlor-1*H*-indol-3-yl)-piperidin-1-yl]-1-phenyl-cyclohexyl}-dimethylamin; dihydrochlorid (unpolareres Diastereoisomer)
{4-[4-(5-Chlor-1H-indol-3-yl)-piperidin-1-yl]-1-phenyl-cyclohexyl}-dimethylamin; dihydrochlorid (polareres Diastereoisomer)
{4-[4-(1H-Indol-3-yl)-piperidin-1-yl]-1-phenyl-cyclohexyl}-dimethylamin; dihydrochlorid (polareres Diastereoisomer)
{4-[4-(1*H*-Indol-3-yl)piperidin-1-yl]-1-phenylcyclohexyl}-dimethylamin; dihydrochlorid (unpolareres Diastereoisomer)
{4-[4-(5-Methoxy-1H-indol-3-yl)-piperidin-1-yl]-1-phenyl-cyclohexyl}-dimethylamin; dihydrochlorid (unpolareres Diastereoisomer)
{4-[4-(5-Methoxy-1*H*-indol-3-yl)piperidin-1-yl]-1-phenylcyclohexyl}-dimethylamin; dihydrochlorid (polareres Diastereoisomer)
{4-[3-(1H-Indol-3-yl)-piperidin-1-yl]-1-phenyl-cyclohexyl}-dimethylamin; dihydrochlorid (unpolareres Diastereoisomer)
{4-[3-(1*H*-Indol-3-yl)piperidin-1-yl]-1-phenylcyclohexyl}-dimethylamin; dihydrochlorid (polareres Diastereoisomer)
2-(4-Dimethylamino-4-phenylcyclohexyliden)-1-[3-(1*H*-indol-3-yl)pyrrolidin-1-yl]ethanon; hydrochlorid (Diastereoisomerengemisch)
{1-(4-Fluorphenyl)-4-[3-(1*H*-indol-3-yl)pyrrolidin-1-yl]cyclohexyl}-dimethylamin; dihydrochlorid (polareres und unpolareres Diastereoisomer)
4-(5- Methoxy- 1*H*-indol- 3- yl)- 3,6- dihydro- 2*H*-pyridin- 1- carbonsäure-(4- dimethylamino- 4- phenylcyclohexylmethyl)-amid; citrat (polareres und unpolareres Diastereoisomer)
4-(1*H*-Indol-3-yl)piperidin-1-carbonsäure-(4-dimethylamino-4-phenylcyclohexylmethyl)-amid; hemicitrat bzw. -citrat (polareres und unpolareres Diastereoisomer)
4-(5-Chlor-1*H*-indol-3-yl)piperidin-1-carbonsäure-(4-dimethylamino-4-phenylcyclohexylmethyl}-amid; citrat (polareres Diastereoisomer) bzw. -hemicitrat (unpolareres Diastereoisomer)
4-(1*H*-indol)-3-yl)-3,6-dihydro-2*H*-pyridin-1-carbonsäure-(4-dimethylamino-4-phenylcyclohexylmethyl)-amid; citrat (polareres und unpolareres Diastereoisomer)
4-(5-Fluor-1*H*-indol-3-yl)piperidin-1-carbonsäure-(4-dimethylamino-4-phenylcyclohexylmethyl)-amid; hemicitrat bzw. -citrat (polareres und unpolareres Diastereoisomer)
2-(4-Dimethylamino-4-phenylcyclohexyliden)-1-[4-(1*H*-indol-3-yl)-3,6-dihydro-2*H*-pyridin-1-yl]ethanon; Hydrochlorid
2-(4-Dimethylamino-4-phenylcyclohexyliden)-1-[4-(1*H*-indol-3-yl)-piperidin-1-yl]ethanon; Hydrochlorid
1-[4-(5-Chlor-1*H*-indol-3-yl)piperidin-1-yl]-2-(4-dimethylamino-4-phenylcyclohexyliden)ethanon; Hydrochlorid
2-(4-Dimethylamino-4-phenylcyclohexyliden)-1-[4-(5-methoxy-1*H*-indol-3-yl)-3,6-dihydro-2*H*-pyridin-1-yl]ethanon; Hydrochlorid
4-(1*H*-indol-3-yl)piperidin-1-carbonsäure-(4-dimethylamino-4-phenylcyclohexyl)-amid; Hydrochlorid (polareres und unpolareres Diastereoisomer)
4-(5-Chlor-1*H*-indol-3-yl)piperidin-1-carbonsäure-(4-dimethylamino-4-phenylcyclohexyl)-amid; Hydrochlorid (unpolareres Diastereoisomer)
4-(5-Methoxy-1*H*-indol-3-yl)piperidin-1-carbonsäure-(4-dimethylamino-4-phenylcyclohexyl)-amid; Hydrochlorid (unpolareres Diastereoisomer)
4-(5-Fluor-1*H*-indol-3-yl)piperidin-1-carbonsäure-(4-dimethylamino-4-phenylcyclohexyl)-amid; Hydrochlorid (polareres und unpolareres Diastereoisomer)
3-(5-Chlor-1*H*-indol-3-yl)piperidin-1-carbonsäure-(4-dimethylamino-4-phenylcyclohexyl)-amid; Hydrochlorid (polareres und unpolareres Diastereoisomer)
3-(5-Methoxy-1*H*-indol-3-yl)piperidin-1-carbonsäure-(4-dimethylamino-4-phenylcyclohexyl)-amid; Hydrochlorid

(polareres und unpolareres Diastereoisomer)
2-(4-Dimethylamino-4-phenylcyclohexyl)-1-[3-(1*H*-indol-3-yl)pyrrolidin-1-yl]-ethanon; Hydrochlorid
2-[4-Dimethylamino-4-(4-fluorphenyl)cyclohexyl]-1-[3-(1*H*-indol-3-yl)pyrrolidin-1-yl]-ethanon; Hydrochlorid (polareres und unpolareres Diastereoisomer)
3-(5-Fluor-1*H*-Indol-3-yl)piperidin-1-carbonsäure-(4-dimethylamino-4-phenylcyclohexyl)-amid; Hydrochlorid (polareres Diastereoisomer)
4-(5- Fluor- 1*H*-indol- 3- yl)- 3,6- dihydro- 2*H*-pyridin- 1- carbonsäure-(4- dimethylamino- 4- phenylcyclohexylmethyl)-amid; citrat (polareres und unpolareres Diastereoisomer)
4-(5-Methoxy-1*H*-indol-3-yl)piperidin-1-carbonsäure-(4-dimethylamino-4-phenylcyclohexylmethyl)-amid; citrat (polareres und unpolareres Diastereoisomer)
3-(1*H*-Indol-3-yl)piperidin-1-carbonsäure-(4-dimethylamino-4-phenylcyclohexylmethyl)-amid; citrat (polareres und unpolareres Diastereoisomer)
3-(5-Fluor-1*H*-indol-3-yl)piperidin-1-carbonsäure-(4-dimethylamino-4-phenylcyclohexylmethyl)-amid; citrat (polareres und unpolareres Diastereoisomer)
3-(1*H*-Indol-3-yl)pyrrolidin-1-carbonsäure-(4-dimethylamirto-4-phenylcyclohexylmethyl)-amid; citrat (polareres und unpolareres Diastereoisomer)
2-(4-Dimethylamino-4-phenylcyclohexyl)-1-[3-(1*H*-indol-3-yl)piperidin-1-yl]-ethanon; Hydrochlorid (Diastereoisomerengemisch)
2-(4-Dimethylamino-4-phenylcyclohexyliden)-1-[3-(1*H*-indol-3-yl)-piperidin-1-yl]-ethanon; Hydrochlorid (Diastereoisomerengemisch)
4-[4-(5-Chlor-1*H*-indol-3-yl)-3,6-dihydro-2*H*-pyridin-1-yl]-1-phenylcyclohexyl-dimethylamin; diHydrochlorid (Diastereoisomerengemisch)
{4-[4-(1*H*-Indol-3-yl)-3,6-dihydro-2*H*-pyridin-1-yl]-1-phenylcyclohexyl}-dimethylamin; dihydrochlorid (Diastereoisomerengemisch)
{4-[4-(5-Methoxy-1*H*-indol-3-yl)-3,6-dihydro-2*H*-pyridin-1-yl]-1-phenylcyclohexyl}-dimethylamin; dihydrochlorid (Diastereoisomerengemisch)
4-(5- Chlor- 1*H*-indol- 3- yl)- 3,6- dihydro- 2H- pyridin- 1- carbonsäure-(4- dimethylamino- 4- phenyl- cyclohexylmethyl)-amid; Hydrochlorid (unpolareres und polareres Diastereoisomer)
2-(4-Dimethylamino-4-(4-fluorphenyl)cyclohexyliden)-1-[3-(1 H-indol-3-yl)pyrrolidin-1-yl]-ethanon; Hydrochlorid (Diastereoisomerengemisch)

**8.** Arzneimittel enthaltend mindestens ein 4-substituierte 1-Aminocyclohexan-Derivat gemäß einem der Ansprüche 1 bis 7 sowie gegebenenfalls geeignete Zusatz- und/oder Hilfsstoffe und/oder gegebenenfalls weitere Wirkstoffe.

**9.** Verwendung eines 4-substituierten 1-Aminocyclohexan-Derivats gemäß einem der Ansprüche 1 bis 7 zur Herstellung eines Arzneimittels zur Behandlung von Schmerz, insbesondere von akutem, viszeralem, neuropathischem oder chronischem Schmerz.

**10.** Verwendung eines 4-substituierten 1-Aminocyclohexan-Derivats gemäß einem der Ansprüche 1 bis 7 zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

**11.** Verfahren zur Herstellung von 4-substituierten 1-Aminocyctohexan-Derivatengemäß einem der Ansprüche 1-7, **dadurch gekennzeichnet, dass** ein Pyrrolidin- oder Piperidin-Derivat

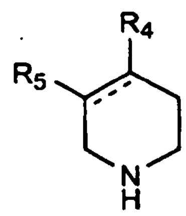 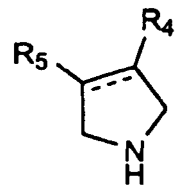

mit einem geeigneten 4-Aminocyclohexanon- oder 4-Aminocyclohexancarbaldehyd unter Bedingungen zur reduktiven Aminierung, beispielweise mit Hydriden wie Natrium- oder Lithiumborhydrid, Natriumcyanoborhydrid, Natriumtriacetoxyborhydrid, Diisobutylaluminiumhydrid, Lithium-tri-(sec.-butyl)borhydrid oder Lithiumaluminiumhydrid, umgesetzt werden.

**12.** Verfahren zur Herstellung von 4-substituierten 1-Aminocyclohexan-Derivatengemäß einem der Ansprüche 1-7, **dadurch gekennzeichnet, dass** ein Pyrrolidin- oder Piperidin-Derivat

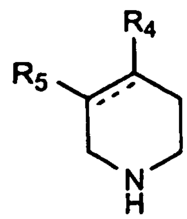 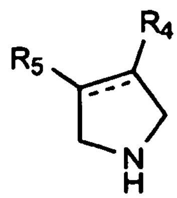

mit einem geeigneten 4-Amino-cyclohexancarbonsäure- oder 4-Aminocyclohexylessigsäure-Derivat unter Einsatz von Kupplungsreagenzien oder nach Überführung der Carbonsäuren in ein Säurechlorid oder einen Aktivester, beispielsweise einen 4-Nitrophenylester oder einen N-Hydroxysuccinimidester, umgesetzt wird.

**13.** Verfahren zur Herstellung von 4-substituierten 1-Aminocyclohexan-Derivatengemäß einem der Ansprüche 1-7, **dadurch gekennzeichnet, dass** ein Pyrrolidin- oder Piperidin-Derivat

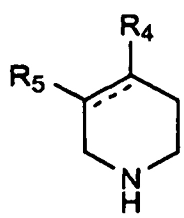 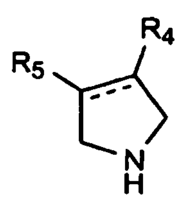

mit einem geeigneten (4-Amino-cyclohexyl)-carbaminsäurephenylester- oder (4-Amino-cyclohexyl)methyl-carbaminsäurephenylester -Derivat, das durch Umsetzung von Chlorameisensäurephenylester und einem 1,4-Diaminocyclohexan- oder 4-Aminomethyl-cyclohexylamin-Derivat hergestellt wird, bei einer Temperatur von 50-130°C umgesetzt wird.

**Claims**

**1.** 4-substituted 1-aminocyclohexane derivatives of the general formula I,

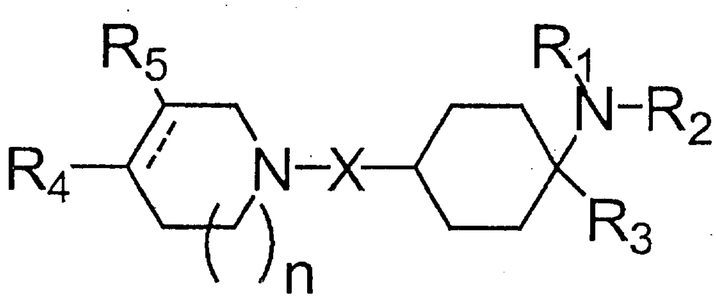

,

wherein

n = 0 or 1,
X denotes a bond or C(O), C(O)NH, $C(O)CH_2$, C(O)CH= or $C(O)NHCH_2$,
$R^1$ and $R^2$ independently of one another denote H; methyl or ethyl, or the radicals $R_1$ and $R_2$ together form a ring and denote $(CH_2)_5$,
$R^3$ denotes $C_{1-8}$-alkyl or $C_{3-8}$-cycloalkyl, in each case saturated or unsaturated, branched or unbranched, singly or multiply substituted or unsubstituted; aryl, heteroaryl, in each case unsubstituted or singly or multiply substituted; aryl, $C_{3-8}$-cycloalkyl or heteroaryl bonded via a saturated or unsaturated, branched or unbranched, substituted or unsubstituted $C_{1-4}$-alkyl group, in each case unsubstituted or singly or multiply substituted;
$R^4$ and $R^5$ independently of one another denote H; or $(CH_2)_mR^7$,
in which m = 0-6 and $R^7$ denotes H; $C_{3-8}$-cycloalkyl, aryl or heteroaryl, in each case unsubstituted or singly or multiply substituted, wherein only one of the radicals $R_4$ and $R_5$ can be H,

with the proviso that if n = 1, one of $R^1$ and $R^2$ is $CH_3$ and the other one of $R^1$ and $R^2$ is H, $R^3$ is phenyl and $R^5$ is H, $R^4$ is not 4-fluoro-phenyl,
optionally in the form of their racemates, their pure stereoisomers, in particular enantiomers or diastereomers, or in the form of mixtures of the stereoisomers, in particular of the enantiomers or diastereomers, in an arbitrary mixture ratio; in the form of their acids or their bases or in the form of their salts, in particular of the physiologically compatible salts or salts of physiologically compatible acids or cations; or in the form of their solvates, in particular the hydrates.

2. 4-substituted 1-aminocyclohexane derivatives according to claim 1, **characterised in that** $R^1$ and $R^2$ independently of one another denote $CH_3$ or H, wherein $R^1$ and $R^2$ do not simultaneously denote H.

3. 4-substituted 1-aminocyclohexane derivatives according to one of claims 1 and 2, **characterised in that**
$R^3$ denotes $C_{3-8}$-cycloalkyl, aryl or heteroaryl, in each case unsubstituted or singly or multiply substituted; aryl, $C_{3-8}$-cycloalkyl or heteroaryl bonded via a saturated or unsaturated, unbranched, substituted or unsubstituted $C_{1-2}$-alkyl group, in each case unsubstituted or singly or multiply substituted;
preferably
$R^3$ denotes cyclopentyl, cyclohexyl, phenyl, benzyl, naphthyl, anthracenyl, thiophenyl, benzothiophenyl, furyl, benzofuranyl, benzodioxolanyl, indolyl, indanyl, benzodioxanyl, pyrrolyl, pyridyl, pyrimidyl or pyrazinyl, in each case unsubstituted or singly or multiply substituted; $C_{5-6}$-cycloalkyl, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl, bonded via a saturated, unbranched $C_{1-2}$-alkyl group, in each case unsubstituted or singly or multiply substituted;
in particular
$R^3$ denotes phenyl, furyl, thiophenyl, naphthyl, benzyl, benzofuranyl, indolyl, indanyl, benzodioxanyl, benzodioxolanyl, pyridyl, pyrimidyl, pyrazinyl or benzothiophenyl, in each case unsubstituted or singly or multiply substituted; phenyl, furyl or thiophenyl, bonded via a saturated, unbranched $C_{1-2}$-alkyl group, in each case unsubstituted or singly or multiply substituted.

4. 4-substituted 1-aminocyclohexane derivatives according to one of claims 1 and 2, **characterised in that** $R^3$ denotes phenyl, thiophenyl, pyridyl or benzyl, in each case substituted or unsubstituted, and particularly preferably denotes phenyl.

5. 4-substituted 1-aminocyclohexane derivatives according to one of claims 1 to 4, wherein $R^7$ denotes H; cyclopentyl, cyclohexyl,cycloheptyl, cyclooctyl, indolyl, naphthyl, benzofuranyl, benzothiophenyl, indanyl, acenaphthyl, phenyl,

thiophenyl, furyl, pyridyl, pyrrolyl, pyrazinyl or pyrimidyl, in each case unsubstituted or singly or multiply substituted.

6. 4-substituted 1-aminocyclohexane derivatives according to one of claims 1 to 5, **characterised in that** $R^7$ denotes indolyl, unsubstituted or singly or multiply substituted.

7. 4-substituted 1-aminocyclohexane derivatives according to one of claims 1 to 6 from the following group:

{4-[3-(1*H*-indol-3-yl)-pyrrolidine-1-yl]-1-phenylcyclohexyl}-dimethylamine; dihydrochloride (non-polar diastereoisomer)
{4-[3-(1*H*-indol-3-yl)-pyrrolidine-1-yl]-1-phenylcyclohexyl}-dimethylamine; dihydrochloride (diastereoisomer mixture)
{4-[4-(5-chloro-1*H*-indol-3-yl)-piperidine-1-yl]-1-phenylcyclohexyl}-dimethylamine; dihydrochloride (non-polar diastereoisomer)
{4-[4-(5-chloro-1*H*-indol-3-yl)-piperidine-1-yl]-1-phenylcyclohexyl}-dimethylamine; dihydrochloride (polar diastereoisomer)
{4-[4-(1H-indol-3-yl)-piperidine-1-yl]-1-phenylcyclohexyl}-dimethylamine; dihydrochloride (polar diastereoisomer)
{4-[4-(1*H*-indol-3-yl)piperidine-1-yl]-1-phenylcyclohexyl}-dimethylamine; dihydrochloride (non-polar diastereoisomer)
{4-[4-(5-methoxy-1H-indol-3-yl)-piperidine-1-yl]-1-phenylcyclohexyl}-dimethylamine; dihydrochloride (non-polar diastereoisomer)
{4-[4-(5-methoxy-1*H*-indol-3-yl)piperidine-1-yl]-1-phenylcyclohexyl}-dimethylamine; dihydrochloride (polar diastereoisomer)
{4-[3-(1H-indol-3-yl)-piperidine-1-yl]-1-phenylcyclohexyl}-dimethylamine; dihydrochloride (non-polar diastereoisomer)
{4-[3-(1*H*-indol-3-yl)piperidine-1-yl]-1-phenylcyclohexyl}-dimethylamine; dihydrochloride (polar diastereoisomer)
2-(4-dimethylamino-4-phenylcyclohexylidene)-1-[3-(1*H*-indol-3-yl)pyrrolidine-1-yl]ethanone; hydrochloride (diastereoisomer mixture)
{1-(4-fluorophenyl)-4-[3-(1*H*-indol-3-yl)pyrrolidine-1-yl]cyclohexyl}-dimethylamine; dihydrochloride (polar and non-polar diastereoisomer)
4-(5-methoxy-1*H*-indol-3-yl)-3,6-dihydro-2*H*-pyridine-1-carboxylic acid-(4-dimethylamino-4-phenylcyclohexylmethyl)-amide; citrate (polar and non-polar diastereoisomer)
4-(1*H*-indol-3-yl)piperidine-1-carboxylic acid-(4-dimethylamino-4-phenylcyclohexylmethyl)-amide; hemicitrate and citrate (polar and non-polar diastereoisomer)
4-(5-chloro-1*H*-indol-3-yl)piperidine-1-carboxylic acid-(4-dimethylamino-4-phenylcyclohexylmethyl)-amide; citrate (polar diastereoisomer) and hemicitrate (non-polar diastereoisomer)
4-(1*H*-indol-3-yl)-3,6-dihydro-2*H*-pyridine-1-carboxylic acid-(4-dimethylamino-4-phenylcyclohexylmethyl)-amide; citrate (polar and non-polar diastereoisomer)
4-(5-fluoro-1*H*-indol-3-yl)piperidine-1-carboxylic acid-(4-dimethylamino-4-phenylcyclohexylmethyl)-amide; hemicitrate and citrate (polar and non-polar diastereoisomer)
2-(4-dimethylamino-4-phenylcyclohexylidene)-1-[4-(1*H*-indol-3-yl)-3,6-dihydro-2*H*-pyridine-1-yl]ethanone; hydrochloride
2-(4-dimethylamino-4-phenylcyclohexylidene)-1-[4-(1*H*-indol-3-yl)-piperidine-1-yl]ethanone; hydrochloride
1-[4-(5-chloro-1*H*-indol-3-yl)piperidine-1-yl]-2-(4-dimethylamino-4-phenylcyclohexylidene)-ethanone; hydrochloride
2-(4-dimethylamino-4-phenylcyclohexylidene)-1-[4-(5-methoxy-1*H*-indol-3-yl)-3,6-dihydro-2*H*-pyridine-1-yl] ethanone; hydrochloride
4-(1*H*-indol-3-yl)piperidine-1-carboxylic acid-(4-dimethylamino-4-phenylcyclohexyl)-amide; hydrochloride (polar and non-polar diastereoisomer)
4-(5-chloro-1*H*-indol-3-yl)piperidine-1-carboxylic acid-(4-dimethylamino-4-phenylcyclohexyl)-amide; hydrochloride (non-polar diastereoisomer)
4-(5-methoxy-1*H*-indol-3-yl)piperidine-1-carboxylic acid-(4-dimethylamino-4-phenyl-cyclohexyl)-amide; hydrochloride (non-polar diastereoisomer)
4-(5-fluoro-1*H*-indol-3-yl)piperidine-1-carboxylic acid-(4-dimethylamino-4-phenylcyclohexyl)-amide; hydrochloride (polar and non-polar diastereoisomer)
3-(5-chloro-1*H*-indol-3-yl)piperidine-1-carboxylic acid-(4-dimethylamino-4-phenylcyclohexyl)-amide; hydrochloride (polar and non-polar diastereoisomer)

3-(5-methoxy-1*H*-indol-3-yl)piperidine-1-carboxylic acid-(4-dimethylamino-4-phenylcyclohexyl)-amide; hydrochloride (polar and non-polar diastereoisomer)
2-(4-dimethylamino-4-phenylcyclohexyl)-1-[3-(1*H*-indol-3-yl)pyrrolidine-1-yl]-ethanone; hydrochloride
2-[4-dimethylamino-4-(4-fluorophenyl)cyclohexyl]-1-[3-(1*H*-indol-3-yl)pyrrolidine-1-yl]-ethanone; hydrochloride (polar and non-polar diastereoisomer)
3-(5-fluoro-1*H*-indol-3-yl)piperidine-1-carboxylic acid-(4-dimethylamino-4-phenylcyclohexyl)-amide; hydrochloride (polar diastereoisomer)
4-(5-fluoro-1*H*-indol-3-yl)-3,6-dihydro-2*H*-pyridine-1-carboxylic acid-(4-dimethylamino-4-phenylcyclohexylmethyl)-amide; citrate (polar and non-polar diastereoisomer)
4-(5-methoxy-1*H*-indol-3-yl)piperidine-1-carboxylic acid-(4-dimethylamino-4-phenylcyclohexylmethyl)-amide; citrate (polar and non-polar diastereoisomer)
3-(1*H*-indol-3-yl)piperidine-1-carboxylic acid-(4-dimethylamino-4-phenylcyclohexylmethyl)-amide; citrate (polar and non-polar diastereoisomer)
3-(5-fluoro-1*H*-indol-3-yl)piperidine-1-carboxylic acid-(4-dimethylamino-4-phenylcyclohexylmethyl)-amide; citrate (polar and non-polar diastereoisomer)
3-(1*H*-indol-3-yl)pyrrolidine-1-carboxylic acid-(4-dimethylamino-4-phenylcyclohexylmethyl)-amide; citrate (polar and non-polar diastereoisomer)
2-(4-dimethylamino-4-phenylcyclohexyl)-1-[3-(1*H*-indol-3-yl)piperidine-1-yl]-ethanone; hydrochloride (diastereoisomer mixture)
2-(4-dimethylamino-4-phenylcyclohexylidene)-1-[3-(1*H*-indol-3-yl)-piperidine-1-yl]-ethanone; hydrochloride (diastereoisomer mixture)
4-[4-(5-chloro-1*H*-indol-3-yl)-3,6-dihydro-2*H*-pyridine-1-yl]-1-phenylcyclohexyldimethylamine; dihydrochloride (diastereoisomer mixture)
{4-[4-(1*H*-indol-3-yl)-3,6-dihydro-2*H*-pyridine-1-yl]-1-phenylcyclohexyl}-dimethylamine; dihydrochloride (diastereoisomer mixture)
{4-[4-(5-methoxy-1*H*-indol-3-yl)-3,6-dihydro-2*H*-pyridine-1-yl]-1-phenylcyclohexyl}-dimethylamine; dihydrochloride (diastereoisomer mixture)
4-(5-chloro-1*H*-indol-3-yl)-3,6-dihydro-2*H*-pyridine-1-carboxylic acid-(4-dimethylamino-4-phenylcyclohexylmethyl)-amide; hydrochloride (polar and non-polar diastereoisomer)
2-(4-dimethylamino-4-(4-fluorophenyl)cyclohexylidene)-1-[3-(1*H*-indol-3-yl)pyrrolidine-1-yl]-ethanone; hydrochloride (diastereoisomer mixture)

**8.** Medicament containing at least one 4-substituted 1-aminocyclohexane derivative according to one of claims 1 to 7 as well as optionally suitable additives and/or auxiliary substances and/or optionally further active constituents.

**9.** Use of a 4-substituted 1-aminocyclohexane derivative according to one of claims 1 to 7 for the production of a medicament for treating pain, in particular acute, visceral, neuropathic or chronic pain.

**10.** Use of a 4-substituted 1-aminocyclohexane derivative according to one of claims 1 to 7 for producing a medicament for the treatment of anxiety states, stress and syndromes associated with stress, depression, epilepsy, Alzheimer's disease, senile dementia, general cognitive dysfunctions, learning and memory disorders (as a nootropic), withdrawal symptoms, alcohol and/or drug and/or medicament misuse and/or dependency, sexual dysfunctions, cardiovascular conditions, hypotension, hypertension, tinnitus, pruritis, migraine, impaired hearing, lack of intestinal motility, eating disorders, anorexia, obesity, locomotor disturbances, diarrhoea, cachexia, urinary incontinence, or as a muscle relaxant, anticonvulsant or anaesthetic, or for combined administration in treatment with an opioid analgesic or with an anaesthetic, for diuresis or anti-natriuresis, anxiolysis, for modulating motor activity, for modulating neurotransmitter secretion and treating related neurodegenerative diseases, and for treating withdrawal symptoms and/or for reducing the addictive potential of opioids.

**11.** Process for the preparation of 4-substituted 1-aminocyclohexane derivatives according to one of claims 1 to 7, **characterised in that** a pyrrolidine or piperidine derivative

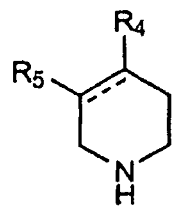

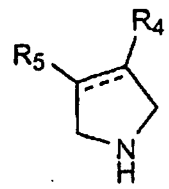

is reacted with a suitable 4-aminocyclohexanone carbaldehyde or 4-aminocyclohexane carbaldehyde under reductive amination conditions, for example with hydrides such as sodium or lithium boron hydride, sodium cyano boron hydride, sodium triacetoxy boron hydride, diisobutyl aluminium hydride, lithium-tri-(sec.-butyl)boron hydride or lithium aluminium hydride.

**12.** Process for the preparation of 4-substituted 1-aminocyclohexane derivatives according to one of claims 1 to 7, **characterised in that** a pyrrolidine or piperidine derivative

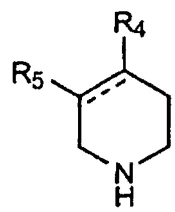

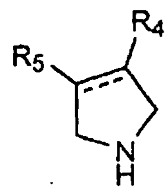

is reacted with a suitable 4-aminocyclohexane-carboxylic acid derivative or 4-aminocyclohexylacetic acid derivative with the use of coupling reagents or after conversion of the carboxylic acids into an acid chloride or an active ester, for example a 4-nitrophenyl ester or an N-hydroxysuccinimide ester.

**13.** Process for the preparation of 4-substituted 1-aminocyclohexane derivatives according to one of claims 1 to 7, **characterised in that** a pyrrolidine or piperidine derivative

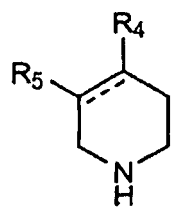

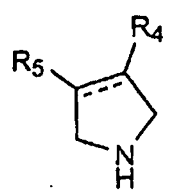

is reacted at a temperature of 50°-130°C with a suitable (4-aminocyclohexyl)-carbamic acid phenyl ester derivative or (4-aminocyclohexyl)-methylcarbamic acid phenyl ester derivative, which is prepared by reacting phenyl chloroformate and a 1,4-diaminocyclohexane derivative or 4-aminomethylcyclohexylamine derivative.

## Revendications

**1.** Dérivés de 1-aminocyclohexanes substitués en position 4 de formule générale I,

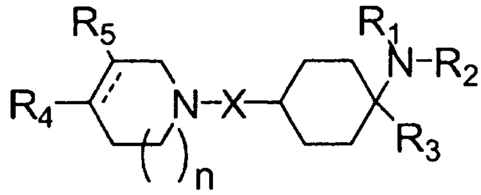

dans laquelle

n a la valeur 0 ou 1,
X représente une liaison ou un groupe C(O), C(O)NH, $C(O)CH_2$, C(O)CH= ou $C(O)NHCH_2$,
$R^1$ et $R^2$ représentent, indépendamment l'un de l'autre, H ; un reste méthyle ou éthyle, ou bien les restes $R^1$ et $R^2$ forment ensemble un noyau et représentent $(CH_2)_5$ ;
$R^3$ est un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste aryle, cycloalkyle en $C_3$ à $C_8$ ou hétéroaryle, lié par l'intermédiaire d'un groupe alkyle en $C_1$ à $C_4$ saturé ou non saturé, ramifié ou non ramifié, substitué ou non substitué, chacun non substitué ou substitué une ou plusieurs fois ;
$R^4$ et $R^5$ représentent, indépendamment l'un de l'autre, H ; ou un groupe $(CH_2)_mR^7$,
où m a une valeur de 0 - 6 et $R^7$ représente H ; un reste cycloalkyle en $C_3$ à $C_8$, aryle ou hétéroaryle, chacun non substitué ou substitué une ou plusieurs fois, un seul des restes $R^4$ et $R^5$ pouvant représenter H,
sous le réserve que
lorsque n = 1, l'un de $R^1$ ou $R^2$ représente $CH_3$ et l'autre de $R^1$ ou $R^2$ représente H, $R^3$ représente phényle et $R^5$ représente H, $R^4$ ne soit pas 4-fluoro- phényle,

le cas échéant sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous forme de leurs acides ou de leurs bases ou sous forme de leurs sels, en particulier des sels physiologiquement acceptables ou des sels d'acides ou de cations physiologiquement acceptables ; ou sous forme de leurs produits de solvatation, en particulier des hydrates.

2. Dérivés de 1-aminocyclohexanes substitués en position 4 selon la revendication 1, **caractérisés en ce que** $R^1$ et $R^2$ représentent, indépendamment l'un de l'autre, $CH_3$ ou H, $R^1$ et $R^2$ ne représentant pas en même temps H.

3. Dérivés de 1-aminocyclohexanes substitués en position 4 suivant l'une des revendications 1 et 2, **caractérisés en ce que**
$R^3$ représente un reste cycloalkyle en $C_3$ à $C_8$, aryle ou hétéroaryle, chacun non substitué ou substitué une ou plusieurs fois ; un reste aryle, cycloalkyle en $C_3$ à $C_8$ ou hétéroaryle, lié par l'intermédiaire d'un groupe alkyle en $C_1$ ou $C_2$ saturé ou non saturé, non ramifié, substitué ou non substitué, chacun non substitué ou substitué une ou plusieurs fois ;
avantageusement
$R^3$ représente un reste cyclopentyle, cyclohexyle, phényle, benzyle, naphtyle, anthracényle, thiophényle, benzothiophényle, furyle, benzofurannyle, benzodioxolannyle, indolyle, indanyle, benzodioxannyle, pyrrolyle, pyridyle, pyrimidyle ou pyrazinyle, chacun non substitué ou substitué une ou plusieurs fois ; un reste cycloalkyle en $C_5$ ou $C_6$, phényle, naphtyle, anthracényle, thiophényle, benzothiophényle, pyridyle, furyle, benzofurannyle, benzodioxolannyle, indolyle, indanyle, benzodioxannyle, pyrrolyle, pyrimidyle ou pyrazinyle lié par l'intermédiaire d'un groupe alkyle en $C_1$ ou $C_2$ saturé non ramifié, chacun non substitué
ou substitué une ou plusieurs fois ;
en particulier
$R^3$ représente un reste phényle, furyle, thiophényle, naphtyle, benzyle, benzofurannyle, indolyle, indanyle, benzodioxannyle, benzodioxolannyle, pyridyle, pyrimidyle, pyrazinyle ou benzothiophényle, chacun non substitué ou substitué une ou plusieurs fois ; un reste phényle, furyle ou thiophényle lié par l'intermédiaire d'un groupe alkyle en $C_1$ ou $C_2$ saturé non ramifié, chacun non substitué ou substitué une ou plusieurs fois.

4. Dérivés de 1-aminocyclohexanes substitués en position 4 suivant l'une des revendications 1 à 2, **caractérisés en ce que** $R^3$ représente un reste phényle, thiophényle, pyridyle ou benzyle, chacun substitué ou non substitué, très

avantageusement le reste phényle.

5. Dérivés de 1-aminocyclohexanes substitués en position 4 selon l'une des revendications 1 à 4, dans lesquels $R^7$ représente H ; un reste cyclopentyle, cyclohexyle, cycloheptyle, cyclo-octyle, indolyle, naphtyle, benzofurannyle, benzothiophényle, indanyle, acénaphtyle, phényle, thiophényle, furyle, pyridyle, pyrrolyle, pyrazinyle ou pyrimidyle, chacun non substitué ou substitué une ou plusieurs fois.

6. Dérivés de 1-aminocyclohexanes substitués en position 4 selon l'une des revendications 1 à 5, **caractérisés en ce que** $R^7$ représente un reste indolyle, non substitué ou substitué une ou plusieurs fois.

7. Dérivés de 1-aminocyclohexanes substitués en position 4 selon l'une des revendications 1 à 6, du groupe:

{4-[3-(1*H*-indole-3-yl)-pyrrolidine-1-yl]-1-phénylcyclohexyl}-diméthylamine ; dichlorhydrate (diastéréoisomère le moins polaire)
{4-[3-(1*H*-indole-3-yl)-pyrrolidine-1-yl]-1-phénylcyclohexyl}-diméthylamine ; dichlorhydrate (mélange de diastéréoisomères)
{4-[4-(5-chloro-1*H*-indole-3-yl)-pipéridine-1-yl]-1-phénylcyclohexyl}-diméthylamine ; dichlorhydrate (diastéréoisomère le moins polaire)
{4-[4-(5-chloro-1*H*-indole-3-yl)-pipéridine-1-yl]-1-phénylcyclohexyl}-diméthylamine ; dichlorhydrate (diastéréoisomère le plus polaire)
{4-[4-(1H-indole-3-yl)-pipéridine-1-yl]-1-phénylcyclohexyl}-diméthylamine ; dichlorhydrate (diastéréoisomère le plus polaire)
{4-[4-(1*H*-indole-3-yl)-pipéridine-1-yl]-1-phénylcyclohexyl}-diméthylamine ; dichlorhydrate (diastéréoisomère le moins polaire)
{4-[4-(5-méthoxy-1*H*-indole-3-yl)-pipéridine-1-yl]-1-phénylcyclohexyl}-diméthylamine ; dichlorhydrate (diastéréoisomère le moins polaire)
{4-[4-(5-méthoxy-1H-indole-3-yl)-pipéridine-1-yl]-1-phénylcyclohexyl}-diméthylamine ; dichlorhydrate (diastéréoisomère le plus polaire)
{4-[3-(1H-indole-3-yl)-pipéridine-1-yl]-1-phénylcyclohexyl}-diméthylamine ; dichlorhydrate (diastéréoisomère le moins polaire)
{4-[3-1*H*-indole-3-yl)-pipéridine-1-yl]-1-phénylcyclohexyl}-diméthylamine ; dichlorhydrate (diastéréoisomère le plus polaire)
2-(4-diméthylamino-4-phénylcyclohexylidène)-1-[3-(1*H*-indole-3-yl)-pyrrolidine-1-yl]-éthanone ; chlorhydrate (mélange de diastéréoisomères)
{1-(4-fluorophényl)-4-[3-(1*H*-indole-3-yl)-pyrrolidine-1-yl]-cyclohexyl}-diméthylamine ; dichlorhydrate (diastéréoisomère le plus polaire et diastéréoisomère le moins polaire)
(4-diméthylamino-4-phénylcyclohexylméthyl)-amide d'acide 4-(5-méthoxy-1*H*-indole-3-yl)-3,6-dihydro-2*H*-pyridine-1-carboxylique ; citrate (diastéréoisomère le plus polaire et diastéréoisomère le moins polaire)
(4-diméthylamino-4-phénylcyclohexylméthyl)-amide d'acide 4-(1*H*-indole-3-yl)-pipéridine-1-carboxylique; hémicitrate et citrate (diastéréoisomère le plus polaire et diastéréoisomère le moins polaire)
(4-diméthylamino-4-phénylcyclohexylméthyl)-amide d'acide 4-(5-chloro-1*H*-indole-3-yl)-pipéridine-1-carboxylique ; citrate (diastéréoisomère le plus polaire) et hémicitrate (diastéréoisomère le moins polaire)
(4-diméthylamino-4-phénylcyclohexylméthyl)-amide d'acide 4-(1*H*-indole-3-yl)-3,6-dihydro-2*H*-pyridine-1-carboxylique ; citrate (diastéréoisomère le plus polaire et diastéréoisomère le moins polaire)
(4-diméthylamino-4-phénylcyclohexylméthyl)-amide d'acide 4-(5-fluoro-1*H*-indole-3-yl)-pipéridine-1-carboxylique ; hémicitrate et citrate (diastéréoisomère le plus polaire et diastéréoisomère le moins polaire)
2-(4-diméthylamino-4-phénylcyclohexylidène)-1-[4-(1*H*-indole-3-yl)-3,6-dihydro-2*H*-pyridine-1-yl]-éthanone ; chlorhydrate
2-(4-diméthylamino-4-phénylcyclohexylidène)-1-[4-(1*H*-indole-3-yl)-pipéridine-1-yl]-éthanone ; chlorhydrate
1-[4-(5-chloro-1*H*-indole-3-yl)-pipéridine-1-yl]-2-(4-diméthylamino-4-phénylcyclohexylidène)-éthanone ; chlorhydrate
2-(4-diméthylamino-4-phénylcyclohexylidène)-1-[4-(5-méthoxy-1*H*-indole-3-yl)-3,6-dihydro-2*H*-pyridine-1-yl]-éthanone ; chlorhydrate
(4-diméthylamino-4-phénylcyclohexyl)-amide d'acide 4-(1*H*-indole-3-yl)-pipéridine-1-carboxylique ; chlorhydrate (diastéréoisomère le plus polaire et diastéréoisomère le moins polaire)
(4-diméthylamino-4-phénylcyclohexyl)-amide d'acide 4-(5-chloro-1*H*-indole-3-yl)-pipéridine-1-carboxylique ; chlorhydrate (diastéréoisomère le moins polaire)
(4-diméthylamino-4-phénylcyclohexyl)-amide d'acide 4-(5-méthoxy-1*H*-indole-3-yl)-pipéridine-1-carboxylique ;

chlorhydrate (diastéréoisomère le moins polaire)
(4-diméthylamino-4-phénylcyclohexyl)-amide d'acide 4-(5-fluoro-1*H*-indole-3-yl)-pipéridine-1-carboxylique ; chlorhydrate (diastéréoisomère le plus polaire et diastéréoisomère le moins polaire)
(4-diméthylamino-4-phénylcyclohexyl)-amide d'acide 3-(5-chloro-1*H*-indole-3-yl)-pipéridine-1-carboxylique ; chlorhydrate (diastéréoisomère le plus polaire et diastéréoisomère le moins polaire)
(4-diméthylamino-4-phénylcyclohexyl)-amide d'acide 3-(5-méthoxy-1*H*-indole-3-yl)-pipéridine-1-carboxylique; chlorhydrate (diastéréoisomère le plus polaire et diastéréoisomère le moins polaire)
2-(4-diméthylamino-4-phénylcyclohexyl)-1-[3-(1*H*-indole-3-yl)-pyrrolidine-1-yl]-éthanone ;chlorhydrate2-[4-(diméthylamino-4-(4-fluorophényl)-cyclohexyl]-1-[3-(1*H*-indole-3-yl)-pyrrolidine-1-yl]-éthanone ; chlorhydrate (diastéréoisomère le plus polaire et diastéréoisomère le moins polaire)
(4-diméthylamino-4-phénylcyclohexyl)-amide d'acide 3-(5-fluoro-1*H*-indole-3-yl)-pipéridine-1-carboxylique ; chlorhydrate (diastéréoisomère le plus polaire)
(4-diméthylamino-4-phénylcyclohexylméthyl)-amide d'acide 4-(5-fluoro-1*H*-indole-3-yl)-3,6-dihydro-2*H*-pyridine-1-carboxylique ; citrate (diastéréoisomère le plus polaire et diastéréoisomère le moins polaire)
(4-diméthylamino-4-phénylcyclohexylméthyl)-amide d'acide 4-(5-méthoxy-1*H*-indole-3-yl)-pipéridine-1-carboxylique ; citrate (diastéréoisomère le plus polaire et diastéréoisomère le moins polaire)
(4-diméthylamino-4-phénylcyclohexylméthyl)-amide d'acide 3-(1*H*-indole-3-yl)-pipéridine-1-carboxylique ; citrate (diastéréoisomère le plus polaire et diastéréoisomère le moins polaire)
(4-diméthylamino-4-phénylcyclohexylméthyl)-amide d'acide 3-(5-fluoro-1*H*-indole-3-yl)-pipéridine-1-carboxylique ; citrate (diastéréoisomère le plus polaire et diastéréoisomère le moins polaire)
(4-diméthylamino-4-phénylcyclohexylméthyl)-amide d'acide 3-(1*H*-indole-3-yl)-pyrrolidine-1-carboxylique ; citrate (diastéréoisomère le plus polaire et diastéréoisomère le moins polaire)
2-(4-diméthylamino-4-phénylcyclohexyl)-1-[3-(1*H*-indole-3-yl)-pipéridine-1-yl]-éthanone ; chlorhydrate (mélange de diastéréoisomères)
2-(4-diméthylamino-4-phénylcyclohexylidène)-1-[3-(1*H*-indole-3-yl)-pipéridine-1-yl]-éthanone ; chlorhydrate (mélange de diastéréoisomères)
4-[4-(5-chloro-1*H*-indole-3-yl)-3,6-dihydro-2*H*-pyridine-1-yl]-1-phénylcyclohexyl-diméthylamine ; dichlorhydate (mélange de diastéréoisomères)
{4-[4-(1*H*-indole-3-yl)-3,6-dihydro-2*H*-pyridine-1-yl]-1-phénylcyclohexyl}-diméthylamine ; dichlorhydrate (mélange de diastéréoisomères)
{4-[4-(5-méthoxy-1*H*-indole-3-yl)-3,6-dihydro-2*H*-pyridine-1-yl]-1-phénylcyclohexyl}-diméthylamine ; dichlorhydrate (mélange de diastéréoisomères)
(4-diméthylamino-4-phénylcyclohexylméthyl)-amide d'acide 4-(5-chloro-1*H*-indole-3-yl)-3,6-dihydro-2*H*-pyridine-1-carboxylique ; chlorhydrate (diastéréoisomère le plus polaire et diastéréoisomère le moins polaire)
2-(4- diméthylamino- 4-(4- fluorophényl)-cyclohexylidène)- 1-[3-(1*H*-indole- 3- yl)-pyrrolidine- 1- yl]- éthanone ; chlorhydrate (mélange de diastéréoisomères).

**8.** Médicament contenant au moins un dérivé de 1-aminocyclohexane substitué en position 4 selon l'une des revendications 1 à 7 ainsi que, le cas échéant, des additifs et/ou des substances auxiliaires convenables et/ou le cas échéant d'autres substances actives.

**9.** Utilisation d'un dérivé de 1-aminocyclohexane substitué en position 4 selon l'une des revendications 1 à 7 pour la préparation d'un médicament destiné au traitement de la douleur, en particulier d'une douleur aiguë, viscérale, neuropathique ou chronique.

**10.** Utilisation d'un dérivé de 1-aminocyclohexane substitué en position 4 selon l'une des revendications 1 à 7 pour la préparation d'un médicament destiné au traitement d'états d'anxiété, de stress et de syndromes liés au stress, de dépressions, de l'épilepsie, de la maladie d'Alzheimer, de la démence sénile, de troubles cognitifs généraux, de troubles de l'apprentissage et de la mémoire (comme agent nootrope), de phénomènes de privation, d'abus d'alcool et/ou de drogues et/ou de médicaments et/ou de dépendance envers ces substances, de dysfonctionnements sexuels, de maladies cardio-vasculaires, de l'hypotension, de l'hypertension, d'acouphènes, du prurit, de migraines, de la surdité, de la motilité intestinale déficiente, de troubles de l'absorption de nourriture, de l'anorexie, de l'obésité, de troubles moteurs, de diarrhées, de la cachexie, d'incontinence d'urine, ou comme myorelaxant, anticonvulsif ou anesthésique ou en vue d'une coadministration en cas de traitement avec un analgésique opioïde ou avec un anesthésique, en vue de la diurèse ou de l'antinatriurèse, en vue de l'anxiolyse, pour la modulation de l'activité motrice, pour la modulation de l'excrétion de neurotransmetteurs et pour le traitement de maladies neurodégénératives qui y sont liées, pour le traitement de phénomènes de privation et/ou pour la réduction du risque de dépendance envers les opioïdes.

**11.** Procédé de production de dérivés de 1-aminocyclohexanes substitués en position 4 selon l'une des revendications 1 à 7, **caractérisé en ce qu'**un dérivé de pyrrolidine ou de pipéridine

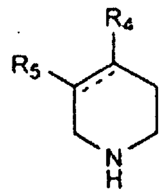
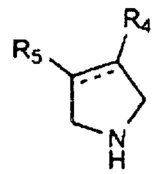

est amené à réagir avec un 4-aminocyclohexanone ou 4-aminocyclohexane-carbaldéhyde convenable dans des conditions d'amination par voie de réduction, par exemple avec des hydrures tels que le borohydrure de sodium ou de lithium, le cyanoborohydrure de sodium, le triacétoxyborohydrure de sodium, l'hydrure de diisobutylaluminium, le tri-(sec.-butyl)-borohydrure de lithium ou l'hydrure de lithium et d'aluminium.

**12.** Procédé de production de dérivés de 1-aminocyclohexanes substitués en position 4 selon l'une des revendications 1 à 7, **caractérisé en ce qu'**un dérivé de pyrrolidine ou de pipéridine

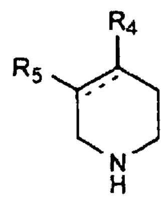
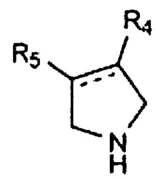

est amené à réagir avec un dérivé d'acide 4-aminocyclohexanecarboxylique ou d'acide 4-aminocyclohexylacétique convenable en présence de réactifs de couplage ou après transformation des acides carboxyliques en un chlorure d'acide ou en un ester actif, par exemple un ester de 4-nitrophényle ou un ester de N-hydroxysuccinimide.

**13.** Procédé de production de dérivés de 1-aminocyclohexanes substitués en position 4 selon l'une des revendications 1 à 7, **caractérisé en ce qu'**un dérivé de pyrrolidine ou de pipéridine

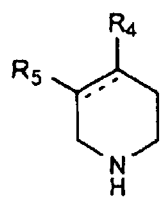
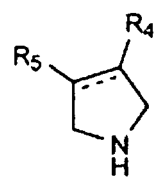

est amené à réagir à une température de 50 à 130°C avec un dérivé convenable d'ester phénylique d'acide (4-aminocyclohexyl)-carbamique ou d'ester phénylique d'acide (4-aminocyclohexyl)-méthylcarbamique, qui est préparé par réaction de l'ester phénylique d'acide chloroformique et d'un dérivé de 1,4-diaminocyclohexane ou de 4-aminométhylcyclohexylamine.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0187838 A
- US 4113866 A
- WO 0290317 A


### In der Beschreibung aufgeführte Nicht-Patentliteratur


- **MEUNIER et al.** *Nature,* 1995, vol. 377, 532-535
- **REINSCHEID et al.** *Science,* 1995, vol. 270, 792-794
- **MOGIL et al.** *Neuroscience,* 1996, vol. 75, 333-337
- **JENCK et al.** *Proc. Natl. Acad. Sci. USA,* 1997, vol. 94, 14854-14858
- **KING et al.** *Neurosci. Lett.,* 1997, vol. 223, 113-116
- **ABDULLA ; SMITH.** *J. Neurosci.,* 1998, vol. 18, 9685-9694
- **MANABE et al.** *Nature,* 1997, vol. 394, 577-581
- **NISHI et al.** *EMBO J.,* 1997, vol. 16, 1858-1864
- **CALO et al.** *Br.J. Pharmacol.,* 2000, vol. 129, 1261-1283
- **LEDNICER et al.** *J. Med. Chem.,* 1980, vol. 23, 424-430
- **ARDATI et al.** *Mol. Pharmacol.,* 1997, vol. 51, 816-824